(19) Europäisches Patentamt / European Patent Office / Office européen des brevets

(11) EP 4 242 204 A1

(12) **EUROPEAN PATENT APPLICATION**
published in accordance with Art. 153(4) EPC

(43) Date of publication:
**13.09.2023 Bulletin 2023/37**

(21) Application number: **21888667.9**

(22) Date of filing: **05.11.2021**

(51) International Patent Classification (IPC):
***C07D 403/04*** $^{(2006.01)}$ ***A61P 35/00*** $^{(2006.01)}$
***A61K 31/513*** $^{(2006.01)}$

(52) Cooperative Patent Classification (CPC):
**A61K 31/513; A61P 35/00; C07D 403/04**

(86) International application number:
**PCT/CN2021/129079**

(87) International publication number:
**WO 2022/095975 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.11.2020 CN 202011225900**
**30.04.2021 CN 202110480100**

(71) Applicant: **Wuhan Humanwell Innovative Drug Research and Development Center Limited Company**
**Wuhan, Hubei 430075 (CN)**

(72) Inventors:
- **ZHANG, Xuejun**
  **Wuhan, Hubei 430075 (CN)**
- **CHANG, Shaohua**
  **Wuhan, Hubei 430075 (CN)**
- **YE, Dabing**
  **Wuhan, Hubei 430075 (CN)**
- **LEI, Sijun**
  **Wuhan, Hubei 430075 (CN)**
- **WANG, Yonggang**
  **Wuhan, Hubei 430075 (CN)**
- **LIU, Yong**
  **Wuhan, Hubei 430075 (CN)**
- **SUN, Hongna**
  **Wuhan, Hubei 430075 (CN)**
- **YANG, Jun**
  **Wuhan, Hubei 430075 (CN)**
- **LI, Lie**
  **Wuhan, Hubei 430075 (CN)**

(74) Representative: **DehnsGermany Partnerschaft von Patentanwälten**
**Theresienstraße 6-8**
**80333 München (DE)**

# (54) CD73 INHIBITOR AND USE THEREOF

(57) The present disclosure provides a novel compound for effectively inhibiting the activity of CD73, a preparation method thereof, and use thereof in the preparation of drugs, and the novel compound is a compound represented by formula I, or a tautomer, stereoisomer, hydrate, solvate, pharmaceutically acceptable salt, or prodrug thereof.

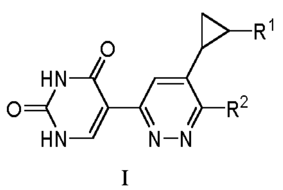

I

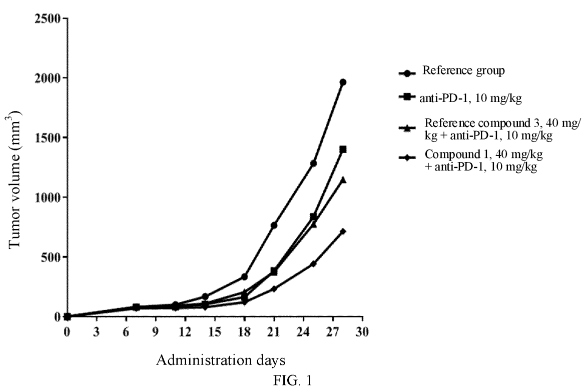


FIG. 1

EP 4 242 204 A1

## Description

### CROSS-REFERENCE TO RELATED APPLICATIONS

This application claims priority to and the benefit of Chinese Patent Application No. 202011225900.8, filed on November 05, 2020 before the China National Intellectual Property Administration, and Chinese Patent Application No. 202110480100.9, filed on April 30, 2021 before the China National Intellectual Property Administration, which are hereby incorporated by reference in their entireties.

### TECHNICAL FIELD

The present disclosure belongs to the field of medicinal chemistry, specifically relates to a CD73 inhibitor and use thereof, and more specifically relates to a pyrimidinedione compound, a preparation method thereof, and use thereof in the preparation of drugs.

### BACKGROUND

CD73, also known as ecto-5'-nucleotidase, is an exonuclease belonging to the metallophosphatase superfamily, and is a peripheral glycoprotein. CD73 is mainly anchored on the plasma membrane through glycosylphosphatidylinositol (GPI), has a molecular weight of 70 kDa, and is encoded by the NT5E gene. CD73 is widely expressed on the cell surfaces of different tissues, including brain, lung, heart, spleen, lymph node, kidney, colon, vascular endothelium, and bone marrow. CD73 is also expressed in a variety of immune cells, including macrophages, neutrophils, myeloid-derived suppressor cells (MDSCs), dendritic cells (DCs), natural killer cells (NK), and regulatory T cells (Treg) (Soleimani A et al., Biochimie, 2020, 176: 21-30. CD73 is also highly expressed in many types of tumor cells such as melanoma, breast cancer, pancreatic cancer, ovarian cancer, colon cancer, and prostate cancer (Gao Z et al., Biomed Res Int, 2014, 2014: 460654). CD73 is also present in biological fluids including serum in a soluble form (sCD73) and retains the total enzyme activity.

CD73 exerts physiological and pathological effects mainly by hydrolyzing adenosine monophosphate (AMP) to produce extracellular adenosine (ADO), and ADO exerts effects by binding to 4 G protein-coupled receptors: the adenosine A1 receptor (A1AR), the adenosine A2A receptor (A2AR), the adenosine A2B receptor (A2BR), and the adenosine A3 receptor (A3AR), among which A2AR plays the dominant role (Linden J et al., Annu. Rev. Immunol., 2019, 37: 325-347). Adenosine receptors (ARs) are expressed not only in tumor cells, but also on the cell surface of immune cells and vascular endothelial cells that are infiltrated in a tumor microenvironment, and ADO exerts multiple immunosuppressive and tumor-promoting effects by binding to receptors.

CD73 is closely associated with the growth, angiogenesis, and metastasis of tumors. Under normal physiological conditions, a level of extracellular ADO is 20 to 300 nM, which is increased to and maintained at a micromole level (30 to 100 $\mu$M) in a tumor microenvironment, and the high concentration of extracellular ADO is mainly affected by hydrolysis of AMP with CD73. Studies show that a level of soluble CD73 (sCD73) in the plasma of a cancer patient is higher than that of a healthy person (Klemens M R et al., Biochem. Biophys. Res. Commun., 1990, 172: 1371-7). In gastrointestinal stromal tumor, a higher level of CD73 is expressed in tumor-infiltrated NK cells, loss of A2AR signaling in NK cells can improve the metastasis of $CD73^+$ tumors and enhance anti-tumor immune response (Young Aet al., Cancer Cell. 2016; 30 (3): 391-403). Compared with the normal pancreatic tissue, the expression of CD73 is up-regulated in pancreatic ductal adenocarcinoma (PDAC), and is associated with tumor size, metastasis, and poor prognosis (Harvey Jerry B et al., Front Immunol, 2020, 11: 508). In the preclinical study carried out by ORIC, the CD73-selective inhibitor ORIC-533 significantly reduces the concentration of ADO in a tumor microenvironment and also reduces tumor volume. Results of these studies show that the expression of CD73 is up-regulated in a variety of tumors, and inhibition of CD73 may reduce the concentration of ADO, so as to inhibit the growth and metastasis of tumors.

CD73 inhibitors can be used alone to block the growth of tumors by relieving immunosuppression, and can also be used in combination with other targeted therapies and/or immunotherapies, or radiotherapy to enhance an anti-tumor effect. In mouse models of some tumors, the combination of anti-CD73 antibody and anti-PD-1/L1 (programmed death receptor 1/ligand 1) and/or anti-CTLA-4 (cytotoxic T-lymphocyte-associated protein 4) antibody is more effective than the use of the anti-PD-L1 and/or anti-CTLA-4 antibody alone (Allard B et al., Clin. Cancer Res., 2013, 19: 5626-35). It is found that a level of CD73 in a patient with melanoma is up-regulated after immunological treatment with an anti-PD-1 antibody, a distinctive macrophage population highly expressing CD73 is persistent in a patient with glioblastoma after anti-PD-1 treatment, and the deficiency of CD73 enhances the efficacy of the anti-PD-1 and anti-CTLA-4 antibodies in a mouse model of glioblastoma (Goswami S et al., Nat. Med., 2020, 26: 39-46). Radiotherapy causes cytoclasis of some tumor cells, such that abundant intracellular ATPs are released to the outside of cells and transformed into adenosine under the action of CD73 on the surface of tumor cells or free CD73 to exert an immunosuppressive effect,

which is regarded as one of the reasons for poor prognosis of some patients after radiotherapy. Therefore, the combination of a CD73 inhibitor and radiotherapy may have a synergistic effect (Wennerberg E et al., Cancer Immunol Res, 2020, 8: 465-478).

Currently, some anti-CD73 monoclonal antibodies (MEDI9447, BMS986179, SRF373/NZV930, CPI-006/CPX-006, and TJ004309) and selective small-molecule inhibitors (LY3475070 and AB680) have entered clinical stage, with encouraging early results (NCT02754141) from some trials, and CD73 inhibitors may be a promising approach for the treatment of tumors.

## SUMMARY

The present disclosure is intended to propose a novel CD73 inhibitor, which can be used for the preparation of drugs for treating a tumor-associated disease.

In a first aspect of the present disclosure, the present disclosure proposes a compound, which is a compound represented by formula I, or a tautomer, stereoisomer, hydrate, solvate, pharmaceutically acceptable salt, or prodrug thereof:

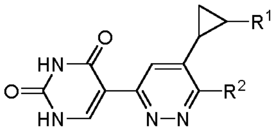

I

wherein:

$R^1$ is selected from

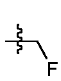, 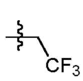, 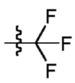, 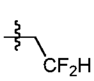, 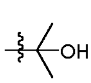, 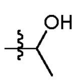, 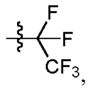, or 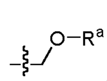,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four-to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P; and

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five-to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the ($C_1$-$C_6$ alkyl)-O- substituted with $R^b$, the ($C_1$-$C_6$ alkyl)-S-substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, and ($C_1$-$C_6$ alkyl)-O-, wherein when more than one substituents are present, the more than one substituents are identical or different, and wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

In a preferred embodiment of the present disclosure, the compound represented by formula I, or the tautomer, stereoisomer, hydrate, solvate, pharmaceutically acceptable salt, or prodrug thereof is:

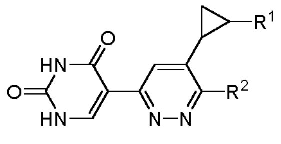

I

wherein:

$R^1$ is selected from

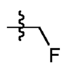, 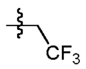, 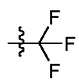, 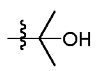, 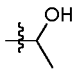, 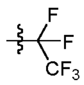, or 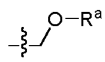,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four-to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P;

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five-to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S-substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different, and wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

In a preferred embodiment of the present disclosure, when $R^a$ is $C_1$-$C_6$ alkyl, the $C_1$-$C_6$ alkyl is $C_1$-$C_4$ alkyl, and preferably methyl, ethyl, n-propyl, isopropyl, n-butyl, or isobutyl.

In a preferred embodiment of the present disclosure, when $R^a$ is $C_1$-$C_6$ alkyl substituted with 1 to 5 identical or different halogen atoms, the $C_1$-$C_6$ alkyl is $C_1$-$C_4$ alkyl, and preferably methyl, ethyl, n-propyl, isopropyl, n-butyl, or isobutyl.

In a preferred embodiment of the present disclosure, when $R^a$ is $C_1$-$C_6$ alkyl substituted with 1 to 5 identical or different halogen atoms, the halogen atoms are F, Cl, Br, or I, and preferably F or Cl.

In a preferred embodiment of the present disclosure, when $R^a$ is $C_1$-$C_6$ alkyl substituted with 1 to 5 identical or different halogen atoms, the number of the halogen atoms is 1, 2 or 3, and preferably 3.

In a preferred embodiment of the present disclosure, when $R^a$ is $C_3$-$C_6$ cycloalkyl, the $C_3$-$C_6$ cycloalkyl is independently cyclopropyl, cyclobutyl, cyclopentyl, or cyclohexyl, and preferably cyclopropyl.

In a preferred embodiment of the present disclosure, when $R^a$ is five- to eight-membered aryl, the five- to eight-membered aryl is independently phenyl or naphthyl, and preferably phenyl.

In a preferred embodiment of the present disclosure, when $R^a$ is five- to eight-membered heteroaryl, the five-to eight-membered heteroaryl is independently pyrrole, pyrazole, triazole, furan, oxazole, thiophene, thiazole, pyridine, pyrazine, or pyrimidine, and preferably pyrazole, furan, thiophene, or pyridine.

In a preferred embodiment of the present disclosure, when $R^a$ is four- to eight-membered heterocycloalkyl, the four-to eight-membered heterocycloalkyl is independently azetidine, oxetane, tetrahydropyrrolidinyl, tetrahydrofuranyl, hexahydropyran, or tetrahydro-2H-thiopyran 1,1-dioxide, and preferably azetidine or oxetane.

In a preferred embodiment of the present disclosure, when $R^a$ is four- to eight-membered heterocycloalkenyl, the four- to eight-membered heterocycloalkenyl is independently dihydropyridyl, tetrahydropyridyl, tetrahydropyrimidinyl,

pyrrolinyl, imidazolinyl, pyrazolinyl, dihydroimidazolyl, dihydropyrazolyl, dihydrooxazolyl, dihydrooxadiazolyl, dihydrothiazolyl, dihydroisothiazolyl, dihydrothienyl, dihydropyrrolyl, 3,4-dihydro-2H-pyranyl, dihydrofuranyl, dihydropyrazinyl, dihydropyrimidyl, or fluorodihydrofuranyl, and preferably 1,2,3,4-tetrahydropyridyl, 1,2-dihydropyridyl, 1,4-dihydropyridyl, 1,2,3,6-tetrahydropyridyl, 3,4-dihydro-2H-pyranyl, or dihydrofuranyl.

In a preferred embodiment of the present disclosure, $R^2$ is cyano.

In a preferred embodiment of the present disclosure, when $R^2$ is halogen, the halogen is F, Cl, Br, or I, and preferably Cl.

In a preferred embodiment of the present disclosure, when $R^2$ is $C_1$-$C_6$ alkyl unsubstituted or substituted with $R^b$, the $C_1$-$C_6$ alkyl is $C_1$-$C_4$ alkyl, and preferably methyl, ethyl, n-propyl, isopropyl, n-butyl, or isobutyl.

In a preferred embodiment of the present disclosure, when $R^2$ is ($C_1$-$C_6$ alkyl)-O- unsubstituted or substituted with $R^b$, the ($C_1$-$C_6$ alkyl)-O- is ($C_1$-$C_4$ alkyl)-O-, and preferably methyl-O-.

In a preferred embodiment of the present disclosure, when $R^2$ is ($C_1$-$C_6$ alkyl)-S- unsubstituted or substituted with $R^b$, the ($C_1$-$C_6$ alkyl)-S- is ($C_1$-$C_4$ alkyl)-S-, and preferably methyl-S-.

In a preferred embodiment of the present disclosure, when $R^2$ is five- to eight-membered aryl unsubstituted or substituted with $R^b$, the five- to eight-membered aryl is independently phenyl or naphthyl, and preferably phenyl.

In a preferred embodiment of the present disclosure, when $R^2$ is five- to eight-membered heteroaryl unsubstituted or substituted with $R^b$, the five- to eight-membered heteroaryl is independently pyrrole, pyrazole, triazole, furan, oxazole, thiophene, thiazole, pyridine, pyrazine, or pyrimidine, and preferably pyrazole, furan, thiophene, or pyridine.

In a preferred embodiment of the present disclosure, when $R^2$ is four- to eight-membered heterocycloalkyl unsubstituted or substituted with $R^b$, the four- to eight-membered heterocycloalkyl is independently azetidine, oxetane, tetrahydropyrrolidinyl, tetrahydrofuranyl, hexahydropyran, or tetrahydro-2H-thiopyran 1,1-dioxide, and preferably azetidine or oxetane.

In a preferred embodiment of the present disclosure, when $R^2$ is four- to eight-membered heterocycloalkenyl unsubstituted or substituted with $R^b$, the four- to eight-membered heterocycloalkenyl is independently dihydropyridyl, tetrahydropyridyl, tetrahydropyrimidinyl, pyrrolinyl, imidazolinyl, pyrazolinyl, dihydroimidazolyl, dihydropyrazolyl, dihydrooxazolyl, dihydrooxadiazolyl, dihydrothiazolyl, dihydroisothiazolyl, dihydrothienyl, dihydropyrrolyl, 3,4-dihydro-2H-pyranyl, dihydrofuranyl, dihydropyrazinyl, dihydropyrimidyl, or fluorodihydrofuranyl, and preferably 1,2,3,4-tetrahydropyridyl, 1,2-dihydropyridyl, 1,4-dihydropyridyl, 1,2,3,6-tetrahydropyridyl, 3,4-dihydro-2H-pyranyl, or dihydrofuranyl.

In a preferred embodiment of the present disclosure, when $R^2$ is $C_2$-$C_6$ alkenyl unsubstituted or substituted with $R^b$, the $C_2$-$C_6$ alkenyl is vinyl, 1-propenyl, 2-propenyl, or allyl, and preferably vinyl or allyl.

In a preferred embodiment of the present disclosure,

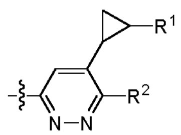

is

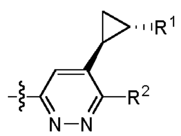

or

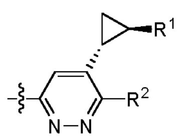

.

In a preferred embodiment of the present disclosure,

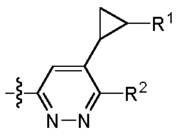

is

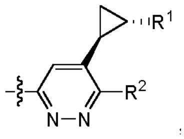

wherein $R^1$ is selected from

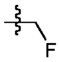, 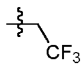, 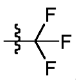, 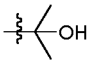, 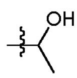, 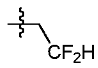, or 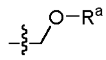,

wherein $R^a$ is $C_1$-$C_6$ alkyl unsubstituted or substituted with one or more identical or different halogen atoms.

In a preferred embodiment of the present disclosure, $R^a$ is $C_1$-$C_4$ alkyl unsubstituted or substituted with 1 to 5 identical or different halogen atoms.

In a preferred embodiment of the present disclosure, $R^a$ is selected from methyl, trifluoromethyl, or difluoromethyl.

In a preferred embodiment of the present disclosure, $R^2$ is selected from hydrogen, halogen, cyano, or $C_1$-$C_4$ alkyl unsubstituted or substituted with $R^b$, wherein $R^b$ is each independently halogen.

In a preferred embodiment of the present disclosure, $R^2$ is selected from Cl or methyl.

In a preferred embodiment of the present disclosure, $R^b$ is each independently halogen, wherein the halogen is F, Cl, or Br.

In a preferred embodiment of the present disclosure,

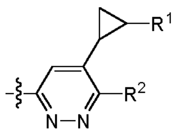

is

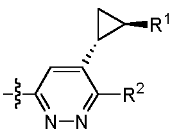,

wherein $R^1$ is selected from

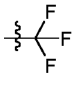 or 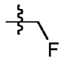.

In a preferred embodiment of the present disclosure, $R^2$ is selected from hydrogen, halogen, cyano, or $C_1$-$C_4$ alkyl unsubstituted or substituted with $R^b$, wherein $R^b$ is each independently halogen.

In a preferred embodiment of the present disclosure, $R^2$ is Cl.

In a preferred embodiment of the present disclosure,

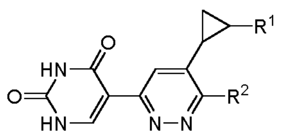

I

is a racemic mixture of

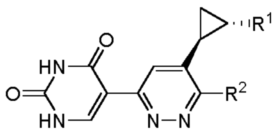

II

and

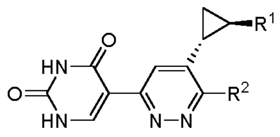

III

.

In a preferred embodiment of the present disclosure, $R^2$ is selected from hydrogen, halogen, cyano, and $C_1$-$C_4$ alkyl unsubstituted or substituted with $R^b$.

In a preferred embodiment of the present disclosure, $C_1$-$C_4$ alkyl is selected from methyl, ethyl, n-propyl, isopropyl, n-butyl, and isobutyl.

In a preferred embodiment of the present disclosure, $R^2$ is Cl.

In a more preferred embodiment of the present disclosure,

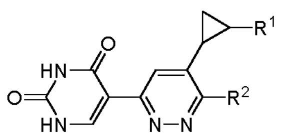

I

is a racemic mixture of

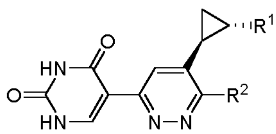

II

and

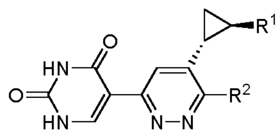

III

,

wherein $R^1$ is

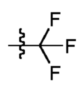

;

and $R^2$ is selected from hydrogen, halogen, cyano, or $C_1$-$C_4$ alkyl unsubstituted or substituted with $R^b$, wherein the $C_1$-$C_4$ alkyl is selected from methyl or ethyl, preferably, $R^2$ is halogen, wherein the halogen is F, Cl, Br, or I, and preferably Cl.

In a preferred embodiment of the present disclosure, the compound represented by formula I, or the tautomer, stereoisomer, hydrate, solvate, pharmaceutically acceptable salt, or prodrug thereof is:

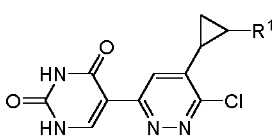

II

,

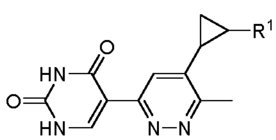

III

, or

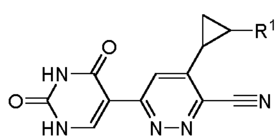

III-A

,

wherein $R^1$ is

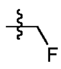, 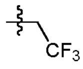, 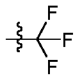, 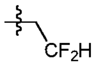, 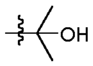, 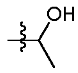, 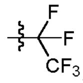, or 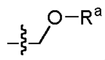,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four-to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P.

In a preferred embodiment of the present disclosure, the compound represented by formula I, or the tautomer, stereoisomer, hydrate, solvate, pharmaceutically acceptable salt, or prodrug thereof is:

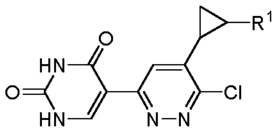

II

or

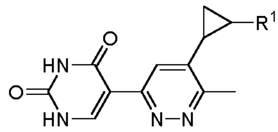

III

,

wherein $R^1$ is

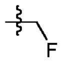, 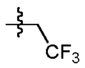, 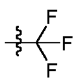, 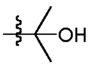, 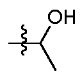, 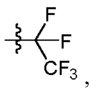, or 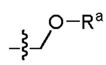,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four-to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P.

In a preferred embodiment of the present disclosure, the compound represented by formula I, or the tautomer, stereoisomer, hydrate, solvate, pharmaceutically acceptable salt, or prodrug thereof is:

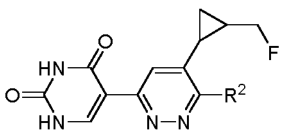

IV

wherein:

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five-to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different; and

the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with

$R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

In a preferred embodiment of the present disclosure, the compound represented by formula I, or the tautomer, stereoisomer, hydrate, solvate, pharmaceutically acceptable salt, or prodrug thereof is:

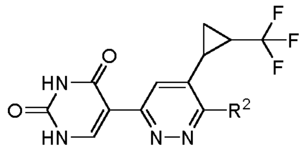

V

,

wherein:

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five-to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different; and

the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

In a preferred embodiment of the present disclosure, the compound represented by formula I, or the tautomer, stereoisomer, hydrate, solvate, pharmaceutically acceptable salt, or prodrug thereof is:

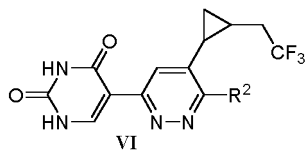

VI

wherein:

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five-to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different; and

the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

In a preferred embodiment of the present disclosure, the compound represented by formula I, or the tautomer, stereoisomer, hydrate, solvate, pharmaceutically acceptable salt, or prodrug thereof is:

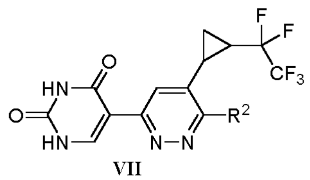
VII

,

wherein:

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five-to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the ($C_1$-$C_6$ alkyl)-O- substituted with $R^b$, the ($C_1$-$C_6$ alkyl)-S-substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, whrein when more than one substituents are present, the more than one substituents are identical or different; and

the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

In a preferred embodiment of the present disclosure, the compound represented by formula I, or the tautomer, stereoisomer, hydrate, solvate, pharmaceutically acceptable salt, or prodrug thereof is any one of the following compounds:

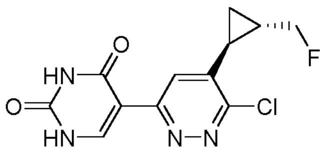
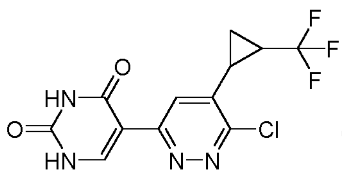
Trans racemic mixtures
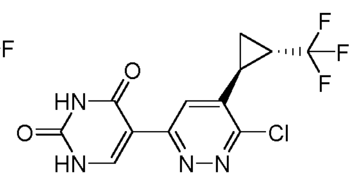

**1** **2** **3**

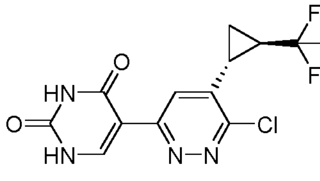
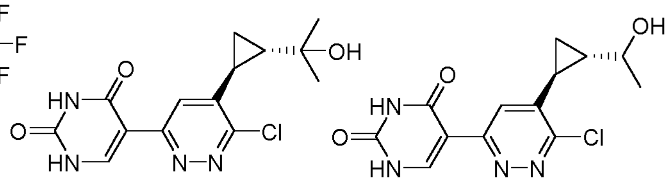

**4** **5** **6**

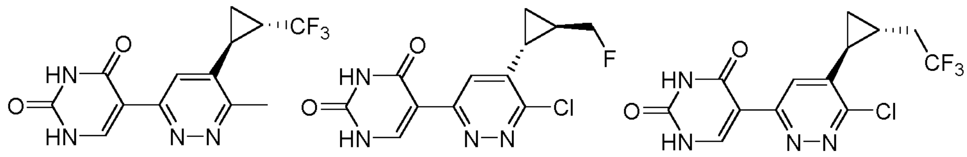

7 8 9

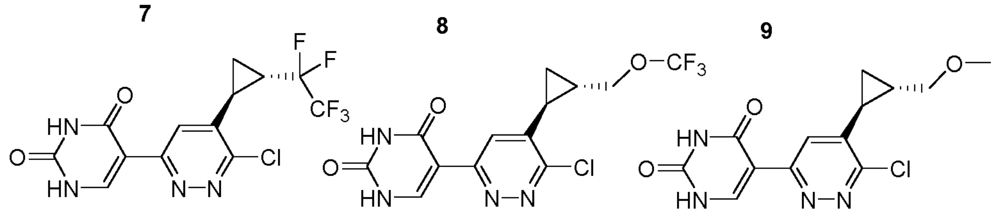

10 11 12

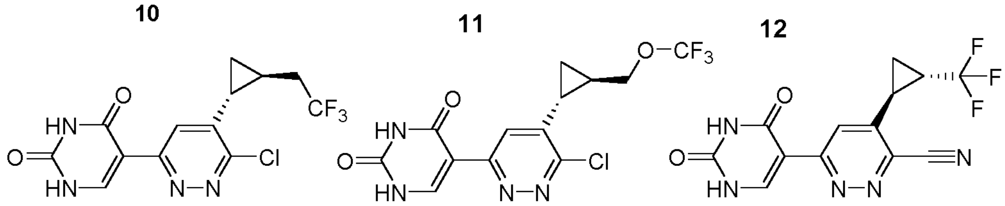

13 14 15

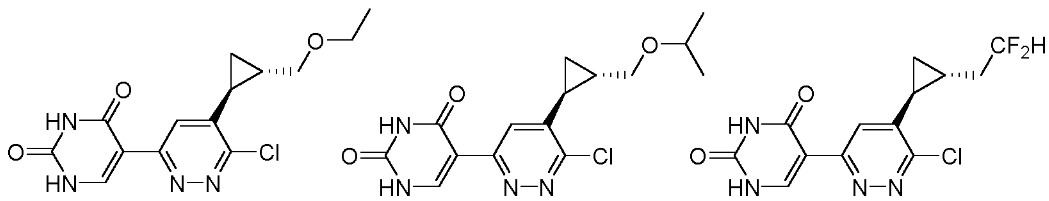

16 17 18

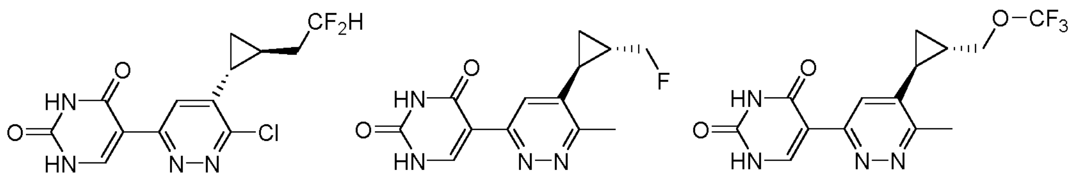

19 20 21

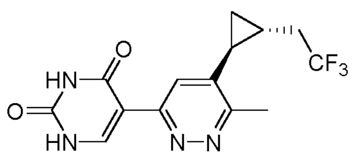

22

In a second aspect of the present disclosure, the present disclosure proposes a pharmaceutical composition, which includes a therapeutically effective amount of the above compound or the tautomer, stereoisomer, hydrate, solvate, pharmaceutically acceptable salt, or prodrug thereof, and a pharmaceutically acceptable excipient.

According to specific embodiments of the present disclosure, the pharmaceutical composition of the present disclosure may be a pharmaceutical preparation formed by mixing a therapeutically effective amount of the above compound or the tautomer, stereoisomer, hydrate, solvate, pharmaceutically acceptable salt, or prodrug thereof with a pharmaceutically acceptable carrier, diluent or excipient, which is suitable for oral or parenteral administration. Administration methods include, but are not limited to, intradermal administration, intramuscular administration, intraperitoneal administration, intravenous administration, subcutaneous administration, intranasal administration, and oral administration. The preparation can be administered by a variety of routes, for example, administered by infusion or bolus through the epithelium or skin and mucosa (e.g., oral mucosa and rectum) absorption. Administration may be performed systemically or locally. Examples of preparations suitable for oral administration include solid and liquid dosage forms, and specifically, include tablets, pills, granules, powder, capsules, syrups, emusions, suspensions, etc. The preparation can

be prepared by the methods known in the art, and contains a carrier, diluent or excipient conventionally used in the field of pharmaceutical preparations.

In a third aspect of the present disclosure, the present disclosure proposes use of the above compound or the tautomer, stereoisomer, hydrate, solvate, pharmaceutically acceptable salt, or prodrug thereof in combination with PD-1/PD-L1/CTLA-4 antibodies or PD-1/PD-L1/CTLA-4 inhibitors in the preparation of a drug for treating a CD73-associated disease, and the drug can be used for treating cancer. The cancer includes, for example, bladder cancer, breast cancer, cholangiocarcinoma, rectal cancer, colon cancer, gastric cancer, gallbladder cancer, neuroblastoma, head and neck cancer, liver cancer, lung cancer, lymphoma, medulloblastoma, melanoma, ovarian cancer, pancreatic cancer, prostate cancer, and kidney cancer.

In a fourth aspect of the present disclosure, the present disclosure proposes use of the above compound or the tautomer, stereoisomer, hydrate, solvate, pharmaceutically acceptable salt, or prodrug thereof, or the above pharmaceutical composition in the preparation of a drug for treating a CD73-associated disease.

According to specific embodiments of the present disclosure, the above compound or the tautomer, stereoisomer, hydrate, solvate, pharmaceutically acceptable salt, or prodrug thereof, or the above pharmaceutical composition is used for preparing a drug for treating a CD73-associated disease, and the drug can be used for treating cancers. The cancers include, for example, bladder cancer, breast cancer, cholangiocarcinoma, colorectal cancer, colon cancer, gastric cancer, gallbladder cancer, glioblastoma, head and neck cancer, liver cancer, lung cancer, lymphoma, medulloblastoma, melanoma, ovarian cancer, pancreatic cancer, prostate cancer, and kidney cancer.

In a fifth aspect of the present disclosure, the present disclosure proposes a method for treating a CD73-associated disease, which includes: administering the above compound or the tautomer, stereoisomer, hydrate, solvate, pharmaceutically acceptable salt, or prodrug thereof, and/or the above pharmaceutical composition to a subject in need. The CD73-associated disease is cancer. The cancer includes, for example, bladder cancer, breast cancer, cholangiocarcinoma, rectal cancer, colon cancer, gastric cancer, gallbladder cancer, neuroblastoma, head and neck cancer, liver cancer, lung cancer, lymphoma, medulloblastoma, melanoma, ovarian cancer, pancreatic cancer, prostate cancer, and kidney cancer.

Terms and definitions

Unless otherwise indicated, terms and definitions used in the present disclosure, including the description and claims of the present disclosure, are as follows.

Those skilled in the art will understand that, in accordance with the conventions used in the art, in the structural formulas of this disclosure, 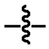 is used to depict a chemical bond, which is a point at which a moiety or substituent group is linked to a core structure or backbone structure.

The term "pharmaceutically acceptable" is used for illustrating compounds, materials, compositions and/or dosage forms, which are within the scope of reliable medical judgement, are suitable for use in contact with tissues of humans and animals without causing excessive toxicity, irritation, allergic reactions or other problems or complications, and are commensurate with a reasonable benefit/risk ratio.

The term "pharmaceutically acceptable salt" refers to salts of pharmaceutically acceptable nontoxic acids and bases, including salts of inorganic acids and bases, and organic acids and bases.

In addition to the pharmaceutically acceptable salt, other salts are also taken into account in the present disclosure. They can be used as intermediates during purification of compounds or preparation of other pharmaceutically acceptable salts, or can be used for the identification, characterization, or purification of the compound of the present disclosure.

The term "pharmaceutical composition" refers to mixtures of one or more of the compounds or the physiologically/pharmaceutically acceptable salts or prodrugs thereof of the present disclosure and other chemical components such as physiologically/pharmaceutically acceptable carriers and excipients. The purpose of the pharmaceutical composition is to promote administration of the compound to an organism.

The term "adjuvant" refers to medicinal inert ingredients. Examples of types of the term "excipient" include, but are not limited to, an adhesive, a disintegrant, a lubricant, a glidant, a stabilizer, a filler, a diluent, etc. Excipients can enhance the operating characteristics of pharmaceutical preparations, that is, improve the fluidity and/or adhesiveness to enable preparations to be more suitable for direct compression.

The term "prodrug" refers to a material that can be transformed into a compound of the present disclosure having bioactivity under the physiological conditions or by dissolving in a solvent. The prodrug of the present disclosure is prepared by modifying functional groups in the compound, and the modification can be removed conventionally or in vivo to obtain a parent compound. The prodrug includes a compound formed by linking one hydroxyl group or amino group in the compound of the present disclosure to any group, and when administered to an individual mammal, the prodrug of the compound of the present disclosure is cleaved to form a free hydroxyl group or free amino group.

The term "stereoisomer" refers to isomers formed due to different arrangement modes of atoms in a molecule in space, including a cis-trans isomer, an enantiomer, a diastereoisomer, and a conformational isomer.

According to selected raw materials and methods, the compound of the present disclosure may be present in the form of a possible isomer or a mixture of isomers, for example, in the form of a purely optically active isomer or a mixture of isomers such as a mixture of a racemate and a diastereoisomer, depending on the number of asymmetric carbon atoms. When an optically active compound is described, prefixes D and L, or R and S are used to represent an absolute configuration of a molecule with respect to a chiral center (or multiple chiral centers) in the molecule. Prefixes D and L, or (+) and (-) are symbols used for designating the rotation of plane polarized light caused by a compound, and (-) or L indicates that a compound is levorotatory. Compounds with the prefix (+) or D are dextrorotatory. With respect to the given chemical structure, these stereoisomers are identical except that they are mirror images of each other. Specific stereoisomers may also be referred to as enantiomers, and mixtures of the isomers are usually referred to as mixtures of enantiomers. A mixture of enantiomers in a ratio of 50: 50 is referred to as a racemic mixture or racemate, and when there is no stereoselectivity or stereospecificity in a chemical reaction or method, a racemic mixture or racemate may appear. Many geometrical isomers of olefin and C=N double bond and the like may present in the compound of the present disclosure, and such stable isomers are all taken into account in the present disclosure. When the compound of the present disclosure contains an olefinic double bond, unless otherwise indicated, such a double bond includes E and Z geometrical isomers. If the compound contains a disubstituted cycloalkyl group, substituent groups of the cycloalkyl group may be in a cis- or trans-configuration.

When a bond to a chiral carbon in the formula of the present disclosure is depicted as a straight line, it is to be understood that (R) and (S) configurations of the chiral carbon and produced enantiomerically pure compounds and mixtures are all included within the scope of the general formula. The racemate or enantiomerically pure compounds of the present disclosure are graphically represented with reference to Maehr, J. Chem. Ed. 1985, 62: 114-120. Unless otherwise indicated, a wedge bond and a dashed bond are used to represent an absolute configuration of a stereocenter.

An optically active (R)- or (S)-isomer can be prepared from a chiral synthon or chiral preparation, or prepared by a conventional resolution technique. The compound containing asymmetrically substituted carbon atoms of the present disclosure can be separated in the optically active form or racemic form. Resolution of a racemic mixture of the compound can be performed by any method known in the art. Exemplary methods include fractional recrystallization using a chiral resolving acid that is an optically active salt-forming organic acid. Resolving agents applicable to fractional recrystallization are, for example, optically active acids such as tartaric acid, diacetyltartaric acid, dibenzoyltartaric acid, mandelic acid, malic acid, lactic acid, and D- and L-configurations of various optically active camphorsulfonic acid such as β-camphorsulfonic acid. Other resolving agents applicable to fractional recrystallization include stereoisomerically pure α-methyl-benzylamine (e.g., S- and R-configurations or a diastereomerically pure configuration), 2-phenylglycinol, norephedrine, ephedrine, N-methylephedrine, cyclohexylethylamine, 1,2-diaminocyclohexane, etc. Resolution of a racemic mixture can also be performed by elution using a chromatographic column filled with an optically active resolving agent (e.g., dinitrobenzoylphenylglycine). High-performance liquid chromatography (HPLC) or supercritical fluid chromatography (SFC) can be adopted. Those skilled in the art can select a specific method, elution conditions, and a chromatographic column according to the structure of the compound and test results. Further, any enantiomer or diastereomer of the compound of the present disclosure can also be obtained by stereo organic synthesis using optically pure starting materials in known configurations or reagents.

The term "tautomer" refers to functional group isomers formed by rapid movement of a certain atom in a molecule between two positions. The compound of the present disclosure may have a tautomerism phenomenon. The tautomeric compound may be present in two or more interconvertible forms. A prototropic tautomer is formed by migration of a hydrogen atom covalently bonded between two atoms. Tautomers are generally present in an equilibrium form, and separation of a single tautomer usually yields one mixture whose physicochemical properties are consistent with those of a mixture of compounds. The position of equilibrium depends on intramolecular chemical properties. For example, in several aliphatic aldehydes and ketones such as acetaldehyde, ketonic configurations prevail, while in phenol, an enolic configuration prevails. The present disclosure covers all tautomers of the compound.

The compound of the present disclosure may contain an unnatural proportion of atomic isotopes at one or more atoms forming the compound. For example, the compound can be labeled with radioisotopes such as deuterium ($^{2}H$), tritium ($^{3}H$), iodine-125 ($^{125}I$), and C-14 ($^{14}C$). All changes made to the isotope composition of the compound of the present disclosure, regardless of whether they are radioactive or not, shall fall within the scope of the present disclosure.

With respect to pharmaceutical or pharmacological activators, the term "effective amount" or "therapeutically effective amount" refers to an amount of a drug or pharmaceutical preparation that is nontoxic but sufficient to achieve a desired effect. For oral preparations of the present disclosure, an "effective amount" of an active substance in a composition refers to an amount required to achieve a desired effect when the active substance is used in combination with another active substance in the composition. The effective amount varies from person to person, and is determined based on age and general conditions of a subject as well as a specific active substance to be used, and those skilled in the art can determine appropriate effective amounts for individual cases according to conventional tests.

The term "active ingredient", "therapeutic agent", "active substance", or "activator" refers to a chemical entity that can effectively treat a target disorder, disease or symptom.

The term "substituted" refers to that any one or more hydrogen atoms on a specified atom are substituted with substituent groups, including variants of deuterium and hydrogen, as long as the valence state of the specific atom is normal and the substituted compound is stable. When the substituent group is a ketone group (i.e., =O), two hydrogen atoms are substituted. Ketone substitution will not occur on an aromatic group. The term "optionally substituted" refers to unsubstituted or substituted, unless otherwise specified, types and number of substituent groups may be arbitrary on the basis of chemical realization.

The term "$C_1$-$C_6$ alkyl" is to be understood as straight or branched saturated monovalent hydrocarbyl having 1, 2, 3, 4, 5, or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or isomers thereof. Particularly, the group has 1, 2, or 3 carbon atoms ("$C_1$-$C_3$ alkyl"). For example, the $C_1$-$C_6$ alkyl is methyl, ethyl, n-propyl, or isopropyl.

The term "($C_1$-$C_6$ alkyl)-O-" is to be understood as an alkyl group linked to the rest moiety of a molecule through an oxygen atom, where "$C_1$-$C_6$ alkyl" is defined as above. For example, the ($C_1$-$C_6$ alkyl)-O- is methyl-O- or ethyl-O-.

The term "($C_1$-$C_6$ alkyl)-S-" is to be understood as an alkyl group linked to the rest moiety of a molecule through a sulfur atom, where "$C_1$-$C_6$ alkyl" is defined as above. For example, the ($C_1$-$C_6$ alkyl)-S- is methyl-S- or ethyl-S-.

The term "$C_2$-$C_6$ alkenyl" is to be understood as a straight or branched hydrocarbon chain group that is composed of carbon atoms and hydrogen atoms only, contains at least one double bond, has 2 to 6 carbon atoms, and is linked to the rest moiety of a molecule through a single bound. Examples of $C_2$-$C_6$ alkenyl include, but are not limited to, vinyl, propenyl, allyl, but-1-enyl, but-2-enyl, pent-1-enyl, pent-1,4-dienyl, etc.

The term "$C_3$-$C_6$ cycloalkyl" is to be understood as a saturated monovalent monocyclic or dicyclic hydrocarbon ring that has 3 to 6 carbon atoms and includes a fused and bridged polycyclic system. For example, the $C_3$-$C_6$ cycloalkyl is cyclopropyl, cyclobutyl, cyclopentyl, or cyclohexyl.

The term "four- to eight-membered heterocyclyl" or "four- to eight-membered heterocycloalkyl" is to be understood as a saturated, unsaturated, or partially saturated monocyclic ring, bicyclic ring or tricyclic ring that has 4 to 8 atoms, in which 1, 2, 3, 4, or 5 ring atoms are selected from N, O, or S, and can be linked through carbon or nitrogen unless otherwise indicated, the -$CH_2$- group is optionally substituted by -C(O)-, ring nitrogen atoms or ring sulfur atoms are optionally oxidized to form an N-oxide or S-oxide or ring nitrogen atoms are optionally quaternized unless otherwise indicated, -NH in the ring is optionally substituted with acetyl, formyl, methyl, or methane sulfonyl, and the ring is optionally substituted with one or more halogen atoms. It is to be understood that, when the total number of S atoms and O atoms in the heterocyclyl is greater than 1, these heteroatoms are not adjacent to each other. If the heterocyclyl is a bicyclic ring or tricyclic ring, at least one ring is optionally a heteroaromatic ring or aromatic ring, as long as at least one ring is non-heteroaromatic. When the heterocyclyl is a monocyclic ring, it is non-aromatic. Examples of the heterocyclyl include, but are not limited to, piperidinyl, N-acetylpiperidinyl, N-methylpiperidinyl, N-formylpiperazinyl, N-methanesulfonylpiperazinyl, homopiperazinyl, piperazinyl, azetidinyl, oxetanyl, morpholinyl, tetrahydroisoquinolinyl, tetrahydroquinolinyl, indolinyl, tetrahydropyranyl, dihydro-2H-pyranyl, tetrahydrofuranyl, tetrahydrothiopyranyl, tetrahydrothiopyran-1-oxide, tetrahydrothiopyran-1,1-dioxide, 1H-pyridin-2-one, and 2,5-dioxoimidazolidinyl.

The term "four- to eight-membered heterocycloalkenyl" is to be understood as a non-aromatic monocyclic or polycyclic group that contains 4 to 8 ring atoms, and preferably 5 or 6 ring atoms, and the four- to eight-membered heterocycloalkenyl contains 1 to 3 heteroatoms selected from N, O, S, or P and contains at least one carbon-carbon double bond or carbon-nitrogen double bond. Aza, oxa or thia included in a group name means that at least one nitrogen, oxygen, or sulfur atom respectively serves as a ring atom. Nitrogen or sulfur atoms in the four- to eight-membered heterocycloalkenyl may be optionally oxidized to a corresponding N-oxide, S-oxide, or S-dioxide. Preferred examples of four- to eight-membered heterocycloalkenyl include, but are not limited to, 1,2,3,4-tetrahydropyridyl, 1,2-dihydropyridyl, 1,4-dihydropyridyl, 1,2,3,6-tetrahydropyridyl, 1,4,5,6-tetrahydropyrimidinyl, 2-pyrrolinyl, 3-pyrrolinyl, 2-imidazolinyl, 2-pyrazolinyl, dihydroimidazolyl, dihydrooxazolyl, dihydrooxadiazolyl, dihydrothiazolyl, 3,4-dihydro-2H-pyranyl, dihydrofuranyl, fluorodihydrofuranyl, and oxides thereof. The "four- to eight-membered heterocycloalkenyl" also includes cases where two available hydrogen atoms on the same carbon atom on the ring are substituted with a single group =O at the same time (that is, to form carbonyl).

The term "five- to eight-membered aryl" is to be understood as a monovalent aromatic or partially aromatic monocyclic, dicyclic, or tricyclic hydrocarbon ring that has 5 to 8 carbon atoms, especially a ring having 6 carbon atoms ("$C_6$ aryl"). For example, the five- to eight-membered aryl is phenyl. When the five- to eight-membered aryl is substituted, it may be monosubstituted or multi-substituted. Furthermore, the substitution site is not limited, for example, may be ortho-, para-, or meta-substitution.

The term "five- to eight-membered heteroaryl" is to be understood as a monovalent monocyclic, dicyclic, ortricyclic aromatic ring group that has 5 to 8 ring atoms, especially 5 or 6 carbon atoms, and contains 1 to 5 heteroatoms

independently selected from N, O, or S. Preferably, the five- to eight-membered heteroaryl is a monovalent monocyclic, dicyclic, or tricyclic aromatic ring group that contains 1 to 3 heteroatoms independently selected from N, O, or S, and may be benzofused in each case. Particularly, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, etc.; or pyridyl, pyridazinyl, pyrimidinyl, pyrazinyl, triazinyl, etc.; or cinnolinyl, phthalazinyl, quinazolinyl, quinoxalinyl, naphthyridinyl, pteridyl, carbazolyl, acridinyl, phenazinyl, phenothiazinyl, phenoxazinyl, etc.

The term "halogenated" or "halogen" refers to fluorine, chlorine, bromine, or iodine.

In addition, it is to be noted that, unless otherwise expressly stated, the description mode "... independently" used in the present disclosure is to be understood in a broad sense, which means that the described individuals are independent of each other, and may be independently identical or different specific groups. More specifically, the description mode "... independently" can either mean that in different groups, the specific options expressed by the same symbol do not affect each other, or it can mean that in identical groups, the specific options expressed by the same symbol do not affect each other.

BENEFICIAL EFFECTS

According to the embodiments of the present disclosure, the present disclosure provides a small-molecule CD73 inhibitor with a novel structure, excellent pharmacokinetic properties, and good efficacy or druggability, which can be used for effectively treating a CD73-associated disease or symptom.

The compound of the present disclosure not only has a good inhibitory effect on recombinant human CD73 enzyme and a strong inhibitory activity to CD73 enzyme bound to the surface of A375 cells, but also can significantly relieve AMP-induced proliferation inhibition of $CD4^+$ T cells, with a good in vitro efficacy. The compound of the present disclosure has a relatively high fraction unbound in plasma and shows better druggability compared to a reference compound. In addition, results of pharmacokinetic tests on mice and canine indicate that the compounds of the present disclosure show excellent pharmacokinetic properties, and especially compound 3, compound 4, compound 9, and compound 11 have significantly improved pharmacokinetic properties and good druggability, compared to the reference compounds.

In addition, the compound of the present disclosure has a significant inhibitory effect on the growth of CT-26 colorectal cancer and E.G7-OVA T cell lymphoma when used alone or in combination with PD-1/L1 antibodies, an inhibitory effect of a PD-1 antibody on the growth of A375 melanoma can be significantly improved when the PD-1 antibody is used in combination with compound 1 of the present disclosure, and the synergistic efficacy is more significant compared to the reference compounds.

Results of in vivo efficacy tests indicate that an inhibitory effect of a PD-1 antibody on the growth of A375 melanoma can be significantly improved when the PD-1 antibody is used in combination with the compound of the present disclosure, and the synergistic efficacy of compound 1 is better than that of the reference compounds at the same dose.

Additional aspects and advantages of the present disclosure will be presented in part in the following description, and in part will become apparent from the following description, or may be learned through the practice of the present disclosure.

## BRIEF DESCRIPTION OF DRAWINGS

FIG. 1 shows changes in tumor volume measured at different time points after administration according to embodiments of the present disclosure.

## DESCRIPTION OF EMBODIMENTS

The solutions of the present disclosure will be described below with reference to examples. Those skilled in the art will understand that the following examples are for the purpose of describing the present disclosure rather than limiting the scope of the present disclosure. Examples described without specific techniques or conditions follow the techniques or conditions described in the documents in the art or the product specification. Reagents or instruments used without indicating manufacturers are all conventional products available in the market.

Unless otherwise indicated, structures of the compounds of the present disclosure are determined by nuclear magnetic resonance (NMR) and/or mass spectrometry (MS). The unit of NMR shift is $10^{-6}$ (ppm). A solvent used for NMR measurement is deuterated dimethyl sulfoxide, deuterated chloroform, deuterated methanol or the like, and an internal standard is tetramethylsilane (TMS).

Abbreviations of the present disclosure are defined as follows:

M: molar concentration, for example, 1 M hydrochloric acid means a hydrochloric acid solution at 1 mol/L
DCM: dichloromethane
PCC: pyridinium chlorochromate
DAST: diethylaminosulfur trifluoride
THF: tetrahydrofuran
TEMPO: 2,2,6,6-tetramethylpiperidine oxide
DMSO: dimethyl sulfoxide
LC-MS: liquid chromatography-mass spectrometry
SFC: supercritical fluid chromatography
Flash: flash chromatography
$IC_{50}$: median inhibitory concentration, referring to a concentration reaching half of the maximal inhibitory effect

Test Example 1: preparation of positive reference compound 1

5-(5-((1S,2S)-2-(difluoromethyl)cyclopropyl)-6-methylpyridazin-3 yl)pyrimidine-2,4(1H,3H)-dione (reference compound 1)

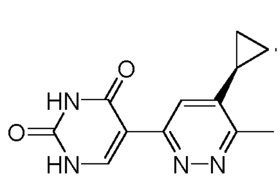

Reference Compound 1

Reference compound 1 was prepared with reference to the method described in the patent WO2019168744A1.
$^1H$ NMR (400 MHz, DMSO-$d_6$) δ 11.51 (s,2H), 8.27 (s, 1H), 7.77 (s, 1H), 6.17-5.85 (m, 1H), 2.69 (s, 3H), 2.32-2.29 (m, 1H), 1.77-1.70(m, 1H), 1.32-1.18 (m, 2H).
LC-MS, M/Z (ESI): 295.0 $[M+H]^+$.
The "reference compound 1" described below refers to the compound described in Test Example 1.

Test Example 2: preparation of positive reference compound 2

5-(6-chloro-5-((1S,2S)-2-(difluoromethyl)cyclopropyl)pyridazin-3-yl)pyrimidine-2,4(1H,3H)-dione (reference compound 2)

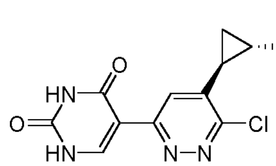

Reference Compound 2

Reference Compound 2 was prepared with reference to the method described in the patent WO2019168744A1.
$^1H$ NMR (400 MHz, CDsOD) δ 8.42 (s, 1H), 8.13 (s, 1H), 5.78-6.08 (m, 1H), 2.48-2.52 (m, 1H), 1.89-1.94 (m, 1H), 1.46-1.51 (m, 1H), 1.33-1.35 (m, 1H).
LC-MS, M/Z (ESI): 315.0 $[M+H]^+$.
The "reference compound 2" described below refers to the compound described in Test Example 2.

Test Example 3: preparation of positive reference compound 3

5-(5-((1S,2R)-2-isopropylcyclopropyl)-6-methylpyridazin-3-yl)pyrimidine-2,4(1H,3H)-dione (reference compound 3)

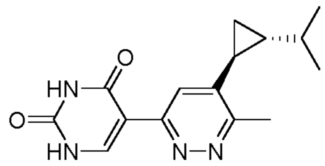

Reference Compound 3

Reference Compound 3 was prepared with reference to the method described in the patent WO2019168744A1.
$^{1}H$ NMR (400 MHz, CDsOD) δ 8.65 (s, 1H), 8.22 (s, 1H), 2.89 (s, 3H), 2.07-2.10 (m, 1H), 1.33-1.45 (m, 4H), 1.10 (d, 6H).
LC-MS, M/Z (ESI): 287.0 $[M+H]^+$.
The "reference compound 3" described below refers to the compound described in Test Example 3.

Example 1: Preparation of target compound 1

5-(6-chloro-5-((1S,2S)-2-(fluoromethyl)cyclopropyl)pyridazin-3-yl)pyrimidine-2,4(1H,3H)-dione (target compound 1)

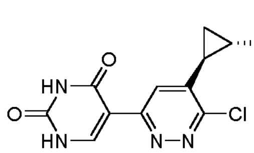

1

The synthesis route of target compound 1 was shown as follows:

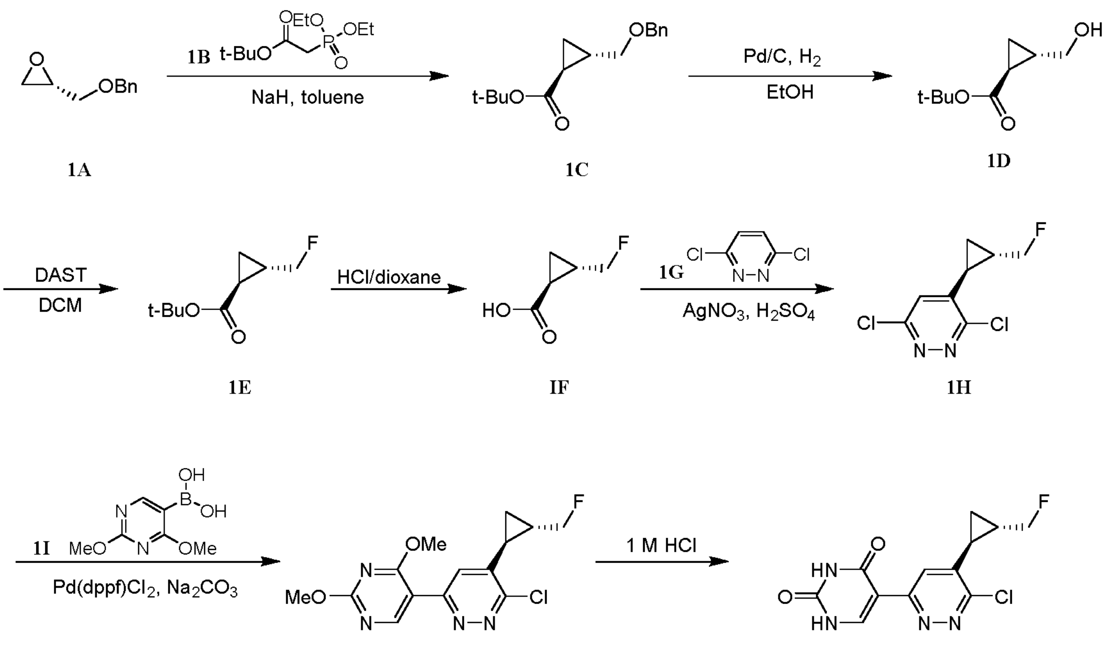


1A 1C 1D 1E IF 1H 1J 1

First step: synthesis of tert-butyl (1S, 2S)-2-((benzyloay)methyl)cyclopropane-1-carboaylate (1C)

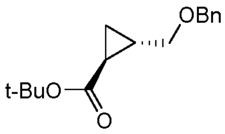

**1C**

Under the protection of nitrogen gas, sodium hydride (14.6 g, 365.4 mmol, content=60%) was suspended in toluene (500 mL), tert-butyl diethylphosphonoacetate (92.2 g, 365.4 mmol) was added dropwise, the reaction solution was stirred at 25°C for 30 min after the dropwise addition was complete, (S)-2-((benzyloxy)methyl)ethylene oxide (50.0 g, 304.5 mmol) was added to the reaction solution, and the reaction solution was heated to 130°C and reacted for 8 h. The reaction mixture was diluted with water (100 mL) and extracted with ethyl acetate (100 mL × 2), and organic phases were combined, washed with a saturated salt solution (50 mL), dried with sodium sulfate, and concentrated to obtain a crude product. The crude product was separated and purified by silica gel column (petroleum ether: ethyl acetate (v/v) = (50: 1) to (10: 1), gradient elution) to obtain a yellow oily compound tert-butyl (1S, 2S)-2-((benzyloay)methyl)cyclopropane-1-carboaylate (1C) (55 g, yield=68.8%).

$^1$H NMR (400 MHz, $CDCl_3$) δ 7.29-7.37 (m, 5H), 4.53 (s, 2H), 3.35-3.46 (m, 2H), 1.66-1.71 (m, 1H), 1.46-1.50 (m, 1H), 1.45 (s, 9H), 1.13-1.17 (m, 1H), 0.79-0.82 (m, 1H).

Second step: synthesis of tert-butyl (1S,2S)-2-(hydroxymethyl)cyclopropane-1-carboxylate (1D)

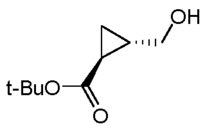

**1D**

Tert-butyl (1S,2S)-2-((benzyloay)methyl)cyclopropane-1-carboaylate (1C) (55 g, 209.6 mmol) was dissolved in ethanol (500 mL), under the protection of nitrogen gas, Pd/C (20.0 g, content=10%) was added, and the reaction solution was subjected to hydrogen gas replacement for 3 times and reacted under 50 Psi at 50°C for 24 h. The reaction solution was cooled to the room temperature and filtered with diatomite to remove Pd/C, an obtained filter cake was washed 3 times with ethanol, and an obtained filtrate was concentrated to obtain a yellow oily compound tert-butyl (1S,2S)-2-(hydroxymethyl)cyclopropane-1-carboxylate (1D) (36.0 g, yield=99.7%).

$^1$H NMR (400 MHz, $CDCl_3$) δ 3.50-3.63 (m, 2H), 1.67-1.72 (m, 1H), 1.47 (s, 9H), 1.38 (t, 1H), 1.14-1.89 (m, 1H), 0.78-0.84 (m, 1H).

Third step: synthesis of tert-butyl (1S,2S)-2-(fluoromethyl)cyclopropane-1-carboaylate (1E)

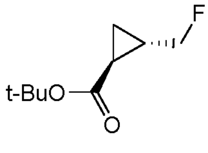

**1E**

Tert-butyl (1S,2S)-2-(hydroxymethyl)cyclopropane-1-carboxylate (1D) (2.5 g, 14.5 mmol) was dissolved in dichloromethane (25 mL), diethylaminosulfur trifluoride (4.68 g, 3.84 mL, 29.0 mmol) was added dropwise at 0°C, and the reaction solution was stirred and reacted at 0°C for 1 h. A saturated sodium bicarbonate aqueous solution (100 mL)

was added to the reaction solution to quench the reaction, the mixture was extracted with dichloromethane (100 mL × 2), and organic phases were combined, washed with a saturated salt solution (50 mL), dried with sodium sulfate, and concentrated to obtain a crude product. The crude product was separated and purified by silica gel column (petroleum ether: ethyl acetate (v/v) = (50: 1) to (10: 1), gradient elution) to obtain a yellow oily compound tert-butyl (1S,2S)-2-(fluoromethyl)cyclopropane-1-carboaylate (1E) (2.0 g, yield=79%).

$^1H$ NMR (400 MHz, $CDCl_3$) δ 4.19-4.42 (m, 2H), 1.75-1.82 (m, 1H), 1.55-1.59 (m, 1H), 1.46 (s, 9H), 1.18-1.23 (m, 1H), 0.83-0.88 (m, 1H).

Fourth step: synthesis of (1S,2S)-2-(fluoromethyl)cyclopropane-1-carboaylic acid (1F)

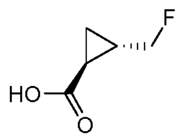

1F

Tert-butyl (1S,2S)-2-(fluoromethyl)cyclopropane-1-carboaylate (1E) (2.0 g, 11.5 mmol) was dissolved in a 1,4-dioxane solution of hydrogen chloride (4 M, 10 mL), and the reaction solution was stirred at 20°C for 1 h. The reaction solution was concentrated to obtain a yellow oily compound (1S,2S)-2-(fluoromethyl)cyclopropane-1-carboaylic acid (1F) (1.3 g, yield=95%)

$^1H$ NMR (400 MHz, $CDCl_3$) δ 4.16-4.52 (m, 2H), 1.88-1.94 (m, 1H), 1.67-1.71 (m, 1H), 1.34-1.37 (m, 1H), 1.01-1.05 (m, 1H).

Fifth step: synthesis of 3,6-dichloro-4-((1S,2S)-2-(fluoromethyl)cyclopropyl)pyridazine (1H)

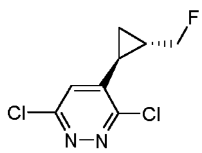

1H

3,6-dichloropyridazine (630 mg, 4.23 mmol) and (1S,2S)-2-(fluoromethyl)cyclopropane-1-carboaylic acid (1F) (500 mg, 4.23 mmol) were dissolved in water, concentrated sulfuric acid (0.5 mL) was added, and under the protection of nitrogen gas, the reaction solution was heated to 70°C. Then, an aqueous solution (359.6 mg, 2.12 mmol, 5 mL) of silver nitrate was added quickly, an aqueous solution (2.90 g, 12.7 mmol, 10 mL) of ammonium persulfate was added slowly dropwise, and the reaction solution reacted at 70°C for 1 h. The reaction solution was regulated with ammonia water until the pH was equal to about 9, and extracted with ethyl acetate (100 mL × 2), and organic phases were combined, washed with a saturated salt solution (50 mL), dried with sodium sulfate, and concentrated to obtain a crude product. The crude product was separated and purified by silica gel column (petroleum ether: ethyl acetate (v/v) = (10: 1) to (3: 1), gradient elution) to obtain a yellow oily compound 3,6-dichloro-4-((1S,2S)-2-(fluoromethyl)cyclopropyl)pyridazine (1H) (500 mg, yield=26%).

Sixth step: synthesis of 3-chloro-6-(2,4-dimethoaypyrimidin-5-yl)-4-((1S,2S)-2-(fluoromethyl)cyclopropyl)pyridazine (1J)

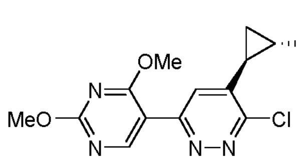

1J

3,6-dichloro-4-((1S,2S)-2-(fluoromethyl)cyclopropyl)pyridazine (300 mg, 1.36 mmol) and 2,4-dimethoxypyrimidine-5-boric acid were dissolved in 1,4-dioxane (10 mL) and water (2 mL), sodium carbonate (359.6 mg, 3.39 mmol) and [1,1'-bis(diphenylphosphino)ferrocene]dichloropalladium(II) (99.3 mg, 135.7 μmol) were added under the protection of nitrogen gas, and the reaction solution was heated to 70°C and reacted for 2 h. The reaction mixture was diluted with water (50 mL) and extracted with ethyl acetate (50 mL × 2), and organic phases were combined, washed with a saturated salt solution (50 mL), dried with sodium sulfate, and concentrated to obtain a crude product. The crude product was separated by reversed phase high-performance liquid chromatography (chromatographic column: Phenomenex Luna C18 (150 mm × 25 mm, 10 μm); mobile phases: A: water + 0.01 vol% of trifluoroacetic acid (99%), B: acetonitrile; gradient: 35%-65% of B, 10 min) to obtain a yellow solid 3-chloro-6-(2,4-dimethoaypyrimidin-5-yl)-4-((1S,2S)-2-(fluoromethyl)cyclopropyl)pyridazine (1J) (150 mg, yield=34%).

$^1$H NMR (400 MHz, $CDCl_3$) δ 9.06 (s, 1H), 7.55 (s, 1H), 4.45-4.58 (m, 2H), 4.10 (s, 3H), 4.08 (s, 3H), 2.24-2.29 (m, 1H), 1.65-1.69 (m, 1H), 1.31-1.35 (m, 1H), 1.19-1.23 (m, 1H)
LC-MS, M/Z (ESI): 324.9 [M+H]$^+$.

Seventh step: synthesis of 5-(6-chloro-5-((1S,2S)-2-(fluoromethyl)cyclopropyl)pyridazin-3-yl)pyrimidine-2,4(1H,3H)-dione (1)

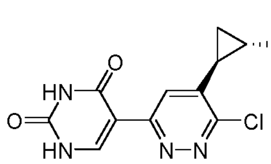

1

3-chloro-6-(2,4-dimethoaypyrimidin-5-yl)-4-((1S,2S)-2-(fluoromethyl)cyclopropyl)pyridazine (150 mg, 461.9 μmol) was dissolved in a hydrochloric acid aqueous solution (1 M, 10 mL), and the reaction solution was heated to 75°C and reacted for 2 h. The reaction solution was cooled to the room temperature to precipitate a solid and filtered, and the solid was collected and dried to obtain a yellow solid 5-(6-chloro-5-((1S,2S)-2-(fluoromethyl)cyclopropyl)pyridazin-3-yl)pyrimidine-2,4(1H,3H)-dione (1) (60 mg, yield=42%).
$^1$H NMR (400 MHz, DMSO-$d_6$) δ 8.30 (d, 1H), 7.96 (s, 1H), 4.39-4.61 (m, 2H), 2.20-2.24 (m, 1H), 1.68-1.72 (m, 1H), 1.19-1.28 (m, 2H).
LC-MS, M/Z (ESI): 297.1 [M+H]$^+$.

Example 2: Preparation of target compound 2

5-(6-chloro-5-(trans-2-(trifluoromethyl)cyclopropyl)pyridazin-3-yl)pyrimidine-2,4(1H,3H)-dione (target compound 2)

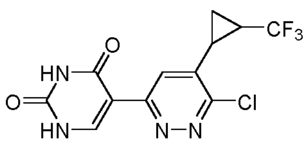

trans racemic mixtures
2

The synthesis route of target compound 2 was shown as follows:

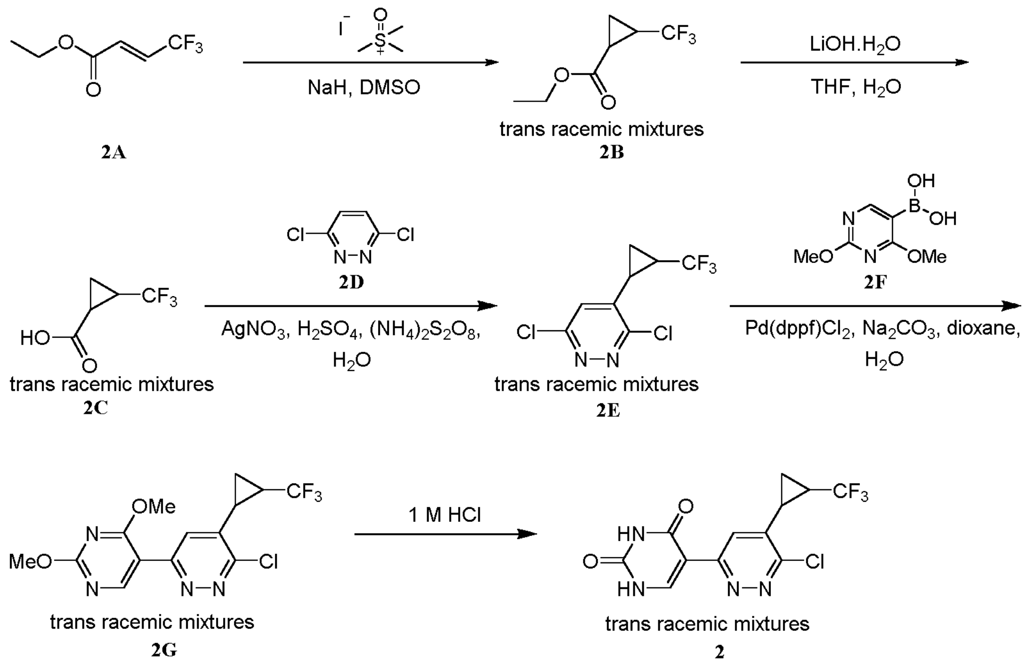


First step: synthesis of ethyl trans-2-(trifluoromethyl)cyclopropane-1-carboaylate (2B)

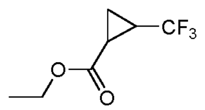

trans racemic mixtures
2B

Under the protection of nitrogen gas, sodium hydride (2.62 g, 65.4 mmol, content=60%) was added slowly in batches to a dimethyl sulfoxide (60 mL) solution of trimethylsulfoxide iodide (14.4 g, 65.4 mmol), and the reaction solution was stirred at 25°C for 30 min. Then, a dimethyl sulfoxide (30 mL) solution of ethyl 4,4,4-trifluorobut-2-enoate (10 g, 59.5 mmol) was added dropwise to the reaction solution, and the reaction solution was stirred at 25°C for 30 min. The reaction solution was poured into an ammonium chloride aqueous solution (500 mL), the mixture was stirred for 20 min and extracted with petroleum ether (200 mL × 2), and organic phases were combined, washed with a saturated salt solution (200 mL), dried with sodium sulfate, and concentrated to obtain a yellow oily compound ethyl trans-2-(trifluoromethyl)cyclopropane-1-carboaylate (2B) (5.00 g, crude product).

$^1H$ NMR (400 MHz, $CDCl_3$) δ 4.17-4.19 (m, 2H), 2.00-2.10 (m, 1H), 1.98-1.99 (m, 1H), 1.10-1.20 (m, 2H), 0.88-0.91 (m, 3H).

Second step: synthesis of hans-2-(trifluoromethyl)cyclopropane-1-carboaylic acid (2C)

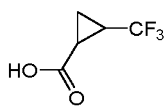

trans racemic mixtures
2C

Ethyl trans-2-(trifluoromethyl)cyclopropane-1-carboaylate (5 g, 27.5 mmol) was dissolved in tetrahydrofuran (50 mL) and water (25 mL), lithium hydroxide monohydrate (2.88 g, 68.6 mmol) was added in batches, and the reaction solution was heated to 80°C and reacted for 6 h. The reaction solution was spin-dried, water (50 mL) was added, and the mixture was extracted with ethyl acetate (50 mL × 2). A separated aqueous phase was regulated with a 2 M hydrochloric acid aqueous solution until the pH was equal to 3, and extracted with ethyl acetate (50 mL × 2), and organic phases were combined, washed with a saturated salt solution (100 mL), dried with sodium sulfate, and concentrated to obtain a yellow oily compound trans-2-(trifluoromethyl)cyclopropane-1-carboaylic acid (2C) (1.5 g, yield=35.5%).

$^1$H NMR (400 MHz, $CDCl_3$) δ 11.09 (br.s, 1H), 2.16-2.23 (m, 1H), 2.02-2.05 (m, 1H), 1.33-1.43 (m, 2H).

Third step: synthesis of 3,6-dichloro-4-(trans-2-(trifluoromethyl)cyclopropyl)pyridazine (2E)

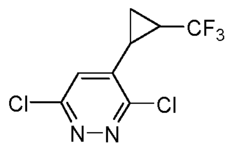

trans racemic mixtures

**2E**

3,6-dichloropyridazine (1.2 g, 8.05 mmol) and trans-2-(trifluoromethyl)cyclopropane-1-carboxylic acid (1.24 g, 8.05 mmol) were dissolved in water (40 mL), concentrated sulfuric acid (1.2 mL) was added, and under the protection of nitrogen gas, the reaction solution was heated to 70°C. Then, an aqueous solution (3.68 g, 21.7 mmol, 5 mL) of silver nitrate was added, an aqueous solution (5.51 g, 24.2 mmol, 20 mL) of ammonium persulfate was added slowly dropwise, and the reaction solution reacted at 70°C for 1 h. The reaction solution was regulated with ammonia water until the pH was equal to about 9, and extracted with ethyl acetate (200 mL × 2), and organic phases were combined, washed with a saturated salt solution (300 mL), dried with sodium sulfate, and concentrated to obtain a crude product. The crude product was purified by column separation (petroleum ether: ethyl acetate (v/v) = 10: 1, gradient elution) to obtain a yellow oily compound 3,6-dichloro-4-(trans-2-(trifluoromethyl)cyclopropyl)pyridazine (2E) (650 mg, yield=19.2%).

LC-MS, M/Z (ESI): 257.0 $[M+H]^+$.

Fourth step: synthesis of 3-chloro-6-(2,4-dimethoxypyrimidin-5-yl)-4-(trans-2-(trifluoromethyl)cyclopropyl)pyridazine (2G)

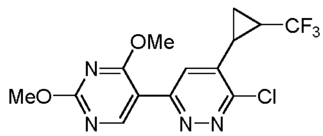

trans racemic mixtures

**2G**

3,6-dichloro-4-(trans-2-(trifluoromethyl)cyclopropyl)pyridazine (500 mg, 1.39 mmol) and 2,4-dimethoxypyrimidine-5-boric acid (255.2 mg, 1.39 mmol) were dissolved in 1,4-dioxane (15 mL) and water (5 mL), sodium carbonate (367.5 mg, 3.47 mmol) and [1,1'-bis(diphenylphosphino)ferrocene]dichloropalladium(II) (101.5 mg, 138.7 μmol) were added under the protection of nitrogen gas, and the reaction solution was heated to 50°C and reacted for 12 h. The reaction mixture was diluted with water (50 mL) and extracted with ethyl acetate (50 mL × 2), and organic phases were combined, washed with a saturated salt solution (50 mL), dried with sodium sulfate, and concentrated to obtain a crude product. The crude product was separated and purified by silica gel column (petroleum ether: ethyl acetate (v/v) = (50: 1) to (5: 1), gradient elution) to obtain a yellow oily compound 3-chloro-6-(2,4-dimethoxypyrimidin-5-yl)-4-(trans-2-(trifluoromethyl)cyclopropyl)pyridazine (2G) (300 mg, yield=33.6%).

LC-MS, M/Z (ESI): 361.1 $[M+H]^+$.

Fifth step: synthesis of 5-(6-chloro-5-(trans-2-(trifluoromethyl)cyclopropyl)pyridazin-3-yl)pyrimidine-2,4(1H,3H)-dione (2)

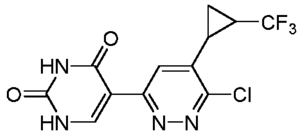

trans racemic mixtures

2

3-chloro-6-(2,4-dimethoxypyrimidin-5-yl)-4-(trans-2-(trifluoromethyl)cyclopropyl)pyridazine (300 mg, 560 μmol) was dissolved in a hydrochloric acid aqueous solution (1 M, 8.32 mL), and the reaction solution was heated to 50°C and reacted for 12 h. The reaction solution was cooled to 20°C to precipitate a solid and filtered, and the solid was collected. The solid was added to methanol (10 mL), and the mixture was stirred at 50°C for 1 h, filtered, and dried to obtain a white solid 5-(6-chloro-5-(trans-2-(trifluoromethyl)cyclopropyl)pyridazin-3-yl)pyrimidine-2,4(1H,3H)-dione (2) (63.7 mg, yield=33.2%).

$^1$H NMR (400 MHz, DMSO-*d*$_6$) δ 8.36 (s, 1H), 8.13 (s, 1H), 2.53-2.58 (m, 1H), 2.37-2.47 (m, 1H), 1.51-1.56 (m, 1H), 1.43-1.48 (m, 1H).

LC-MS, M/Z (ESI): 333.0[M+H]$^+$.

Example 3: preparation of target compounds 3 and 4

5-(6-chloro-5-((1S,2S)-2-(trifluoromethyl)cyclopropyl)pyridazin-3-yl)pyrimidine-2,4(1H,3H)-dione (target compound 3)

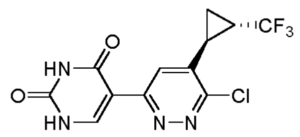

3

5-(6-chloro-5-((1R,2R)-2-(trifluoromethyl)cyclopropyl)pyridazin-3-yl)pyrimidine-2,4(1H,3H)-dione (target compound 4)

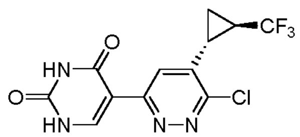

Preparation of target compounds 3 and 4 was as follows:

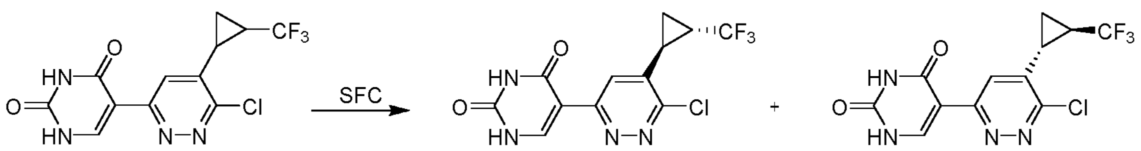


trans racemic mixtures

2

3

4

First step: preparation of 5-(6-chloro-5-((1S,2S)-2-(trifluoromethyl)cyclopropyl)pyridazin-3-yl)pyrimidine-2,4(1H,3H)-dione (3) and 5-(6-chloro-5-((1R,2R)-2-(trifluoromethyl)cyclopropyl)pyridazin-3-yl)pyrimidine-2,4(1H,3H)-dione (4)

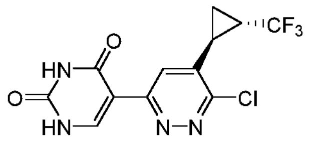

3

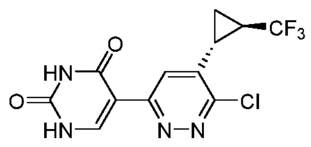

4

5-(6-chloro-5-(trans-2-(trifluoromethyl)cyclopropyl)pyridazin-3-yl)pyrimidine-2,4(1H,3H)-dione (900 mg, 2.49 mmol) was dissolved in dimethyl sulfoxide (20 mL), and separated by supercritical fluid chromatography (chromatographic column: DAICEL CHIRALPAK IG (250 mm × 30 mm, 10μm); mobile phases: A: $CO_2$, B: 0.05% diethylamine ethanol solution; gradient: 50% of B, time: 60 min; flow rate: 3 mL/min; column temperature: 35°C; column pressure: 100 Bar) to obtain white solids 5-(6-chloro-5-((1S,2S)-2-(trifluoromethyl)cyclopropyl)pyridazin-3-yl)pyrimidine-2,4(1H,3H)-dione (3) (360.4 mg, yield=43.4%) and 5-(6-chloro-5-((1R,2R)-2-(trifluoromethyl)cyclopropyl)pyridazin-3-yl)pyrimidine-2,4(1H,3H)-dione (4) (332.5 mg, yield=39.9%).

Compound 3: SFC retention time Rt = 0.743 min, ee% = 100%, detection method: chromatographic column: Chiralpak IG-3 (50 mm × 4.6 mm I.D., 3 μm); mobile phases: A: $CO_2$, B: 0.05% diethylamine ethanol solution; gradient: 40% of B; flow rate: 3 mL/min; column temperature: 35°C; column pressure: 100 Bar.

$^1$H NMR (400 MHz, CDsOD) δ 8.42 (s, 1H), 8.17 (s, 1H), 2.60-2.65 (m, 1H), 2.22-2.29 (m, 1H), 1.47-1.62 (m, 2H).

LC-MS, M/Z (ESI): 333.0 $[M+H]^+$.

Compound 4: SFC retention time Rt = 1.031 min, ee% = 99.3%, detection method: chromatographic column: Chiralpak IG-3 (50 mm × 4.6 mm I.D., 3 μm); mobile phases: A: $CO_2$, B: 0.05% diethylamine ethanol solution; gradient: 40% of B; flow rate: 3 mL/min; column temperature: 35°C; column pressure: 100 Bar.

$^1$H NMR (400 MHz, CDsOD) δ 8.42 (s, 1H), 8.17 (s, 1H), 2.60-2.64 (m, 1H), 2.24-2.28 (m, 1H), 1.48-1.62 (m, 2H).

LC-MS, M/Z (ESI): 333.0 $[M+H]^+$.

Example 4: preparation of target compound 3

5-(6-chloro-5-((1S,2S)-2-(trifluoromethyl)cyclopropyl)pyridazin-3yl)pyrimidine-2,4(1H,3H)-dione (target compound 3)

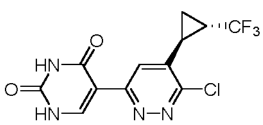

3

The synthesis route of target compound 3 was shown as follows:

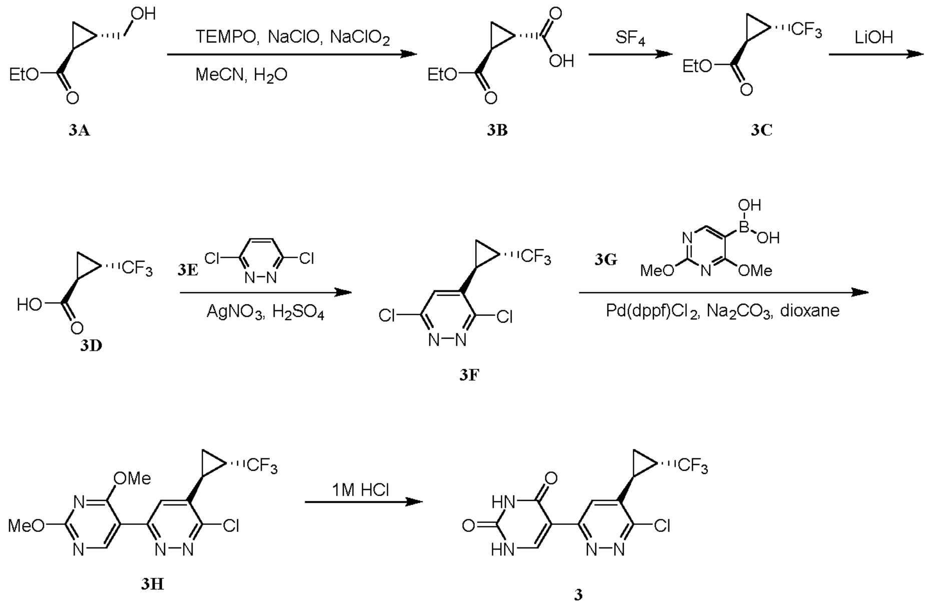


3A 3B 3C 3D 3F 3H 3

First step: synthesis of (1S,2S)-2-(ethoaycarbonyl)cyclopropanecarboxylic acid (3B)

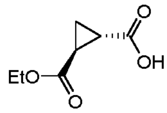

3B

Ethyl (1S,2S)-2-(hydroaymethyl)cyclopropanecarboaylate (5.0 g, 34.7 mmol) was dissolved in acetonitrile (50 mL), and 2,2,6,6-tetramethylpiperidine oxide (436.3 mg, 2.8 mmol), sodium dihydrogen phosphate (6.66 g, 55.5 mmol), and disodium hydrogen phosphate (7.88 g, 55.5 mmol) were added in sequence at 25°C. Then, a sodium hypochlorite solution (0.5 mL) and sodium chlorite (6.27 g, 69.4 mmol) were dissolved in water (25 mL), the solution was added slowly dropwise to the reaction system at 0°C, and the reaction solution was stirred at 25°C for 12 h. The reaction system was diluted with water (100 mL) and extracted with ethyl acetate (100 mL × 2), organic phases were combined, a saturated sodium carbonate aqueous solution (100 mL) was added, and the mixture was stirred for 10 min. The organic phase was separated, an obtained aqueous phase was regulated with a 6 M hydrochloric acid solution until the pH was equal to 2 to 3, and extracted with ethyl acetate (100 mL × 2), and organic phases were combined, washed with a saturated salt solution (100 mL), dried with sodium sulfate, and concentrated to obtain a colorless oily compound (1S,2S)-2-(ethoaycarbonyl)cyclopropanecarboxylic acid (3B) (4.8 g, yield=87.5%).

$^1H$ NMR (400 MHz, $CDCl_3$) δ 10.34 (br.s, 1H), 4.14 (q, 2H), 2.11-2.22 (m, 2H), 1.43-1.50 (m, 2H), 1.25 (t, 3H).

Second step: synthesis of ethyl (1S,2S)-2-(trifluoromethyl)cyclopropanecarboxylate (3C)

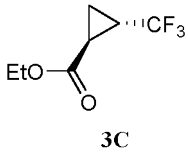

3C

(1S,2S)-2-(ethoxycarbonyl)cyclopropanecarboxylic acid (3.0 g, 19.0 mmol) was placed in a high-pressure autoclave, sulfur tetrafluoride (9.0 g, 83.3 mmol) was added at -78°C, and the reaction system was heated to 70°C in the high-pressure autoclave and reacted for 16 h. Dichloromethane (20 mL) was added to the reaction system, and an obtained organic phase was washed with a saturated sodium bicarbonate aqueous solution (500 mL), dried with sodium

sulfate, and concentrated to obtain a yellow oily compound ethyl (1S,2S)-2-(trifluoromethyl)cyclopropanecarboxylate (3C) (1.17 g, yield=33.9%).

$^1$H NMR (400 MHz, $CDCl_3$) δ 4.17-4.19 (m, 2H), 2.10-2.20 (m, 1H), 2.00-2.05 (m, 1H), 1.20-1.40 (m, 5H).

Third step: synthesis of (1S,2S)-2-(trifluoromethyl)cyclopropanecarboxylic acid (3D)

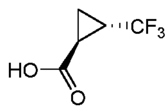

3D

Ethyl (1S,2S)-2-(trifluoromethyl)cyclopropanecarboxylate (1.1 g, 6.0 mmol) was dissolved in tetrahydrofuran (10 mL) and water (5 mL), lithium hydroxide monohydrate (634 mg, 15.1 mmol) was added, and the reaction solution reacted at 80°C for 6 h. After the reaction was completed, water (20 mL) was added, the mixture was extracted with dichloromethane (30 mL × 2), aqueous phases were collected, and the combined aqueous phase was regulated with 6M hydrochloric acid unitl the pH was equal to 3, and then extracted with dichloromethane (30 mL × 3). Organic phases were combined, washed with a saturated salt solution (50 mL), dried with anhydrous sodium sulfate, filtered, and concentrated to obtain a brown oily compound (1S,2S)-2-(trifluoromethyl)cyclopropanecarboxylic acid (3D) (500 mg, yield=53.7%).

$^1$H NMR (400 MHz, $CDCl_3$) δ 9.80 (br.s, 1H), 2.20-2.23 (m, 1H), 2.04-2.06 (m, 1H), 1.27-1.44 (m, 2H).

Fourth step: synthesis of 3,6-dichloro-4-((1S,2S)-2-(trifluoromethyl)cyclopropyl)pyridazine (3F)

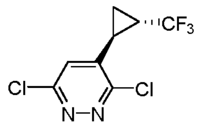

3F

3,6-dichloropyridazine (450 mg, 3.02 mmol) and (1S,2S)-2-(trifluoromethyl)cyclopropanecarboxylic acid (465 mg, 3.02 mmol) were dissolved in water (15 mL), concentrated sulfuric acid (0.5 mL) was added, and under the protection of nitrogen gas, the reaction solution was heated to 70°C. Then, an aqueous solution (257 mg, 1.51 mmol, 1.5 mL) of silver nitrate was added quickly, and an aqueous solution (2.07 g, 9.06 mmol, 5 mL) of ammonium persulfate was added slowly dropwise, and the reaction solution reacted at 70°C for 1 h. The reaction solution was regulated with ammonia water until the pH was equal to about 9, and extracted with ethyl acetate (40 mL × 2), and organic phases were combined, washed with a saturated salt solution (50 mL), dried with sodium sulfate, and concentrated to obtain a crude product. Then, the crude product was separated by reversed phase high-performance liquid chromatography (chromatographic column: Phenomenex luna C18 (150 mm × 40 mm, 15 μm); mobile phases: A: water + 0.1 vol% of TFA, B: acetonitrile; gradient: 35%-65% of B, 10 min) to obtain a yellow oily compound 3,6-dichloro-4-((1S,2S)-2-(trifluoromethyl)cyclopropyl)pyridazine (3F) (350 mg, yield=43.8%).

LC-MS, M/Z (ESI): 256.9 $[M+H]^+$.

Fifth step: synthesis of 3-chloro-6-(2,4-dimethoxypyrimidin-5-yl)-4-((1S,2S)-2-(trifluoromethyl)cyclopropyl)pyridazine (3H)

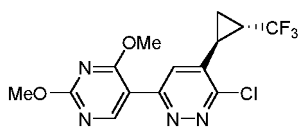

3H

3,6-dichloro-4-((1S,2S)-2-(trifluoromethyl)cyclopropyl)pyridazine (350 mg, 1.32 mmol) and 2,4-dimethoxypyrimidine-5-boric acid (343 mg, 1.32 mmol) were dissolved in dioxane (5 mL) and water (1 mL), sodium carbonate (420 mg, 3.96 mmol) and [1,1'-bis(diphenylphosphino)ferrocene]dichloropalladium(II) (97 mg, 132 μmol) were added under the protection of nitrogen gas, and the reaction solution was heated to 50°C and reacted for 12 h. The reaction solution was diluted with water (20 mL) and extracted with ethyl acetate (20 mL × 2), and organic phases were combined, washed with a saturated salt solution (50 mL), dried with sodium sulfate, and concentrated to obtain a crude product. The crude product was separated and purified by silica gel column (petroleum ether: ethyl acetate (v/v) = (50: 1) to (3: 1), gradient elution) to obtain a yellow oily compound 3-chloro-6-(2,4-dimethoaypyrimidin-5-yl)-4-((1S,2S)-2-(trifluoromethyl)cyclopropyl)pyridazine (3H) (300 mg, yield=44%).

LC-MS, M/Z (ESI): 361.0 [M+H]+.

Sixth step: synthesis of 5-(6-chloro-5-((1S,2S)-2-(trifluoromethyl)cyclopropyl)pyridazin-3-yl)pyrimidine-2,4(1H,3H)-dione (3)

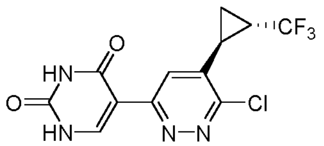

3

3-chloro-6-(2,4-dimethoxypyrimidin-5-yl)-4-((1S,2S)-2-(trifluoromethyl)cyclopropyl)pyridazine (30 mg, 58 μmol) was dissolved in a hydrochloric acid aqueous solution (1 M, 1 mL), and the reaction solution was heated to 50°C and reacted for 12 h. The reaction solution precipitated and was filtered to obtain a white solid 5-(6-chloro-5-((1S,2S)-2-(trifluoromethyl)cyclopropyl)pyridazin-3-yl)pyrimidine-2,4-dione (3) (10.04 mg, yield=50%).

SFC retention time Rt = 0.743 min, ee% = 100%, detection method: chromatographic column: Chiralpak IG-3 (50 mm × 4.6 mm I.D., 3 μm); mobile phases: A: $CO_2$, B: 0.05% diethylamine ethanol solution; gradient: 40% of B; flow rate: 3 mL/min; column temperature: 35°C; column pressure: 100 Bar.

$^1$H NMR (400 MHz, CDsOD) δ 8.42 (s, 1H), 8.17 (s, 1H), 2.60-2.64 (m, 1H), 2.24-2.28 (m, 1H), 1.48-1.62 (m, 2H).

LC-MS, M/Z (ESI): 333.0 [M+H]+.

Absolute configurations of 5-(6-chloro-5-((1S,2S)-2-(trifluoromethyl)cyclopropyl)pyridazin-3-yl)pyrimidine-2,4(1H,3H)-dione (compound 3) and 5-(6-chloro-5-((1R,2R)-2-(trifluoromethyl)cyclopropyl)pyridazin-3-yl)pyrimidine-2,4(1H,3H)-dione (compound 4) of Example 3 could be identified by matching the SFC retention time of target compound 3 provided in the present example.

Example 5: Preparation of target compound 5

5-(6-chloro-5-((1S,2S)-2-(2-hydroxypropan-2-yl)cyclopropyl)pyridazin-3-yl)pyrimidine-2,4(1H,3H)-dione (target compound 5)

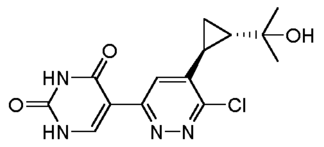

5

The synthesis route of target compound 5 was shown as follows:

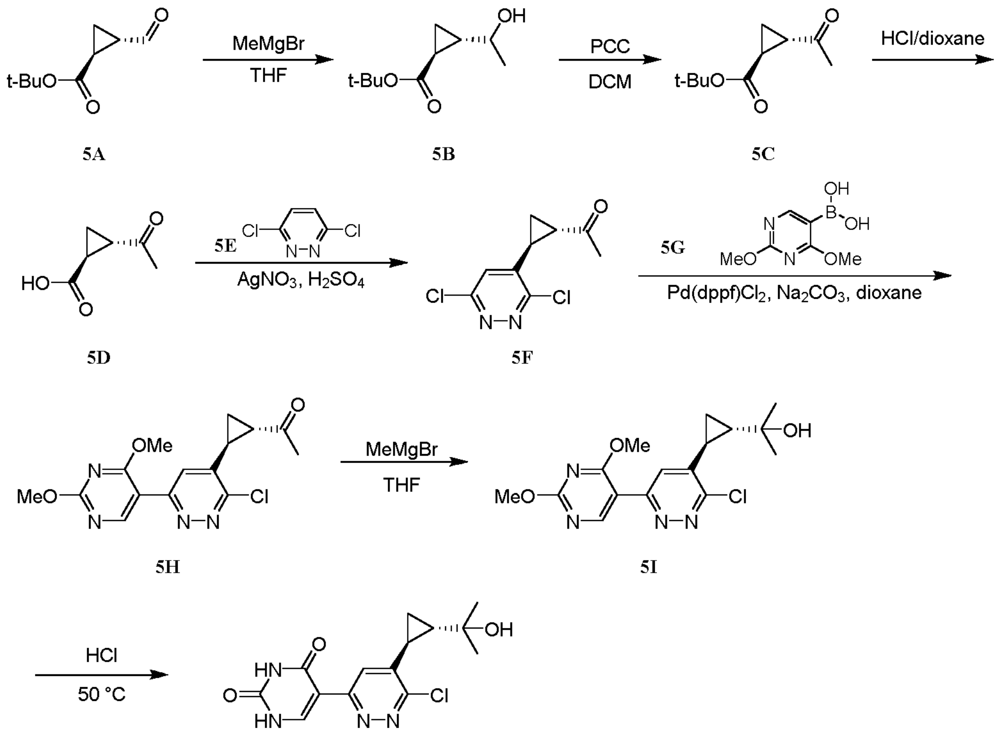


5A 5B 5C 5D 5F 5H 5I 5

First step: synthesis of tert-butyl (1S,2S)-2-((S)-1-hydroxyethyl)cyclopropane-1-carboxylate (5B)

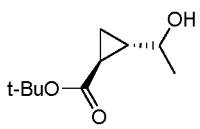

5B

Tert-butyl (1S,2S)-2-formylcyclopropane-1-carboxylate (5.00 g, 29.4 mmol) was dissolved in tetrahydrofuran (50 mL), methylmagnesium bromide (3 M, 19.6 mL) was added dropwise at 20°C, and the reaction solution reacted at 20°C for 2 h after the dropwise addition was complete. After the reaction was completed, methanol (3 mL) was added to quench the reaction, water (100 mL) was added, the mixture was extracted with ethyl acetate (100 mL × 2), and organic phases were combined, washed with a saturated salt solution (50 mL), dried with sodium sulfate, and concentrated to obtain a yellow oily compound tert-butyl (1S,2S)-2-((S)-1-hydroxyethyl)cyclopropane-1-carboxylate (5B) (3.84 g, yield=70.2%) that was directly used at the next step.

Second step: synthesis of tert-butyl (1S,2S)-2-acetylcyclopropane-1-carboxylate (5C)

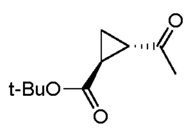

5C

Tert-butyl (1S,2S)-2-((S)-1-hydroxyethyl)cyclopropane-1-carboxylate (5B) (3.84 g, 20.6 mmol) was dissolved in dichloromethane (50 mL), pyridine chlorochromate (13.3 g, 61.8 mmol) was added, and the reaction solution reacted

at 20°C for 2 h. After the reaction was completed, the reaction solution was filtered with diatomite, an obtained filter cake was washed twice with dichloromethane (50 mL), and organic phases were combined and concentrated to obtain a crude product. The crude product was separated and purified by silica gel column (petroleum ether: ethyl acetate (v/v) = (10: 1) to (5: 1), gradient elution) to obtain a yellow oily compound tert-butyl (1S,2S)-2-acetylcyclopropane-1-carboxylate (5C) (2.60 g, yield=68.5%).

$^1H$ NMR (400 MHz, $CDCl_3$) δ 2.37-2.42 (m, 1H), 2.30 (s, 3H), 2.08-2.12 (m, 1H), 1.46 (s, 9H), 1.34-1.38 (m, 2H).

Third step: synthesis of (1S,2S)-2-acetylcyclopropane-1-carboxylic acid (5D)

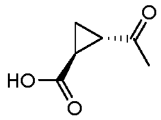

5D

Tert-butyl (1S,2S)-2-acetylcyclopropane-1-carboxylate (5C) (2.60 g, 14.1 mmol) was dissolved in a 1,4-dioxane solution (25 mL) of hydrogen chloride (4 M), and the reaction solution reacted at 20°C for 1 h. After the reaction was completed, the reaction solution was concentrated to obtain a yellow oily compound (1S,2S)-2-acetylcyclopropane-1-carboxylic acid (5D) (1.80 g, yield=99%) that was directly used at the next step.

$^1H$ NMR (400 MHz, $CDCl_3$) δ 2.50-2.55 (m, 1H), 2.33 (s, 3H), 2.17-2.21 (m, 1H), 1.47-1.51 (m, 2H).

Fourth step: synthesis of 1-((1S,2S)-2-(3,6-dichloropyridazin-4-yl)cyclopropyl)ethan-1-one (5F)

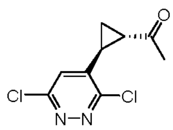

5F

3,6-dichloropyridazine (581.4 mg, 3.90 mmol) and (1S,2S)-2-acetylcyclopropane-1-carboxylic acid (5D) (500 mg, 3.90 mmol) were dissolved in water (20 mL), concentrated sulfuric acid (0.5 mL) was added, and under the protection of nitrogen gas, the reaction solution was heated to 70°C. Then, an aqueous solution (331.5 mg, 1.95 mmol, 5 mL) of silver nitrate was added quickly, an aqueous solution (2.67 g, 11.7 mmol, 10 mL) of ammonium persulfate was added slowly dropwise, and the reaction solution reacted at 70°C for 1 h. After the reaction was completed, the reaction solution was regulated with ammonia water until the pH was equal to about 9, and extracted with ethyl acetate (50 mL × 2), and organic phases were combined, washed with a saturated salt solution (25 mL), dried with sodium sulfate, and concentrated to obtain a crude product. Then, the crude product was separated by reversed phase high-performance liquid chromatography (chromatographic column: 3_Phenomenex Luna C18 (75 mm × 30 mm, 3 μm); mobile phases: A: water + 0.05 vol% of HCl (36.5%), B: acetonitrile; gradient: 22%-42% of B, 8 min) to obtain a yellow oily compound 1-((1S,2S)-2-(3,6-dichloropyridazin-4-yl)cyclopropyl)ethan-1-one (5F) (200 mg, yield=22.2%).

LC-MS, M/Z (ESI): 231.1 $[M+H]^+$.

Fifth step: synthesis of 1-((1S,2S)-2-(3-chloro-6-(2,4-dimethoxypyrimidin-5-yl)pyridazin-4-yl)cyclopropyl)ethan-1-one (5H)

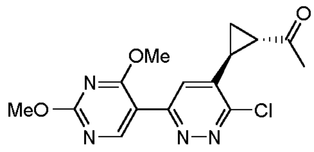

5H

1-((1S,2S)-2-(3,6-dichloropyridazin-4-yl)cyclopropyl)ethan-1-one (185 mg, 0.80 mmol) and 2,4-dimethoxypyrimidine-5-boric acid (154.6 mg, 0.84 mmol) were dissolved in 1,4-dioxane (5 mL) and water (1 mL), sodium carbonate (212.1 mg, 2.00 mmol) and [1,1'-bis(diphenylphosphino)ferrocene]dichloropalladium(II) (58.6 mg, 80.1 μmol) were added under the protection of nitrogen gas, and the reaction solution was heated to 70°C and reacted for 2 h. The reaction solution was diluted with water (50 mL) and extracted with ethyl acetate (50 mL × 2), and organic phases were combined, washed with a saturated salt solution (50 mL), dried with sodium sulfate, and concentrated to obtain a crude product. The crude product was separated and purified by silica gel column (petroleum ether: ethyl acetate (v/v) = (10: 1) to (1: 1), gradient elution) to obtain a yellow solid 1-((1S,2S)-2-(3-chloro-6-(2,4-dimethoxypyrimidin-5-yl)pyridazin-4-yl)cyclopropyl)ethan-1-one (5H) (150 mg, yield=56%).

LC-MS, M/Z (ESI): 335.2 $[M+H]^+$.

Sixth step: synthesis of 2-((1S,2S)-2-(3-chloro-6-(2,4-dimethoxypyrimidin-5-yl)pyridazin-4-yl)cyclopropyl)propan-2-ol (5I)

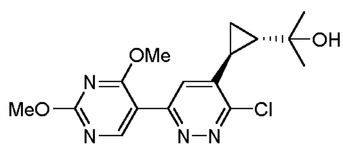

5I

1-((1S,2S)-2-(3-chloro-6-(2,4-dimethoxypyrimidin-5-yl)pyridazin-4-yl)cyclopropyl)ethan-1-one (5H) (100 mg, 0.29 mmol) was dissolved in tetrahydrofuran (5 mL), methylmagnesium bromide (3 M, 0.2 mL) was added dropwise at 0°C, and the reaction solution reacted at 20°C for 1 h after the dropwise addition was complete. After the reaction was completed, methanol (1 mL) was added to quench the reaction, water (25 mL) was added, the mixture was extracted with ethyl acetate (25 mL × 2), and organic phases were combined, washed with a saturated salt solution (25 mL), dried with sodium sulfate, and concentrated to obtain a crude product. Then, the crude product was separated by reversed phase high-performance liquid chromatography (chromatographic column: 3_Phenomenex Luna C18 (75 mm × 30 mm, 3 μm); mobile phases: A: water + 0.05 vol% of HCl (36.5%), B: acetonitrile; gradient: 27%-47% of B, 8.5 min) to obtain a yellow oily compound 2-((1S,2S)-2-(3-chloro-6-(2,4-dimethoxypyrimidin-5-yl)pyridazin-4-yl)cyclopropyl)propan-2-ol (5I) (20 mg, yield=12.7%).

LC-MS, M/Z (ESI): 351.3 $[M+H]^+$.

Seventh step: synthesis of 5-(6-chloro-5-((1S,2S)-2-(2-hydroxypropan-2-yl)cyclopropyl)pyridazin-3-yl)pyrimidine-2,4(1H,3H)-dione (target compound 5)

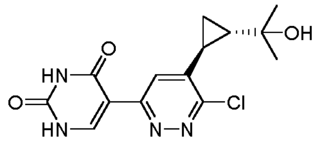

5

2-((1S,2S)-2-(3-chloro-6-(2,4-dimethoxypyrimidin-5-yl)pyridazin-4-yl)cyclopropyl)propan-2-ol (5I) (20 mg, 57.0 μmol) was dissolved in a hydrochloric acid aqueous solution (1 M, 2 mL), and the reaction solution was heated to 50°C

and reacted for 12 h. The reaction solution was concentrated and then separated by reversed phase high-performance liquid chromatography (chromatographic column: 3_Phenomenex Luna C18 (75 mm × 30 mm, 3 μm); mobile phases: A: water + 0.05 vol% of HCl (36.5%), B: acetonitrile; gradient: 11%-31% of B, 7.5 min) to obtain a white solid compound 5-(6-chloro-5-((1S,2S)-2-(2-hydroxypropan-2-yl)cyclopropyl)pyridazin-3-yl)pyrimidine-2,4(1H,3H)-dione (5) (10 mg, yield=49%).

$^1$H NMR (400 MHz, CDsOD) δ 8.53 (s, 1H), 8.06 (s, 1H), 2.38-2.42 (m, 1H), 1.57-1.62 (m, 1H), 1.48-1.51 (m, 1H), 1.35 (s, 3H), 1.34 (s, 3H), 1.22-1.28 (m, 1H).

LC-MS, M/Z (ESI): 323.3 [M+H]$^+$.

Example 6: Preparation of target compound 6

5-(6-chloro-5-((1S,2S)-2-(1-hydroayethyl)cyclopropyl)pyridazin-3-yl)pyrimidine-2,4(1H,3H)-dione (target compound 6)

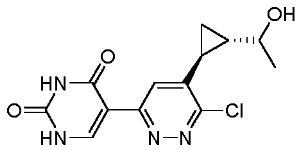

6

The synthesis route of target compound 6 was shown as follows:

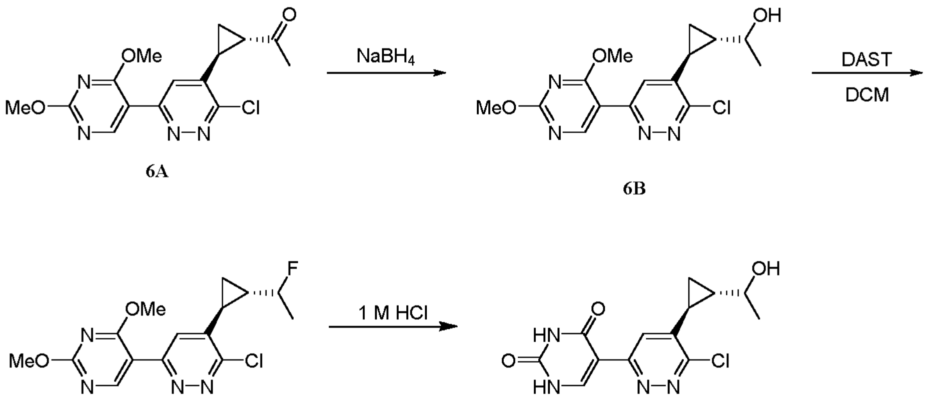


6A 6B 6C 6

First step: synthesis of 1-((1S,2S)-2-(3-chloro-6-(2,4-dimethoxypyrimidin-5-yl)pyridazin-4-yl)cyclopropyl)ethan-1-ol (6B)

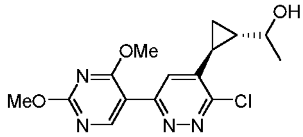

6B

1-((1S,2S)-2-(3-chloro-6-(2,4-dimethoxypyrimidin-5-yl)pyridazin-4-yl)cyclopropyl)ethan-1-one (100 mg, 0.29 mmol) (synthesized with reference to Example 5) was dissolved in methanol (5 mL), sodium borohydride (11.3 mg, 0.29 mmol) was added at 0°C, and the reaction solution reacted at 20°C for 1 h. After the reaction was completed, water (25 mL) was added to quench the reaction, the mixture was extracted with ethyl acetate (25 mL × 2), and organic phases were combined, washed with a saturated salt solution (25 mL), dried with sodium sulfate, and concentrated to obtain a yellow oily compound 1-((1S,2S)-2-(3-chloro-6-(2,4-dimethoxypyrimidin-5-yl)pyridazin-4-yl)cyclopropyl)ethan-1-ol (6B) (100 mg, yield=99%) that was directly used at the next step.

LC-MS, M/Z (ESI): 337.2 $[M+H]^+$.

Second step: synthesis of 3-chloro-6-(2,4-dimethoaypyrimidin-5-yl)-4-((1S,2S)-2-(1-fluoroethyl)cyclopropyl)pyridazine (6C)

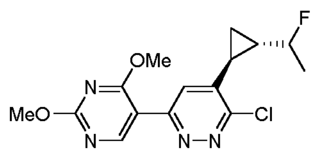

6C

(S)-1-((1S,2S)-2-(3-chloro-6-(2,4-dimethoxypyrimidin-5-yl)pyridazin-4-yl)cyclopropyl)ethan-1-ol (6B) (100 mg, 0.29 mmol) was dissolved in dichloromethane (5 mL), diethylaminosulfur trifluoride (95.7 mg, 0.078 mL, 0.59 mmol) was added dropwise at 0°C, and the reaction was stirred and reacted at 0°C for 0.5 h. A saturated sodium bicarbonate aqueous solution (20 mL) was added to the reaction mixture to quench the reaction, the mixture was then extracted with dichloromethane (25 mL × 3), and organic phases were combined, washed with a saturated salt solution (25 mL), dried with sodium sulfate, and concentrated to obtain a yellow oily compound 3-chloro-6-(2,4-dimethoxypyrimidin-5-yl)-4-((1S,2S)-2-(1-fluoroethyl)cyclopropyl)pyridazine (6C) (100 mg, yield=99%) that was directly used at the next step.
LC-MS, M/Z (ESI): 339.3 $[M+H]^+$.

Third step: synthesis of 5-(6-chloro-5-((1S,2S)-2-(1-hydroxyethyl)cyclopropyl)pyridazin-3-yl)pyrimidine-2,4(1H,3H)-dione (6)

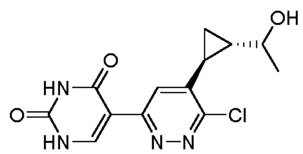

6

3-chloro-6-(2,4-dimethoxypyrimidin-5-yl)-4-((1S,2S)-2-(1-fluoroethyl)cyclopropyl)pyridazine (6C) (100 mg, 0.29 mmol) was dissolved in a hydrochloric acid aqueous solution (1 M, 3 mL), and the reaction solution was heated to 60°C and reacted for 1 h. The reaction solution was concentrated and then separated by reversed phase high-performance liquid chromatography (chromatographic column: 3_Phenomenex Luna C18 (75 mm × 30 mm, 3 μm); mobile phases: A: water + 0.05 vol% of HCl (36.5%), B: acetonitrile; gradient: 8%-28% of B, 7 min) to obtain a pale yellow solid 5-(6-chloro-5-((1S,2S)-2-(1-hydroxyethyl)cyclopropyl)pyridazin-3-yl)pyrimidine-2,4(1H,3H)-dione (6) (5 mg, yield=4.8%).
$^1H$ NMR (400 MHz, CDsOD) δ 8.59 (s, 1H), 8.08 (s, 1H), 3.63-3.68 (m, 1H), 2.28-2.33 (m, 1H), 1.54-1.57 (m, 1H), 1.44-1.48 (m, 1H), 1.32-1.34 (m, 4H).
LC-MS, M/Z (ESI): 309.1 $[M+H]^+$.

Example 7: Preparation of target compound 7

5-(6-methyl-5-((1S,2S)-2-(trifluoromethyl)cyclopropyl)pyridazin-3yl)pyrimidine-2,4(1H,3H)-dione (target compound 7)

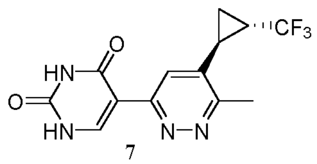

7

The synthesis route of target compound 7 was shown as follows:

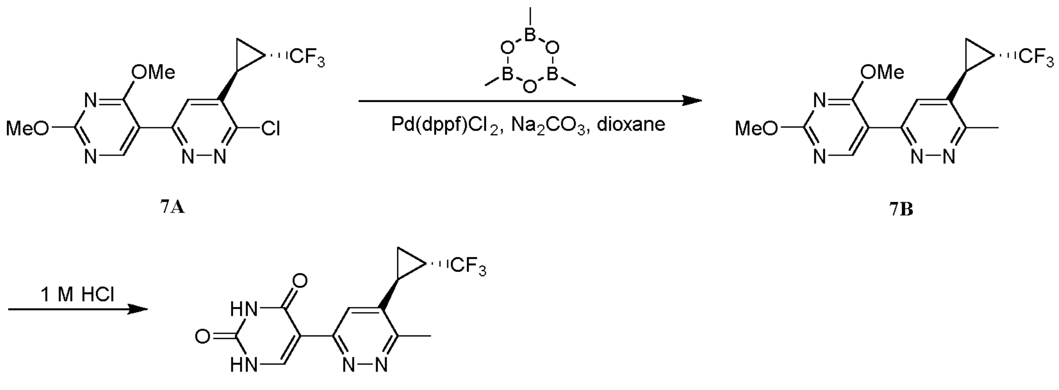


7A

7B

7

First step: synthesis of 6-(2,4-dimethoxypyrimidin-5-yl)-3-methyl-4-((1S,2S)-2-(trifluoromethyl)cyclopropyl)pyridazine (7B)

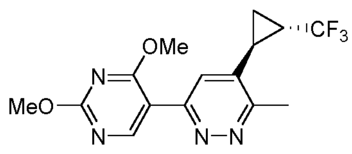

7B

3-chloro-6-(2,4-dimethoaypyrimidin-5-yl)-4-((1S,2S)-2-(trifluoromethyl)cyclopropyl)pyridazine (240 mg, 467 μmol) and 2,4,6-trimethyl-1,3,5,2,4,6-trioxatriborinane (176 mg, 700 μmol) were dissolved in dioxane (5 mL) and water (1 mL), potassium carbonate (161 mg, 1.17 mmol) and [1,1'-bis(diphenylphosphino)ferrocene]dichloropalladium(II) (68.3 mg, 93.4 μmol) were added under the protection of nitrogen gas, and the reaction solution was heated to 90°C and reacted for 12 h. The reaction system was spin-dried to obtain a crude product. The crude product was separated and purified by silica gel column (petroleum ether: ethyl acetate (v/v) = (10: 1) to (1: 1), gradient elution) to obtain a yellow oily compound 6-(2,4-dimethoxypyrimidin-5-yl)-3-methyl-4-((1S,2S)-2-(trifluoromethyl)cyclopropyl)pyridazine (7B) (150 mg, yield=63.9%).

LC-MS, M/Z (ESI): 341.1 $[M+H]^+$.

Second step: synthesis of 5-(6-methyl-5-((1S,2S)-2-(trifluoromethyl)cyclopropyl)pyridazin-3-yl)pyrimidine-2,4(1H,3H)-dione (7)

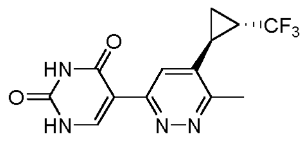

7

6-(2,4-dimethoaypyrimidin-5-yl)-3-methyl-4-((1S,2S)-2-(trifluoromethyl)cyclopropyl)pyridazine (150 mg, 441 μmol) was dissolved in a hydrochloric acid aqueous solution (1 M, 3 mL), and the reaction solution was heated to 50°C and reacted for 12 h. The reaction system was spin-dried to obtain a crude product, and the crude product was separated by reversed phase high-performance liquid chromatography (chromatographic column: 3_Phenomenex Luna C18 (75 mm × 30 mm, 3 μm); mobile phases: A: water + hydrochloric acid (0.05%), B: acetonitrile; gradient: 12%-32% of B, 6.5 min) to obtain a white solid 5-(6-methyl-5-((1S,2S)-2-(trifluoromethyl)cyclopropyl)pyridazin-3 yl)pyrimidine-2,4(1H,3H)-dione (7) (108.5 mg, yield=77.6%).

$^1H$ NMR (400 MHz, CDsOD) δ 8.67 (s, 1H), 8.51 (s, 1H), 2.89 (s, 3H), 2.69-2.72 (m, 1H), 2.45-2.49 (m, 1H), 1.64-1.74 (m, 2H).

LC-MS, M/Z (ESI): 313.0 $[M+H]^+$.

Example 8: preparation of target compound 8

5-(6-chloro-5-((1R,2R)-2-(fluoromethyl)cyclopropyl)pyridazin-3-yl)pyrimidine-2,4(1H,3H)-dione (target compound 8)

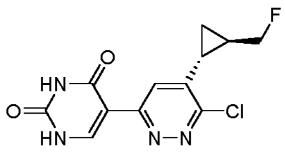

8

The synthesis route of target compound 8 was shown as follows:

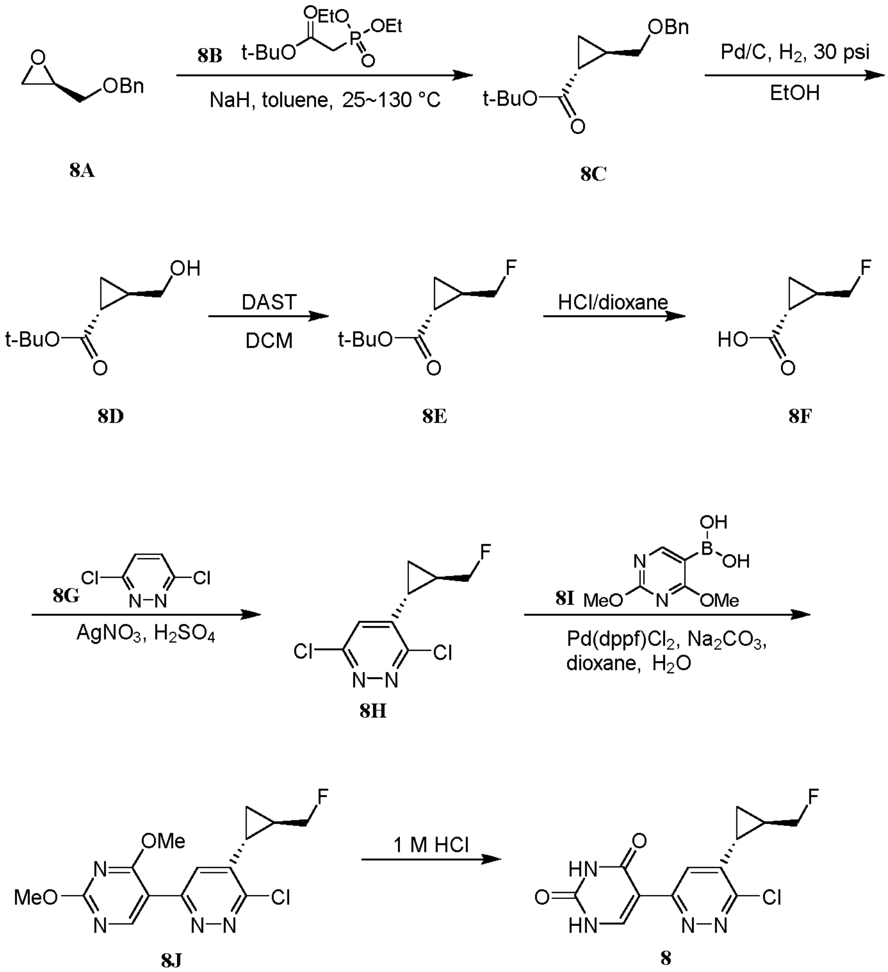


8A 8C 8D 8E 8F 8H 8J 8

First step: synthesis of tert-butyl (1R,2R)-2-((benzyloay)methyl)cyclopropane-1-carboaylate (8C)

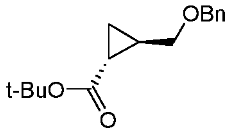

8C

Under the protection of nitrogen gas, sodium hydride (2.92 g, 73.1 mmol, purity=60%) was suspended in toluene (100 mL), tert-butyl diethylphosphonoacetate (16.9 g, 66.9 mmol) was added dropwise, the reaction solution was stirred at 25°C for 30 min after the dropwise addition was complete, benzyl (R)-(-)-glycidyl ether (10.0 g, 60.9 mmol) was added to the reaction solution, and the reaction solution was heated to 130°C and reacted for 1 h. The reaction mixture was diluted with water (200 mL), and extracted with ethyl acetate (200 mL × 2), and organic phases were combined, washed with a saturated salt solution (100 mL), dried with anhydrous sodium sulfate, and concentrated to obtain a crude product. The crude product was separated and purified by silica gel column (petroleum ether: ethyl acetate (v/v) = (50: 1) to (10: 1), gradient elution) to obtain a yellow oily compound tert-butyl (1R,2R)-2-((benzyloxy)methyl)cyclopropane-1-carboxylate (8C) (12 g, yield=75.1%).

$^1$H NMR (400 MHz, $CDCl_3$) δ 7.29-7.36 (m, 5H), 4.53 (s, 2H), 3.38-3.43 (m, 2H), 1.66-1.71 (m, 1H), 1.46-1.50 (m, 1H), 1.45 (s, 9H), 1.13-1.17 (m, 1H), 0.79-0.82 (m, 1H).

Second step: synthesis of tert-butyl (1R,2R)-2-(hydroxymethyl)cyclopropane-1-carboxylate (8D)

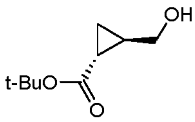

8D

Tert-butyl (1R,2R)-2-((benzyloxy)methyl)cyclopropane-1-carboxylate (12.0 g, 45.7 mmol) was dissolved in ethanol (100 mL), Pd/C (3.00 g, content=10%) was added under the protection of nitrogen gas, and the reaction solution was subjected to hydrogen gas replacement 3 times and reacted under 50 Psi at 60°C for 12 h. The reaction solution was cooled to the room temperature and filtered with diatomite to remove the Pd/C, an obtained filter cake was washed 3 times with ethanol, and an obtained filtrate was concentrated to obtain a yellow oily compound tert-butyl (1R,2R)-2-(hydroxymethyl)cyclopropane-1-carboxylate (8D) (7.50 g, yield=95.2%).

$^1$H NMR (400 MHz, $CDCl_3$) δ 3.56-3.61 (m, 1H), 3.70-3.75 (m, 1H), 1.63-1.71 (m, 2H), 1.45 (s, 9H), 1.14-1.17 (m, 1H), 0.77-0.81 (m, 1H).

Third step: synthesis of tert-butyl (1R,2R)-2-(fluoromethyl)cyclopropane-1-carboxylate (8E)

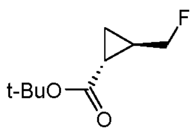

8E

Tert-butyl (1R,2R)-2-(hydroaymethyl)cyclopropane-1-carboxylate (7.50 g, 43.6 mmol) was dissolved in dichloromethane (100 mL), diethylaminosulfur trifluoride (10.5 g, 8.63 mL, 65.3 mmol) was added dropwise at 0°C, and the reaction solution was stirred and reacted at 0°C for 1 h. A saturated sodium bicarbonate aqueous solution (100 mL) was added to the reaction mixture to quench the reaction, the mixture was extracted with dichloromethane (100 mL × 2), and organic phases were combined, washed with saturated salt solution (100 mL), dried with anhydrous sodium sulfate, and concentrated to obtain a crude product. The crude product was separated and purified by silica gel column (petroleum ether: ethyl acetate (v/v) = (50: 1) to (10: 1), gradient elution) to obtain a yellow oily compound tert-butyl (1R,2R)-2-(fluoromethyl)cyclopropane-1-carboxylate (8E) (4.00 g, yield=52.7%).

$^1H$ NMR (400 MHz, $CDCl_3$) δ 4.14-4.44 (m, 2H), 1.75-1.82 (m, 1H), 1.57-1.59 (m, 1H), 1.46 (s, 9H), 1.18-1.23 (m, 1H), 0.83-0.89 (m, 1H).

Fourth step: synthesis of (1R,2R)-2-(fluoromethyl)cyclopropane-1-carboxylic acid (8F)

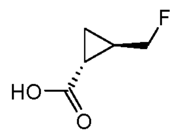

8F

Tert-butyl (1R,2R)-2-(fluoromethyl)cyclopropane-1-carboxylate (4.00 g, 22.9 mmol) was dissolved in a 1,4-dioxane (20 mL) solution of hydrogen chloride (4 M), and the reaction solution was stirred at 25°C for 1 h. The reaction solution was concentrated to obtain a yellow oily compound (1R,2R)-2-(fluoromethyl)cyclopropane-1-carboxylic acid (8F) (2.50 g, yield=92.2%).
$^1H$ NMR (400 MHz, $CDCl_3$) δ 4.16-4.97 (m, 2H), 1.88-1.93 (m, 1H), 1.66-1.71 (m, 1H), 1.32-1.36 (m, 1H), 0.99-1.04 (m, 1H).

Fifth step: synthesis of 3,6-dichloro-4-((1R,2R)-2-(fluoromethyl)cyclopropyl)pyridazine (8H)

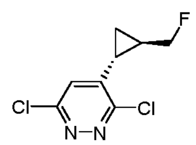

8H

3,6-dichloropyridazine (3.15 g, 21.2 mmol) and (1R,2R)-2-(fluoromethyl)cyclopropane-1-carboxylic acid (2.50 g, 21.2 mmol) were dissolved in water (30 mL), concentrated sulfuric acid (2.71 mL) was added, and under the protection of nitrogen gas, the reaction solution was heated to 70°C. Then, an aqueous solution (1.80 g, 10.6 mmol, 10 mL) of silver nitrate was added quickly, an aqueous solution (14.5 g, 63.5 mmol, 30 mL) of ammonium persulfate was added slowly dropwise, and the reaction solution reacted at 70°C for 1 h. The reaction solution was regulated with ammonia water until the pH was about 9, and extracted with ethyl acetate (200 mL × 2), and organic phases were combined, washed with a saturated salt solution (100 mL), dried with anhydrous sodium sulfate, and concentrated to obtain a crude product. The crude product was separated and purified by silica gel column (petroleum ether: ethyl acetate (v/v) = (10: 1) to (3: 1), gradient elution) to obtain a yellow oily compound 3,6-dichloro-4-((1R,2R)-2-(fluoromethyl)cyclopropyl)pyridazine (8H) (200 mg, yield=4.3%).
LC-MS, M/Z (ESI): 221.0 $[M+H]^+$.

Sixth step: synthesis of 3-chloro-6-(2,4-dimethoxypyrimidin-5-yl)-4-((1R,2R)-2-(fluoromethyl)cyclopropyl)pyridazine (8J)

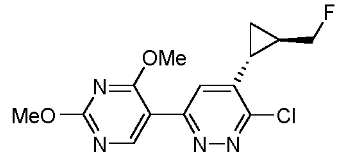

8J

3,6-dichloro-4-((1R,2R)-2-(fluoromethyl)cyclopropyl)pyridazine (200 mg, 1.36 mmol) and 2,4-dimethoxypyrimidine-5-boric acid (149.8 mg, 0.814 mmol) were dissolved in 1,4-dioxane (2 mL) and water (0.5 mL), sodium carbonate

(287.7 mg, 2.71 mmol) and [1,1'-bis(diphenylphosphino)ferrocene]dichloropalladium(II) (66.2 mg, 90.5 μmol) were added under the protection of nitrogen gas, and the reaction solution was heated to 50°C and reacted for 12 h. The reaction mixture was diluted with water (20 mL), and extracted with ethyl acetate (20 mL × 3), and organic phases were combined, washed with a saturated salt solution (50 mL), dried with anhydrous sodium sulfate, and concentrated to obtain a crude product. The crude product was separated and purified by silica gel column (petroleum ether: ethyl acetate (v/v) = (10: 1) to (3: 1), gradient elution) to obtain a yellow oily compound 3-chloro-6-(2,4-dimethoxypyrimidin-5-yl)-4-((1R,2R)-2-(fluoromethyl)cyclopropyl)pyridazine (8J) (100 mg, yield=34%).

LC-MS, M/Z (ESI): 325.1 $[M+H]^+$.

Seventh step: synthesis of 5-(6-chloro-5-((1R,2R)-2-(fluoromethyl)cyclopropyl)pyridazin-3-yl)pyrimidine-2,4(1H,3H)-dione (target compound 8)

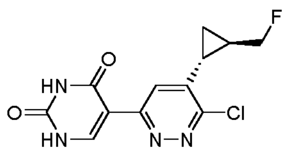

8

3-chloro-6-(2,4-dimethoaypyrimidin-5-yl)-4-((1R,2R)-2-(fluoromethyl)cyclopropyl)pyridazine (80 mg, 246.4 μmol) was dissolved in a hydrochloric acid aqueous solution (1 M, 2 mL), and the reaction solution was heated to 50°C and reacted for 2 h. The reaction system was spin-dried to obtain a crude product, and the crude product was separated by reversed phase high-performance liquid chromatography (chromatographic column: 3_Phenomenex Luna C18 (75 mm × 30 mm, 3 μm); mobile phases: A: water + hydrochloric acid (0.05%), B: acetonitrile; gradient: 17%-37% of B, 6.5 min) to obtain a white solid compound 5-(6-chloro-5-((1R,2R)-2-(fluoromethyl)cyclopropyl)pyridazin-3-yl)pyrimidine-2,4(1H,3H)-dione (8) (19 mg, yield=26%).

$^1$H NMR (400 MHz, CDsOD) δ 8.75 (s, 1H), 8.20 (s, 1H), 4.37-4.65 (m, 2H), 2.43-2.47 (m, 1H), 1.98-2.01 (m, 1H), 1.45-1.56 (m, 2H).

LC-MS, M/Z (ESI): 297.1 $[M+H]^+$.

Example 9: preparation of target compound 9

5-(6-chloro-5-((1S,2R)-2-(2,2,2-trifluoroethyl)cyclopropyl)pyridazin-3-yl)pyrimidine-2,4(1H,3H)-dione (target compound 9)

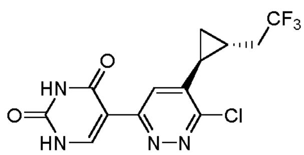

9

The synthesis route of target compound 9 was shown as follows:

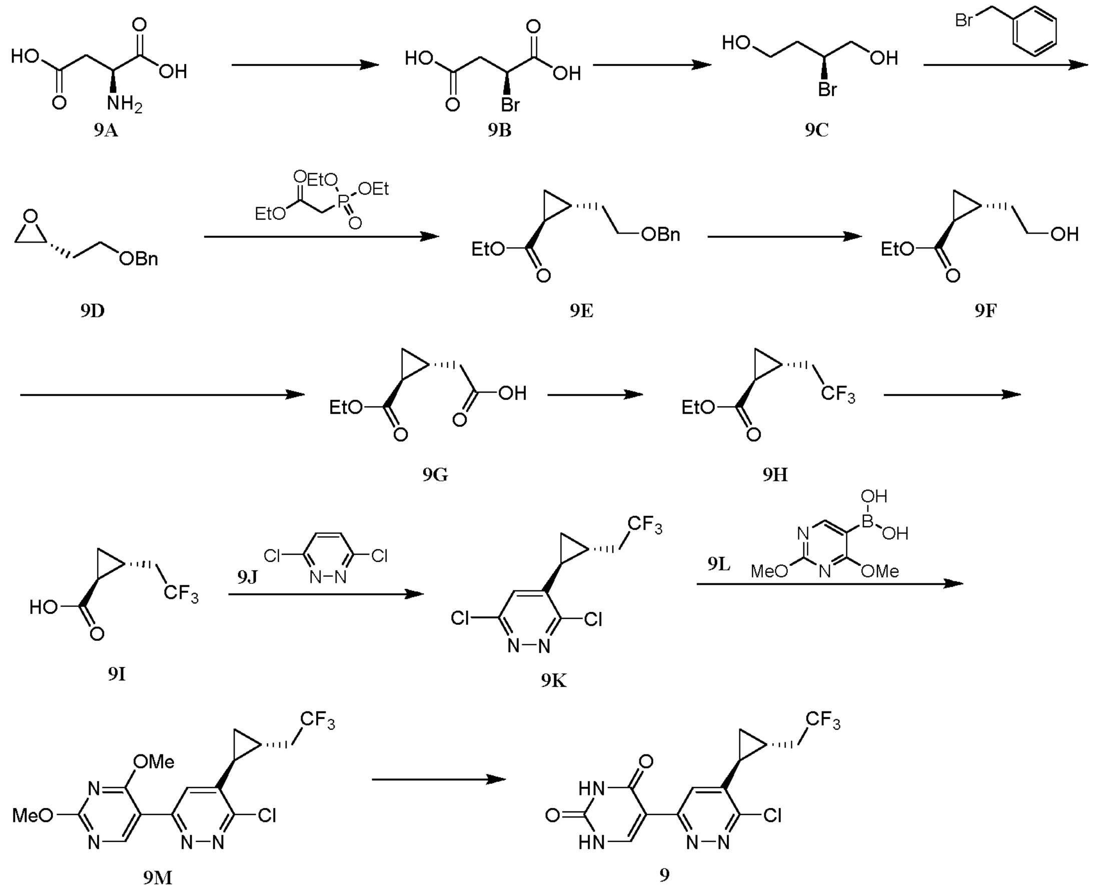

9A 9B 9C 9D 9E 9F 9G 9H 9I 9J 9K 9L 9M 9

First step: synthesis of (S)-2-bromosuccinic acid (9B)

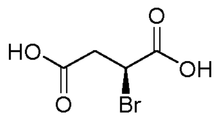

9B

(S)-2-aminosuccinic acid (50.0 g, 375.6 mmol) was dissolved in concentrated sulfuric acid (134.8 mL) and water (900 mL), potassium bromide (205.6 g, 1.73 mol) was added at 0°C, an aqueous solution (46.7 g, 676.2 mmol, 100 mL) of sodium nitrite was added slowly dropwise at 0°C, and the reaction solution reacted at 25°C for 4 h after the dropwise addition was complete. After the reaction was completed, the reaction solution was extracted with ethyl acetate (500 mL × 2), organic phases were combined, washed with a saturated salt solution (500 mL × 2), dried with anhydrous sodium sulfate, filtered, and concentrated to obtain a product, and the product was beaten with petroleum ether (100 mL) to obtain (S)-2-bromosuccinic acid (9B) (70 g, crude product).

Second step: synthesis of (S)-2-bromobutane-1,4-diol (9C)

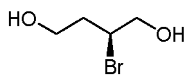

9C

(S)-2-bromosuccinic acid (210.0 g, 1.07 mol) was dissolved in tetrahydrofuran (2100 mL), a borane-dimethyl

sulfide solution (10 M, 319.8 mL) was added slowly dropwise at 0°C, and the reaction solution reacted at the room temperature for 2 h after the dropwise addition was complete. After the reaction was completed, water (320 mL) was added dropwise at 0°C to quench the reaction, solid potassium carbonate (480 g) was then added, the mixture was stirred for 1 h and filtered, an obtained filter cake was washed with tetrahydrofuran (250 mL × 2), and an obtained filtrate was concentrated to obtain (S)-2-bromobutane-1,4-diol (9C) (255.0 g, crude product).

Third step: synthesis of (R)-2-(2-(benzyloxy)ethyl)oxirane (9D)

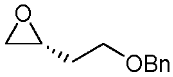

9D

Under the protection of nitrogen gas, sodium hydride (50.3 g, 1.26 mmol, 60%) was added to tetrahydrofuran (900 mL), (S)-2-bromobutane-1,4-diol (85.0 g, 502.9 mmol) was dissolved in tetrahydrofuran (100 mL) and then added dropwise slowly to the above solution, and the temperature of the mixture was controlled at about 0°C. Then, benzyl bromide (120.4 g, 704.1 mmol) was dissolved in tetrahydrofuran (100 mL) and added together with tetrabutylammonium iodide (18.6 g, 50.3 mmol) to the reaction solution, and the reaction solution reacted at 25°C for 5 h. After the reaction was completed, water (500 mL) was added to quench the reaction, the reaction solution was extracted with ethyl acetate (500 mL × 2), and organic phases were combined, washed with a saturated salt solution (500 mL), dried with anhydrous sodium sulfate, filtered, concentrated, and separated and purified by silica gel column (petroleum ether: ethyl acetate (v/v) = (50: 1) to (10: 1)) to obtain (R)-2-(2-(benzyloxy)ethyl)oxirane (9D) (65 g, yield=72.2%).

Fourth step: synthesis of ethyl (1S,2R)-2-(2-(benzyloxy)ethyl)cyclopropanecarboxylate (9E)

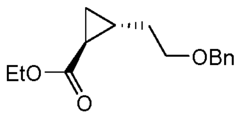

9E

Under the protection of nitrogen gas, sodium hydride (5.25 g, 131.3 mmol, 60%) was suspended in toluene (300 mL), triethyl phosphonoacetate (27.6 g, 123.2 mmol) was added dropwise at 0°C, the reaction solution was stirred at 25°C for 1 h after the dropwise addition was complete, (R)-2-(2-(benzyloxy)ethyl)oxirane (18.0 g, 100.9 mmol) was added to the reaction solution, and the reaction solution was heated to 130°C and reacted for 3 h. The reaction mixture was diluted with water (300 mL), and extracted with ethyl acetate (300 mL × 2), and organic phases were combined, washed with a saturated salt solution (200 mL), dried with sodium sulfate, and concentrated to obtain a crude product. The crude product was separated and purified by silica gel column (petroleum ether: ethyl acetate (v/v) = (1: 0) to (10: 1)) to obtain a yellow oily compound ethyl (1S,2R)-2-(2-(benzyloxy)ethyl)cyclopropanecarboxylate (9E) (17 g, yield=67.8%).

Fifth step: synthesis of ethyl (1S,2R)-2-(2-hydroxyethyl)cyclopropanecarboxylate (9F)

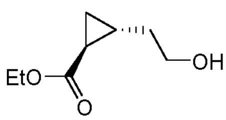

9F

Ethyl (1S,2R)-2-(2-(benzyloxy)ethyl)cyclopropanecarboxylate (32.0 g, 128.9 mmol) was dissolved in ethanol (400 mL), Pd/C (9.0 g, 10%) was added under the protection of nitrogen gas, and the reaction solution was subjected to hydrogen gas replacement 3 times and reacted under 50 Psi at 60°C for 24 h. The reaction solution was cooled to

the room temperature and filtered with diatomite to remove the Pd/C, an obtained filter cake was washed 3 times with ethanol, and an obtained filtrate was concentrated to obtain a yellow oily compound ethyl (1S,2R)-2-(2-hydroxyethyl)cyclopropanecarboxylate (9F) (20.1 g, crude product).

Sixth step: synthesis of 2-((1R,2S)-2-(ethoxycarbonyl)cyclopropyl)acetic acid (9G)

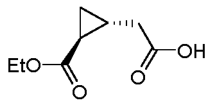

9G

Ethyl (1S,2R)-2-(2-hydroxyethyl)cyclopropanecarboxylate (15.0 g, 94.8 mmol) was dissolved in acetonitrile (380 mL), and 2,2,6,6-tetramethylpiperidine oxide (1.19 g, 7.59 mmol), sodium dihydrogen phosphate (18.2 g, 151.7 mmol), and disodium phosphate (21.5 g, 151.7 mmol) were added in sequence at 25°C. Then, a sodium hypochlorite solution (1.46 mL, 8%) and sodium chlorite (17.2 g, 189.6 mmol) were dissolved in water (190 mL) and added slowly dropwise at 0°C to the reaction system, and the reaction system was then stirred at 25°C for 12 h. The reaction system was diluted with water (500 mL), and extracted with ethyl acetate (200 mL × 2), organic phases were combined, a saturated sodium carbonate aqueous solution (500 mL) was added, and the mixture was stirred for 10 min. An organic phase was separated, an obtained aqueous phase was regulated with a hydrochloric acid solution (6 M) until the pH was 2 to 3, and extracted with ethyl acetate (300 mL × 2), and organic phases were combined, washed with a saturated salt solution (500 mL), dried with sodium sulfate, and concentrated to obtain a yellow oily compound 2-((1R,2S)-2-(ethoxycarbonyl)cyclopropyl)acetic acid (9G) (9.00 g, yield=55.1%).

Seventh step: synthesis of ethyl (1S,2R)-2-(2,2,2-trifluoroethyl)cyclopropanecarboxylate (9H)

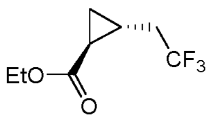

9H

2-((1R,2S)-2-(ethoaycarboiryl)cyclopropyl)acetic acid (9.00 g, 45.9 mmol) was added in a high-pressure autoclave, sulfur tetrafluoride (18.0 g, 166.6 mmol) was added at -78°C, and the reaction system was heated to 70°C and reacted for 16 h in the high-pressure autoclave. Dichloromethane (100 mL) was added to the reaction system, and an obtained organic phase was washed with a saturated sodium bicarbonate aqueous solution (200 mL), dried with anhydrous sodium sulfate, filtered, and concentrated to obtain a yellow oily compound ethyl (1S,2R)-2-(2,2,2-trifluoroethyl)cyclopropanecarboxylate (9H) (8.00 g, crude product) that was directly used at the next step.

Eighth step: synthesis of (1S,2R)-2-(2,2,2-trifluoroethyl)cyclopropanecarboxylic acid (9I)

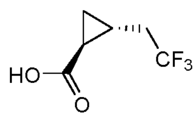

9I

Ethyl (1S,2R)-2-(2,2,2-trifluoroethyl)cyclopropanecarboxylate (8.00 g, 40.8 mmol) was dissolved in tetrahydrofuran (80 mL) and water (40 mL), lithium hydroxide monohydrate (5.13 g, 122.4 mmol) was added, and the reaction solution reacted at 50°C for 12 h. After the reaction was completed, water (100 mL) was added, the mixture was extracted with dichloromethane (100 mL × 2), an aqueous phase was collected, regulated with hydrochloric acid (6 M) until the pH was 3 to 4, and extracted with dichloromethane (100 mL × 2), and organic phases were combined, washed with a

saturated salt solution (100 mL), dried with anhydrous sodium sulfate, filtered, and concentrated to obtain a brown oily compound (1S,2R)-2-(2,2,2-trifluoroethyl)cyclopropanecarboxylic acid (9I) (6.30 g, yield=91.9%).

Ninth step: synthesis of 3,6-dichloro-4-((1S,2R)-2-(2,2,2-trifluoroethyl)cyclopropyl)pyridazine (9K)

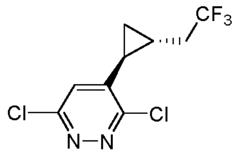

9K

3,6-dichloropyridazine (4.5 g, 30.2 mmol) and (1S,2R)-2-(2,2,2-trifluoroethyl)cyclopropanecarboxylic acid (5.08 g, 16.1 mmol) were dissolved in water (90 mL), concentrated sulfuric acid (4.60 mL) was added, and under the protection of nitrogen gas, the reaction solution was heated to 70°C. Then, an aqueous solution (2.05 g, 12.1 mmol, 25.0 mL) of silver nitrate was added quickly, an aqueous solution (20.7 g, 90.6 mmol, 45.0 mL) of ammonium persulfate was added slowly dropwise, and the reaction solution reacted at 70°C for 1 h. The reaction solution was regulated with ammonia water until the pH was about 9, and extracted with ethyl acetate (100 mL × 2), and organic phases were combined, and washed with a saturated salt solution (100 mL), dried with sodium sulfate, and concentrated to obtain a crude product. Then, the crude product was separated by reversed-phase flash chromatography to obtain a yellow oily compound 3,6-dichloro-4-((1S,2R)-2-(2,2,2-trifluoroethyl)cyclopropyl)pyridazine (9K) (300 mg, yield=2.98%).

LC-MS, M/Z: 271.0 $[M+H]^+$.

Tenth step: synthesis of 3-chloro-6-(2,4-dimethoxypyrimidin-5-yl)-4-((1S,2R)-2-(2,2,2-trifluoroethyl)cyclopropyl)pyridazine (9M)

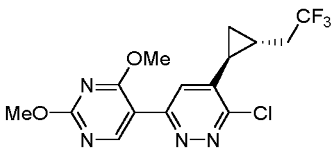

9M

3,6-dichloro-4-((1S,2R)-2-(2,2,2-trifluoroethyl)cyclopropyl)pyridazine (300 mg, 1.11 mmol) and 2,4-dimethoxypyrimidine-5-boric acid (204 mg, 1.11 mmol) were dissolved in dioxane (3 mL) and water (0.8 mL), sodium carbonate (352 mg, 3.32 mmol) and 1,1'-bis(diphenylphosphino)ferrocene-palladium dichloride dichloromethane complex (90.4 mg, 111 μmol) were added under the protection of nitrogen gas, and the reaction solution was heated to 70°C and reacted for 1 h. The reaction mixture was diluted with water (50 mL) and extracted with ethyl acetate (50 mL × 3), and organic phases were combined, washed with a saturated salt solution (50 mL), dried with sodium sulfate, and concentrated to obtain a crude product. The crude product was separated by reversed phase high-performance liquid chromatography (chromatographic column: Phenomenex Gemini-NX C18 (75 mm × 30 mm, 3 μm); mobile phases: A: water + 0.225 vol% of formic acid (99.9%), B: acetonitrile; gradient: 38%-68%, 7.0 min) to obtain a pale yellow solid compound 3-chloro-6-(2,4-dimethoxypyrimidin-5-yl)-4-((1S,2R)-2-(2,2,2-trifluoroethyl)cyclopropyl)pyridazine (9M) (80.0 mg, yield=19.3%).

LC-MS, M/Z: 375.1 $[M+H]^+$.

Eleventh step: synthesis of 5-(6-chloro-5-((1S,2R)-2-(2,2,2-trifluoroethyl)cyclopropyl)pyridazin-3-yl)pyrimidine-2,4(1H,3H)-dione (9)

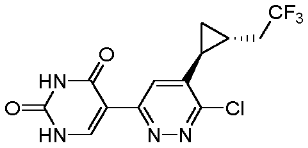

9

3-chloro-6-(2,4-dimethoxypyrimidin-5-yl)-4-((1S,2R)-2-(2,2,2-trifluoroethyl)cyclopropyl)pyridazine (75.0 mg, 200 μmol) was dissolved in tetrahydrofuran (0.5 mL) and added to a hydrochloric acid aqueous solution (1 M, 0.2 mL), and the reaction solution was heated to 50°C and reacted for 12 h. The reaction solution was concentrated to dryness to obtain a crude product, and the crude product was first separated by reversed phase high-performance liquid chromatography (chromatographic column: Phenomenex Gemini-NX C18 (75 mm × 30 mm, 3 μm); mobile phases: A: water + 0.225 vol% of formic acid (99%), B: acetonitrile; gradient: 22%-52%, 7.0 min) and then separated by supercritical fluid chromatography (chromatographic column: DAICEL CHIRALPAK AD (250 mm × 30 mm, 10 μm); mobile phase: 0.1% ammonia methanol solution; gradient: 50%-50%, 40 min) to obtain a white solid compound 5-(6-chloro-5-((1S,2R)-2-(2,2,2-trifluoroethyl)cyclopropyl)pyridazin-3-yl)pyrimidine-2,4(1H,3H)-dione (9) (10 mg, yield=14.3%).

SFC: Rt = 0.787 min, de% = 100%, detection method: chromatographic column: Chiralpak AD-3 (50 mm × 4.6 mm, I.D., 3 μm); mobile phases: A: $CO_2$, B: 0.05% diethylamine methanol solution; elution gradient: 40% methanol (containing 0.05% diethylamine) and 60% carbon dioxide; flow rate: 3 mL/min; detector: PDA; column temperature: 35°C; column pressure: 100 Bar.

$^1$H NMR (400 MHz, CDsOD): δ 8.40 (s, 1H), 8.03 (s, 1H), 2.45-2.48 (m, 1H), 2.36-2.38 (m, 1H), 2.24-2.26 (m, 1H), 1.46-1.48 (m, 1H), 1.27-1.30 (m, 2H).

LC-MS, M/Z: 347.0 $[M+H]^+$.

Example 10: preparation of target compound 10

5-(6-chloro-5-((1S,2S)-2-(perfluoroethyl)cyclopropyl)pyridazin-3-yl)pyrimidine-2,4(1H,3H)-dione (target compound 10)

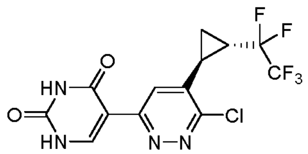

10

Target compound 10 was synthesized with reference to Example 4.

LC-MS, M/Z (ESI): 383.0 $[M+H]^+$.

Example 11: Preparation of target compound 11

5-(6-chloro-5-((1S,2S)-2-((trifluoromethoay)methyl)cyclopropyl)pyridazin-3-yl)pyrimidine-2,4(1H,3H)-dione (target compound 11)

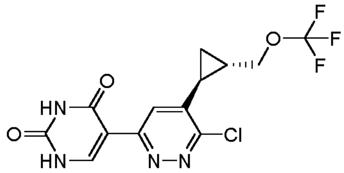

11

The synthesis route of target compound 11 was shown as follows:

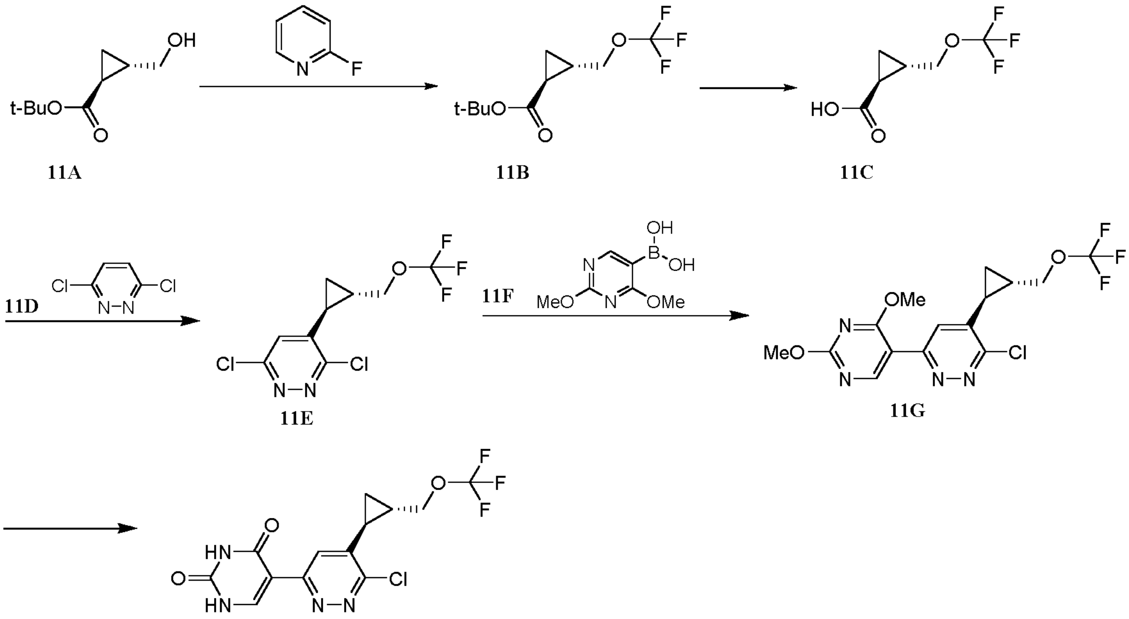


11A 11B 11C 11E 11G 11

First step: synthesis of tert-butyl (1S,2S)-2-((trifluoromethoxy)methyl)cyclopropane-1-carboxylate (11B)

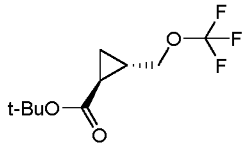

11B

Under the protection of nitrogen gas, tert-butyl (1S,2S)-2-(hydroxymethyl)cyclopropane-1-carboxylate (11A) (20 g, 116 mmol), potassium fluoride (27.0 g, 465 mmol), selectfluor fluorinating reagent (61.6 g, 174 mmol), and silver trifluoromethanesulfonate (90 g, 348 mmol) were added in turn into a reaction flask in the dark, an ethyl acetate (600 mL) solution of 2-fluoropyridine (33.8 g, 348 mmol) and (trifluoromethyl)trimethylsilane (49.5 g, 348 mmol) was added dropwise, and the reaction solution reacted at the room temperature for 18 h after the dropwise addition was complete. The reaction solution was diluted with water (500 mL) and separated, an obtained aqueous phase was extracted with ethyl acetate (500 mL × 2), organic phases were combined, dried with anhydrous sodium sulfate, and concentrated, and an obtained residue was separated and purified by silica gel column (petroleum ether: ethyl acetate (v/v) = 500: 1) to obtain a colorless liquid tert-butyl (1S,2S)-2-((trifluoromethoxy)methyl)cyclopropane-1-carboaylate (11B) (8.5 g, yield=30.5%).

Second step: synthesis of (1S, 2S)-2-((trifluoromethoxy)methyl)cyclopropane-1-carboaylic acid (11C)

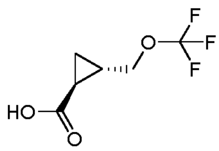

11C

Tert-butyl (1S,2S)-2-((trifluoromethoxy)methyl)cyclopropane-1-carboxylate (11B) (8.5 g, 35.4 mmol) was dissolved in a dioxane (50 mL, 4 M) solution of hydrogen chloride, and the reaction solution was stirred and reacted at the room temperature for 20 h. The reaction solution was concentrated to obtain a yellow oily compound (1S,2S)-2-((trifluoromethoxy)methyl)cyclopropane-1-carboxylic acid (11C) (5.5 g, yield=84.5%).

Third step: synthesis of 3,6-dichloro-4-((lS,2S)-2-((trifluoromethoxy)methyl)cyclopropyl)pyridazine (11E)

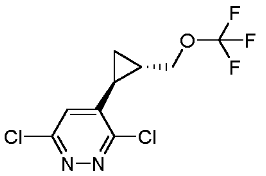

11E

3,6-dichloropyridazine (4.05 g, 27.2 mmol) and (1S,2S)-2-((trifluoromethoxy)methyl)cyclopropane-1-carboxylic acid (11C) (5 g, 27.2 mmol) were dissolved in water (100 mL), silver nitrate (2.307 g, 13.58 mmol) was added, concentrated sulfuric acid (3.5 mL) was then added, and under the protection of nitrogen gas, the reaction solution was heated to 70°C. Then, an aqueous solution (20.45 g, 90 mmol, 100 mL) of ammonium persulfate was added slowly dropwise, and the reaction solution reacted at 70°C for 2 h. The reaction solution was regulated with ammonia water until the pH was about 9, and extracted with ethyl acetate (300 mL × 3), and organic phases were combined, dried with sodium sulfate, and concentrated to obtain a crude product. The crude product was separated and purified by silica gel column (petroleum ether: ethyl acetate (v/v) = (10: 1) to (3: 1)) to obtain a pale yellow solid 3,6-dichloro-4-((lS,2S)-2-((trifluoromethoxy)methyl)cyclopropyl)pyridazine (11E) (5.3 g, yield=68.0%).

LC-MS, M/Z: 287.1 $[M+H]^+$.

Fourth step: synthesis of 3-chloro-6-(2,4-dimethoxypyrimidin-5-yl)-4-((lS,2S)-2-((trifluoromethoxy)methyl)cyclopropyl)pyridazine (11G)

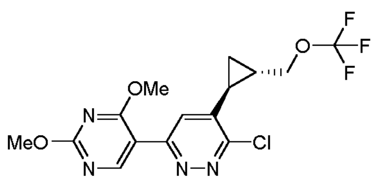

11G

Under the protection of nitrogen gas, 3,6-dichloro-4-((lS,2S)-2-((trifluoromethoxy)methyl)cyclopropyl)pyridazine (11E) (5.3 g, 18.46 mmol), 2,4-dimethoxypyrimidine-5-boric acid (3.06 mg, 16.62 mmol), sodium carbonate (5.87 g, 55.4 mmol), and [1,1'-bis(diphenylphosphino)ferrocene]dichloropalladium(II) (1.351 g, 1.846 mmol) were dissolved in dioxane (100 mL) and water (20 mL), and the reaction solution was heated to 70°C and reacted for 2 h. The reaction mixture was diluted with water (200 mL) and then extracted with ethyl acetate (150 mL × 3), and organic phases were combined, dried with sodium sulfate, and concentrated to obtain a crude product. The crude product was separated by reversed phase high-performance liquid chromatography (chromatographic column: Phenomenex Luna C18 (150 mm × 25 mm, 10 μm); mobile phase: [ water (0.01% TFA)-ACN]; B%: 35%-65%, 10 min) to obtain a pale yellow oily compound 3-chloro-6-(2,4-dimethoxypyrimidin-5-yl)-4-((lS,2S)-2-((trifluoromethoxy)methyl)cyclopropyl)pyridazine (11G) (2 g, yield=27.7%).

LC-MS, M/Z: 391.1 $[M+H]^+$.

Fifth step: synthesis of 5-(6-chloro-5-((lS,2S)-2-((trifluoromethoxy)methyl)cyclopropyl)pyridazin-3-yl)pyrimidine-2,4(1H,3H)-dione (11)

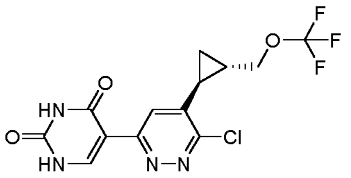

11

3-chloro-6-(2,4-dimethoxypyrimidin-5-yl)-4-((1S,2S)-2-((trifluoromethoxy)methyl)cyclopropyl)pyridazine (11G) (2 g, 0.038 mmol) was dissolved in tetrahydrofuran (10 mL), a hydrochloric acid aqueous solution (1 M, 25.6 mL) was added, and the reaction solution was heated to 50°C and reacted for 20 h. The reaction solution was concentrated to remove the organic solvent, and filtered, and a white solid was collected and dried to obtain 5-(6-chloro-5-((1S,2S)-2-((trifluoromethoxy)methyl)cyclopropyl)pyridazin-3-yl)pyrimidine-2,4(1H,3H)-dione (11) (1.4 g, yield=75.0%).

$^1$H NMR (400 MHz, DMSO_$d_6$) δ 11.58-11.56 (d, 1H), 11.52 (s, 1H), 8.28-8.27 (d, 1H), 7.94 (s, 1H), 4.24-4.12 (m, 2H), 2.27-2.22 (m, 1H), 1.67-1.62 (m, 1H), 1.30-1.19 (m, 2H).

LC-MS, M/Z: 363.0 [M+H]$^+$.

Example 12: Preparation of target compound 12

5-(6-chloro-5-((lS,2S)-2-(methoxymethyl)cyclopropyl)pyridazin-3yl)pyrimidine-2,4(1H,3H)-dione (target compound 12)

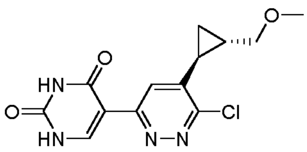

12

The synthesis route of target compound 12 was shown as follows:

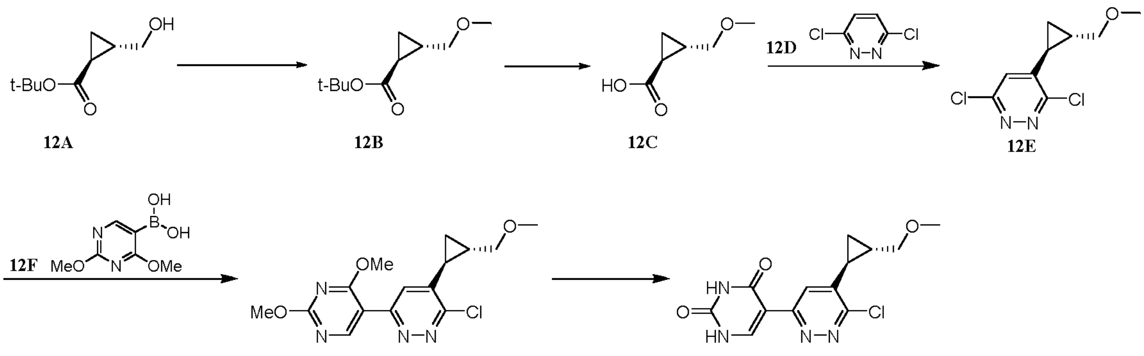


12A 12B 12C 12E 12G 12

First step: synthesis of tert-butyl (1S,2S)-2-(methoxymethyl)cyclopropane-1-carboxylate (12B)

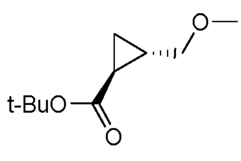

12B

Tert-butyl (1S,2S)-2-(hydroaymethyl)cyclopropane-1-carboaylate (12A) (1 g, 5.81 mmol) was dissolved dry

tetrahydrofuran (10 mL), the reaction solution was cooled to about 0°C, and sodium hydride (0.255 g, 6.39 mmol, 60%) was added. After sodium hydride was added, the reaction solution was heated to the room temperature, and stirred and reacted for 30 min, iodomethane (4.12 g, 29.0 mmol) was added, and the reaction solution was stirred and reacted at the room temperature for 20 h. A saturated ammonium chloride solution (50 mL) was added to quench the reaction, the mixture was extracted with ethyl acetate (50 mL × 3), organic phases were combined, dried with anhydrous sodium sulfate, and concentrated, and an obtained residue was separated and purified by silica gel column (petroleum ether: ethyl acetate (v/v) = (100: 1) to (50: 1)) to obtain a yellow oily compound tert-butyl (1S,2S)-2-(methoxymethyl)cyclopropane-1-carboaylate (12B) (530 mg, yield=49.0%).

Second step: synthesis of (1S,2S)-2-(methoxymethyl)cyclopropane-1-carboxylic acid (12C)

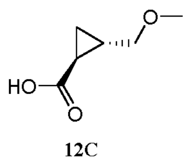

12C

Tert-butyl (1S,2S)-2-(methoxymethyl)cyclopropane-1-carboxylate (12B) (530 mg, 2.85 mmol) was dissolved in a dioxane (10 mL, 4 M) solution of hydrogen chloride, and the reaction solution was stirred and reacted at the room temperature for 20 h. The reaction solution was concentrated to obtain a yellow oily compound (1S,2S)-2-(methoxymethyl)cyclopropane-1-carboxylic acid (12C) (370 mg, yield=100%).

Third step: synthesis of 3,6-dichloro-4-((lS,2S)-2-(methoxymethyl)cyclopropyl)pyridazine (12E)

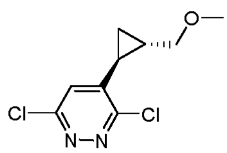

12E

3,6-dichloropyridazine (424 mg, 2.84 mmol) and (1S,2S)-2-(methoxymethyl)cyclopropane-1-carboxylic acid (12C) (370 mg, 2.84 mmol) were dissolved in water (10 mL), concentrated sulfuric acid (0.43 mL) was added, and under the protection of nitrogen gas, the reaction solution was heated to 70°C. Then, an aqueous solution (270 mg, 1.592 mmol, 1 mL) of silver nitrate was added quickly, then an aqueous solution (1.946 g, 8.53 mmol, 5 mL) of ammonium persulfate was added slowly dropwise, and the reaction solution reacted at 70°C for 1 h. The reaction solution was regulated with ammonia water until the pH was about 9, and extracted with ethyl acetate (50 mL × 3), and organic phases were combined, dried with sodium sulfate, and concentrated to obtain a crude product. The crude product was separated and purified by silica gel column (petroleum ether: ethyl acetate (v/v) = (10: 1) to (3: 1)) to obtain a yellow solid 3,6-dichloro-4-((lS,2S)-2-(methoxymethyl)cyclopropyl)pyridazine (12E) (120 mg, yield=18.1%).

LC-MS, M/Z: 233.1 $[M+H]^+$.

Fourth step: synthesis of 3-chloro-6-(2,4-dimethoxypyrimidin-5-yl)-4-((lS,2S)-2-(methoxymethyl)cyclopropyl)pyridazine (12G)

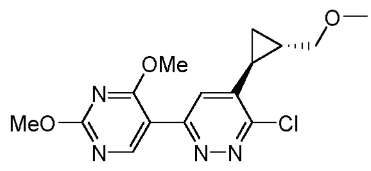

12G

Under the protection of nitrogen gas, 3,6-dichloro-4-((IS,2S)-2-(methoxymethyl)cyclopropyl)pyridazine (12E) (120 mg, 0.515 mmol), sodium carbonate (164 mg, 1.544 mmol), and [1,1'-bis(diphenylphosphino)ferrocene]dichloropalladium(II) (37.7 mg, 0.051 mmol) were dissolved in dioxane (5 mL) and water (5 mL), the reaction solution was heated to 70°C, a dioxane (5 mL)-water (5 mL) solution of 2,4-dimethoxypyrimidine-5-boric acid (95 mg, 0.515 mmol) was added dropwise, and then the reaction solution reacted at 70°C for 1 h. The reaction mixture was diluted with water (50 mL) and extracted with ethyl acetate (50 mL × 3), and organic phases were combined, dried with sodium sulfate, and concentrated to obtain a crude product. The crude product was separated by reversed phase high-performance liquid chromatography (chromatographic column: Phenomenex Luna C18 (150 mm × 25 mm, 10 μm); mobile phase: [water (0.01% trifluoroacetic acid)-acetonitrile]; B%: 35%-65%, 10 min) to obtain a yellow solid 3-chloro-6-(2,4-dimethoxypyrimidin-5-yl)-4-((1S,2S)-2-(methoxymethyl)cyclopropyl)pyridazine (12G) (35 mg, yield=20.2%).

LC-MS, M/Z: 337.1 [M+H]+.

Fifth step: synthesis of 5-(6-chloro-5-((1S,2S)-2-(methoxymethyl)cyclopropyl)pyridazin-3-yl)pyrimidine-2,4(1H,3H)-dione (12)

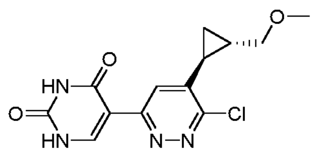

12

3-chloro-6-(2,4-dimethoxypyrimidin-5-yl)-4-((1S,2S)-2-(methoxymethyl)cyclopropyl)pyridazine (12G) (35 mg, 0.104 mmol) was dissolved in methanol (1 mL), a hydrochloric acid aqueous solution (1M, 0.416 mL) was added, and the reaction solution was heated to 50°C and reacted for 20 h. The reaction solution was freeze dried to obtain a pale yellow solid 5-(6-chloro-5-((IS,2S)-2-(methoxymethyl)cyclopropyl)pyridazin-3-yl)pyrimidine-2,4(IH,3H)-dione (12) (24.8 mg, yield=77%).

$^1$H NMR (400 MHz, DMSO_$d_6$) δ 11.56-11.55 (d, 1H), 11.52 (s, 1H), 8.29-8.27 (d, 1H), 7.89 (s, 1H), 3.46-3.36 (m, 2H), 3.25(s, 3H), 2.07-2.05 (m, 1H), 1.52-1.49 (m, 1H), 1.21-1.06 (m, 2H).

LC-MS, M/Z: 309.1 [M+H]+.

Example 13: preparation of target compound 13

5-(6-chloro-5-((1R,2S)-2-(2,2,2-trifluoroethyl)cyclopropyl)pyridazin-3-yl)pyrimidine-2,4(1H,3H)-dione (target compound 13)

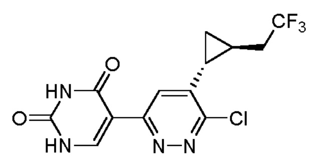

13

The synthesis route of target compound 13 was shown as follows:

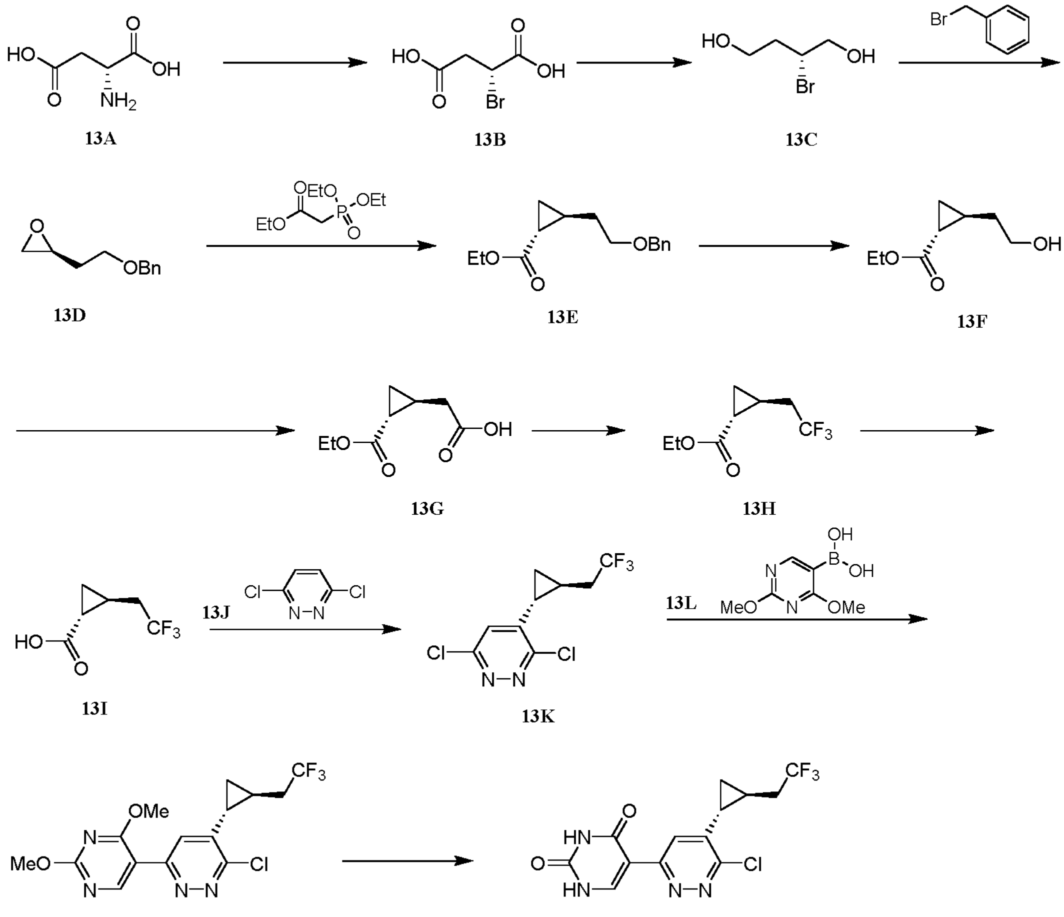


13A 13B 13C 13D 13E 13F 13G 13H 13I 13K 13M 13

First step: synthesis of (R)-2-bromosuccinic acid (13B)

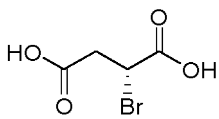

13B

(R)-2-aminosuccinic acid (100.0 g, 751.3 mmol) was dissolved in concentrated sulfuric acid (269.7 mL) and water (1.8 L), potassium bromide (411.3 g, 3.46 mol) was added at 0°C, an aqueous solution (93.3 g, 1.35 mol, 200 mL) of sodium nitrite was added slowly dropwise at 0°C, and the reaction solution reacted at 25°C for 4 h after the dropwise addition was complete. After the reaction was completed, the reaction solution was extracted with ethyl acetate (1500 mL × 2), organic phases were combined, washed with a saturated salt solution (2000 mL × 2), dried with anhydrous sodium sulfate, filtered, and concentrated to obtain a product, and the product was beaten with petroleum ether (500 mL) to obtain (R)-2-bromosuccinic acid (13B) (150 g, crude product).

Second step: synthesis of (R)-2-bromobutane-1,4-diol (13C)

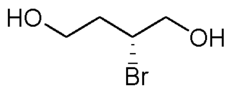

13C

(R)-2-bromosuccinic acid (13.0 g, 66.0 mmol) was dissolved in tetrahydrofuran (200 mL), a borane-dimethyl sulfide solution (10 M, 19.8 mL) was added slowly dropwise at 0°C, and the reaction solution reacted at the room temperature for 2 h after the dropwise addition was complete. After the reaction was completed, water (20 mL) was added dropwise at 0°C to quench the reaction, solid potassium carbonate (30 g) was added, the mixture was stirred for 1 h and filtered, an obtained filter cake was washed with tetrahydrofuran (50 mL × 2), and an obtained filtrate was concentrated to obtain (R)-2-bromobutane-1,4-diol (13C) (12.0 g, crude product).

Third step: synthesis of (S)-2-(2-(benzyloxy)ethyl)oxirane (13D)

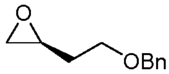

13D

Under the protection of nitrogen gas, sodium hydride (7.10 g, 177.5 mmol, 60%) was added to tetrahydrofuran (60 mL), (R)-2-bromobutane-1,4-diol (12.0 g, 71.0 mmol) was dissolved in tetrahydrofuran (60 mL) and added slowly dropwise to the above solution, and the temperature of the reaction solution was controlled at about 0°C. Then, benzyl bromide (17.0 g, 99.4 mmol) was dissolved in tetrahydrofuran (30 mL) and added together with tetrabutylammonium iodide (2.62 g, 7.10 mmol) to the reaction solution, and the reaction solution reacted at 25°C for 5 h. After the reaction was completed, water (200 mL) was added to quench the reaction, the mixture was extracted with ethyl acetate (200 mL × 2), and organic phases were combined, washed with a saturated salt solution (200 mL), dried with anhydrous sodium sulfate, filtered, concentrated, and then separated and purified by silica gel column (petroleum ether: ethyl acetate (v/v) = (50: 1) to (10: 1)) to obtain (S)-2-(2-(benzyloxy)ethyl)oxirane (13D) (9.50 g, yield=75. 1%).

Fourth step: synthesis of ethyl (IR,2S)-2-(2-(benzyloxy)ethyl)cyclopropanecarboxylate (13E)

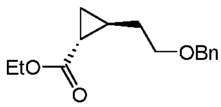

13E

Under the protection of nitrogen gas, sodium hydride (2.60 g, 65.0 mmol, 60%) was suspended in toluene (100 mL), triethyl phosphonoacetate (14.3 g, 63.9 mmol) was added dropwise at 0°C, the reaction solution was stirred at 25°C for 1 h after the dropwise addition was complete, then (S)-2-(2-(benzyloxy)ethyl)oxirane (9.50 g, 53.3 mmol) was added to the reaction solution, and the reaction solution was heated to 130°C and reacted for 3 h. The reaction mixture was diluted with water (100 mL), and extracted with ethyl acetate (100 mL × 2), and organic phases were combined, washed with a saturated salt solution (200 mL), dried with sodium sulfate, and concentrated to obtain a crude product. The crude product was separated and purified by silica gel column (petroleum ether: ethyl acetate (v/v) = (1: 0) to (10: 1)) to obtain a yellow oily compound ethyl (1R,2S)-2-(2-(benzyloxy)ethyl)cyclopropanecarboxylate (13E) (10 g, yield=75.6%).

Fifth step: synthesis of ethyl (1R,2S)-2-(2-hydroxyethyl)cyclopropanecarboxylate (13F)

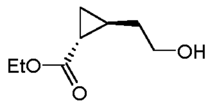

13F

Ethyl (IR,2S)-2-(2-(benzyloxy)ethyl)cyclopropanecarboxylate (9.00 g, 36.2 mmol) was dissolved in ethanol (100 mL), Pd/C (2.0 g, 10%) was added under the protection of nitrogen gas, and the reaction solution was subjected to hydrogen gas replacement 3 times and reacted under 50 Psi at 60°C for 12 h. The reaction solution was cooled to the room temperature, and filtered with diatomite to remove the Pd/C, an obtained filter cake was washed 3 times with ethanol, and an obtained filtrate was concentrated to obtain a yellow oily compound ethyl (1R,2S)-2-(2-hydroxyethyl)cyclopropanecarboxylate (13F) (6.00 g, crude product).

Sixth step: synthesis of 2-((IS,2R)-2-(ethoxycarbonyl)cyclopropyl)acetic acid (13G)

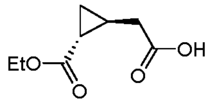

13G

Ethyl (IR,2S)-2-(2-hydroxyethyl)cyclopropanecarboxylate (6.00 g, 37.9 mmol) was dissolved in acetonitrile (150 mL), 2,2,6,6-tetramethylpiperidine oxide (477.1 mg, 3.03 mmol), sodium dihydrogen phosphate (7.28 g, 60.7 mmol), and disodium phosphate (8.61 g, 60.7 mmol) were added in sequence at 25°C. Then, a sodium hypochlorite solution (0.58 mL, 8%) and sodium chlorite (6.86 g, 75.9 mmol) were dissolved in water (75 mL) and added slowly dropwise at 0°C to the reaction system, and the reaction system was stirred at 25°C for 12 h. The reaction system was diluted with water (200 mL), and extracted with ethyl acetate (300 mL × 2), organic phases were combined, a saturated sodium carbonate aqueous solution (200 mL) was added, and the mixture was stirred for 10 min. An organic phase was separated, an obtained aqueous phase was regulated with a hydrochloric acid solution (6 M) until the pH was 2 to 3, and then extracted with ethyl acetate (200 mL × 2), and organic phases were combined, washed with a saturated salt solution (200 mL), dried with sodium sulfate, and concentrated to obtain a yellow oily compound 2-((1S,2R)-2-(ethoxycarbonyl)cyclopropyl)acetic acid (13G) (5.20 g, yield=79.6%).

Seventh step: synthesis of ethyl (IR,2S)-2-(2,2,2-trifluoroethyl)cyclopropanecarboxylate (13H)

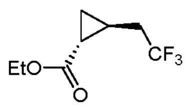

13H

2-((IS,2R)-2-(ethoxycarbonyl)cyclopropyl)acetic acid (5.0 g, 25.5 mmol) was placed in a high-pressure autoclave, sulfur tetrafluoride (9.0 g, 83.3 mmol) was added at -78°C, and the reaction solution was heated to 70°C and reacted for 16 h in the high-pressure autoclave. Dichloromethane (50 mL) was added to the reaction system, and an obtained organic phase was washed with a saturated sodium bicarbonate aqueous solution (200 mL), dried with anhydrous sodium sulfate, filtered, and concentrated to obtain a yellow oily compound ethyl (IR,2S)-2-(2,2,2-trifluoroethyl)cyclopropanecarboxylate (13H) (6.00 g, crude product) that was directly used at the next step.

Eighth step: synthesis of (IR,2S)-2-(2,2,2-trifluoroethyl)cyclopropanecarboxylic acid (13I)

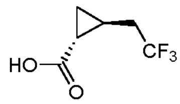

13I

Ethyl (1R,2S)-2-(2,2,2-trifluoroethyl)cyclopropanecarboxylate (6.00 g, 30.6 mmol) was dissolved in tetrahydrofuran (50 mL) and water (25 mL), then lithium hydroxide monohydrate (3.85 g, 91.8 mmol) was added, and the reaction solution reacted at 50°C for 12 h. After the reaction was completed, water (50 mL) was added, the mixture was extracted with dichloromethane (100 mL × 2), an aqueous phase was collected, regulated with hydrochloric acid (6 M) until the pH was 3 to 4, and then extracted with dichloromethane (100 mL × 2), and organic phases were combined, washed with a saturated salt solution (100 mL), dried with anhydrous sodium sulfate, filtered, and concentrated to obtain a brown oily compound (1R,2S)-2-(2,2,2-trifluoroethyl)cyclopropanecarboxylic acid (13I) (3.00 g, yield=58.3%).

Ninth step: synthesis of 3,6-dichloro-4-((IR,2S)-2-(2,2,2-trifluoroethyl)cyclopropyl)pyridazine (13K)

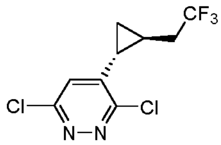

13K

3,6-dichloropyridazine (2.40 g, 16.1 mmol) and (IR,2S)-2-(2,2,2-trifluoroethyl)cyclopropanecarboxylic acid (2.70 g, 16.1 mmol) were dissolved in water (50 mL), concentrated sulfuric acid (2.45 mL) was added, and under the protection of nitrogen gas, the reaction solution was heated to 70°C. Then, an aqueous solution (1.09 g, 6.42 mmol, 10.0 mL) of silver nitrate was added quickly, then an aqueous solution (11.0 g, 48.2 mmol, 20 mL) of ammonium persulfate was added slowly dropwise, and the reaction solution reacted at 70°C for 1 h. The reaction solution was regulated with ammonia water until the pH was about 9, and then extracted with ethyl acetate (200 mL × 2), and organic phases were combined, washed with a saturated salt solution (500 mL), dried with sodium sulfate, and concentrated to obtain a crude product. Then, the crude product was separated by reversed-phase flash chromatography to obtain a brown oily compound 3,6-dichloro-4-((IR,2S)-2-(2,2,2-trifluoroethyl)cyclopropyl)pyridazine (13K) (350 mg, yield=43.8%).

LC-MS, M/Z: 271.1 $[M+H]^+$.

Tenth step: synthesis of 3-chloro-6-(2,4-dimethoxypyrimidin-5-yl)-4-((IR,2S)-2-(2,2,2-trifluoroethyl)cyclopropyl)pyridazine (13M)

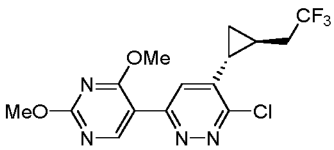

13M

3,6-dichloro-4-((IR,2S)-2-(2,2,2-trifluoroethyl)cyclopropyl)pyridazine (600 mg, 2.21 mmol) and 2,4-dimethoxypyrimidine-5-boric acid (407 mg, 2.21 mmol) were dissolved in dioxane (10 mL) and water (2 mL), sodium carbonate (704 mg, 6.64 mmol) and [1,1'-bis(diphenylphosphino)ferrocene]dichloropalladium(II) (162 mg, 221 μmol) were added under the protection of nitrogen gas, and the reaction solution was heated to 50°C and reacted for 12 h. The reaction mixture was diluted with water (20 mL) and extracted with ethyl acetate (50 mL × 2), and organic phases were combined, washed with a saturated salt solution (100 mL), dried with sodium sulfate, and concentrated to obtain a crude product. The crude product was first separated and purified by silica gel column (petroleum ether: ethyl acetate (v/v) = (50: 1) to (3: 1)) and then separated by reversed phase high-performance liquid chromatography (chromatographic column: 3_Phenomenex Luna C18 (75 mm × 30 mm, 3 μm), mobile phases: A: water + 0.05 vol% of hydrochloric acid (36.5%), B: acetonitrile; gradient: 45%-65%, 6.5 min) to obtain a pale yellow solid compound 3-chloro-6-(2,4-dimethoxypyrimidin-5-yl)-4-((1R,2S)-2-(2,2,2-trifluoroethyl)cyclopropyl)pyridazine (13M) (300 mg, yield=32.7%).

LC-MS, M/Z: 375.1 $[M+H]^+$.

Eleventh step: synthesis of 5-(6-chloro-5-((lR,2S)-2-(2,2,2-trifluoroethyl)cyclopropyl)pyridazin-3-yl)pyrimidine-2,4(1H,3H)-dione (13)

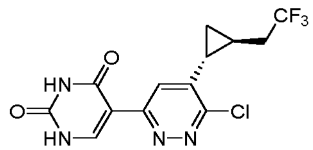

13

3-chloro-6-(2,4-dimethoxypyrimidin-5-yl)-4-((1R,2S)-2-(2,2,2-trifluoroethyl)cyclopropyl)pyridazine (300 mg, 724 μmol) was dissolved in a hydrochloric acid aqueous solution (1 M, 10 mL), and the reaction solution was heated to 50°C and reacted for 12 h. The reaction system was concentrated to dryness to obtain a crude product, and the crude product was first separated by reversed phase high-performance liquid chromatography (chromatographic column: 3_Phenomenex Luna C18 (75 mm × 30 mm, 3 μm), mobile phases: A: water + 0.05 vol% of hydrochloric acid (36.5%), B: acetonitrile; gradient: 26%-46%, 6.5 min) and then separated by supercritical fluid chromatography (chromatographic column: DAICEL CHIRALPAK AD (250 mm × 30 mm, 10 μm); mobile phase: 0.1% ammonia methanol solution; gradient: 45%-45%, 45 min) to obtain a white solid compound 5-(6-chloro-5-((1R,2S)-2-(2,2,2-trifluoroethyl)cyclopropyl)pyridazin-3-yl)pyrimidine-2,4(1H,3H )-dione (13) (108.5 mg, yield=62.9%).

SFC: Rt = 1.645 min, de% = 100%, detection method: chromatographic column: Chiralpak AD-3 (50 mm × 4.6 mm, I.D., 3 μm); mobile phases: A: $CO_2$, B: 0.05% diethylamine methanol solution; elution gradient: 40% methanol (containing 0.05% diethylamine) and 60% carbon dioxide; flow rate: 3mL/min; detector: PDA; column temperature: 35°C; column pressure: 100 Bar.

$^1H$ NMR (400 MHz, CDsOD): δ 8.40 (s, 1H), 8.03 (s, 1H), 2.32-2.52 (m, 2H), 2.22-2.27 (m, 1H), 1.43-1.52 (m, 1H), 1.27-1.32 (m, 2H).

LC-MS, M/Z: 347.0 $[M+H]^+$.

Example 14: Preparation of target compound 14

5-(6-chloro-5-((1R,2R)-2-((trifluoromethoxy)methyl)cyclopropyl)pyridazin-3-yl)pyrimidine-2,4(1H,3H)-dione (target compound 14)

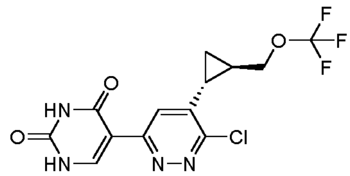

14

The synthesis route of target compound 14 was shown as follows:

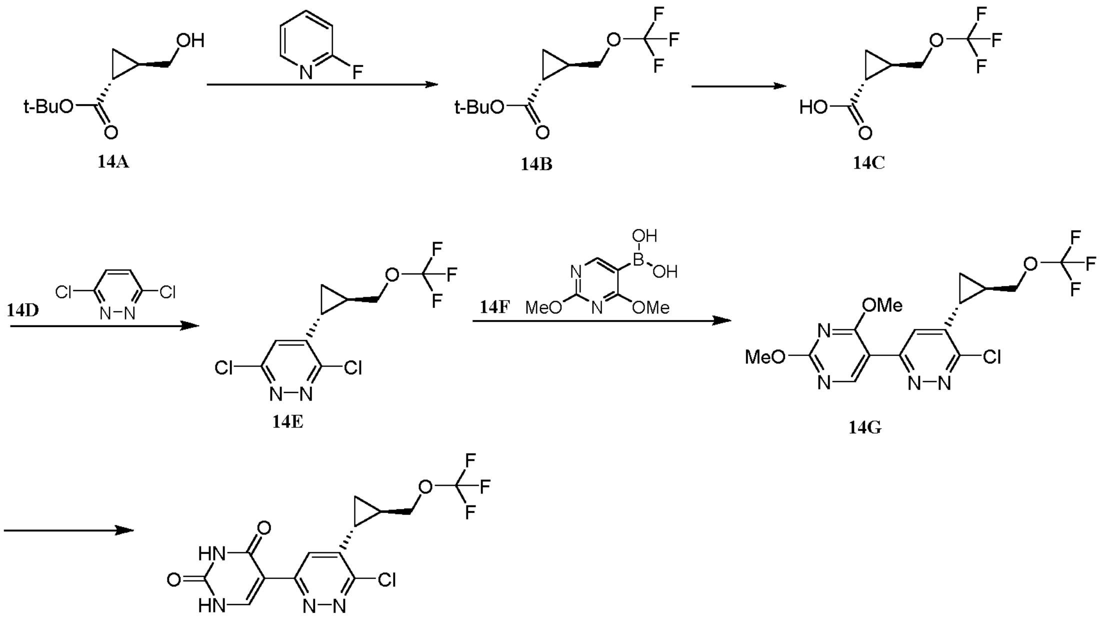


14A 14B 14C 14E 14G 14

First step: synthesis of tert-butyl (1R,2R)-2-((trifluoromethoxy)methyl)cyclopropane-1-carboxylate (14B)

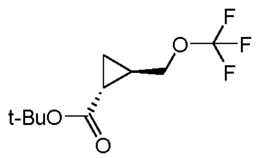

14B

Under the protection of nitrogen gas, tert-butyl (1R,2R)-2-(hydroxymethyl)cyclopropane-1-carboxylate (14A) (4 g, 23.2 mmol), potassium fluoride (5.4 g, 93 mmol), Selectfluor fluorinating reagent (12.3 g, 34.8 mmol), and silver trifluoromethanesulfonate (18 g, 69.6 mmol) were added in turn in a reaction flask in the dark, an ethyl acetate (50 mL) solution of 2-fluoropyridine (6.8 g, 69.6 mmol) and (trifluoromethyl)trimethylsilane (9.9 g, 69.6 mmol) was added dropwise, and then the reaction solution reacted at the room temperature for 18 h. The reaction solution was diluted with water (100 mL) and separated, an obtained aqueous solution was extracted with ethyl acetate (100 mL × 2), organic phases were combined, dried with anhydrous sodium sulfate, and concentrated, and an obtained residue was separated and purified by silica gel column (petroleum ether: ethyl acetate (v/v) = 500: 1) to obtain a colorless liquid tert-butyl (1R,2R)-2-((trifluoromethoxy)methyl)cyclopropane-1-carboxylate (14B) (2.2 g, yield=39.4%).

Second step: synthesis of (1R,2R)-2-((trifluoromethoxy)methyl)cyclopropane-1-carboxylic acid (14C)

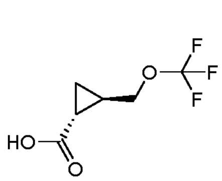

14C

Tert-butyl (1R,2R)-2-((trifluoromethoxy)methyl)cyclopropane-1-carboxylate (14B) (2.2 g, 9.2 mmol) was dissolved in a dioxane (10 mL, 4 M) solution of hydrogen chloride, and the reaction solution was stirred and reacted at the room temperature for 20 h. The reaction solution was concentrated to obtain a yellow oily compound (1R,2R)-2-((trifluoromethoxy)methyl)cyclopropane-1-carboxylic acid (14C) (1.6 g, yield=95%).

Third step: synthesis of 3,6-dichloro-4-((lR,2R)-2-((trifluoromethoxy)methyl)cyclopropyl)pyridazine (14E)

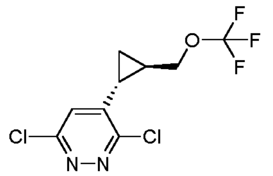

14E

3,6-dichloropyridazine (1.29 g, 8.69 mmol) and (1R,2R)-2-((trifluoromethoxy)methyl)cyclopropane-1-carboxylic acid (14C) (1.6 g, 8.69 mmol) were dissolved in water (40 mL), silver nitrate (0.74 g, 4.35 mmol) was added, then concentrated sulfuric acid (2.2 mL) was added, and under the protection of nitrogen gas, the reaction solution was heated to 70°C. Then, an aqueous solution (6.6 g, 28.9 mmol, 40 mL) of ammonium persulfate was added slowly dropwise, and the reaction solution reacted at 70°C for 2 h. The reaction solution was regulated with ammonia water until the pH was about 9, and then extracted with ethyl acetate (100 mL × 3), and organic phases were combined, dried with sodium sulfate, and concentrated to obtain a crude product. The crude product was separated and purified by silica gel column (petroleum ether: ethyl acetate (v/v) = (10: 1) to (3: 1)) to obtain a pale yellow solid 3,6-dichloro-4-((lR,2R)-2-((trifluoromethoxy)methyl)cyclopropyl)pyridazine (14E) (1.3 g, yield=52.1%).

LC-MS, M/Z: 287.1 $[M+H]^+$.

Fourth step: synthesis of 3-chloro-6-(2,4-dimethoxypyrimidin-5-yl)-4-((lR,2R)-2-((trifluoromethoxy)methyl)cyclopropyl)pyridazine (14G)

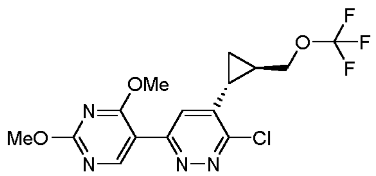

14G

Under the protection of nitrogen gas, 3,6-dichloro-4-((lR,2R)-2-((trifluoromethoxy)methyl)cyclopropyl)pyridazine (14E) (1.3 g, 4.53 mmol), 2,4-dimethoxypyrimidine-5-boric acid (0.67 g, 3.62 mmol), sodium carbonate (1.44 g, 13.6 mmol), and [1,1'-bis(diphenylphosphino)ferrocene]dichloropalladium(II) (0.33 g, 0.45 mmol) were dissolved in dioxane (20 mL) and water (20 mL), and the reaction solution was heated to 70°C and reacted for 2 h. The reaction mixture was diluted with water (100 mL) and extracted with ethyl acetate (150 mL × 3), and organic phases were combined, dried with sodium sulfate, and concentrated to obtain a crude product. The crude product was separated by reversed phase high-performance liquid chromatography (chromatographic column: Phenomenex Luna C18 (150 mm × 25 mm, 10 μm); mobile phase: [ water (0.01% trifluoroacetic acid)-acetonitrile]; B%: 35%-65%, 10 min) to obtain a pale yellow oily compound 3-chloro-6-(2,4-dimethoxypyrimidin-5-yl)-4-((lR,2R)-2-((trifluoromethoxy)methyl)cyclopropyl)pyridazine (14G) (60 mg, yield=3.4%).

LC-MS, M/Z: 391.1 $[M+H]^+$.

Fifth step: synthesis of 5-(6-chloro-5-((lR,2R)-2-((trifluoromethoxy)methyl)cyclopropyl)pyridazin-3-yl)pyrimidine-2,4(1H,3H)-dione (14)

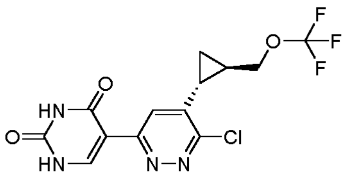

14

3-chloro-6-(2,4-dimethoxypyrimidin-5-yl)-4-((1R,2R)-2-((trifluoromethoxy)methyl)cyclopropyl)pyridazine (14G) (60 mg, 0.15 mmol) was dissolved in tetrahydrofuran (10 mL) and added to a hydrochloric acid aqueous solution (1 M, 10 mL), and the reaction solution was heated to 50°C and reacted for 20 h. The reaction solution was concentrated to remove the organic solvent, and filtered, and a white solid was collected and dried to obtain 5-(6-chloro-5-((1R,2R)-2-((trifluoromethoxy)methyl)cyclopropyl)pyridazin-3-yl)pyrimidine-2,4(1H,3H)-dione (14) (18 mg, yield=31.4%).

$^1$H NMR (400 MHz, DMSO_d$_6$) δ 11.52 (s, 2H), 8.28 (s, 1H), 7.94 (s, 1H), 4.22-4.17 (m, 2H), 2.26 (s, 1H), 1.66 (s, 1H), 1.29-1.21 (m, 2H).

LC-MS, M/Z: 363.1 [M+H]$^+$.

Example 15: preparation of target compound 15

6-(2,4-dioxo-1H-pyrimidin-5-yl)-4-((1S,2S)-2-(trifluoromethyl)cyclopropyl)pyridazine-3-carbonitrile (target compound 15)

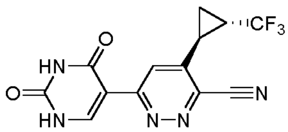

15

The synthesis route of target compound 15 was shown as follows:

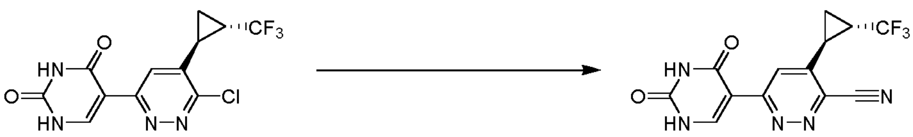

15A

15

First step: synthesis of 6-(2,4-dioxo-1H-pyrimidin-5-yl)-4-((1S,2S)-2-(trifluoromethyl)cyclopropyl)pyridazine-3-carbonitrile (15)

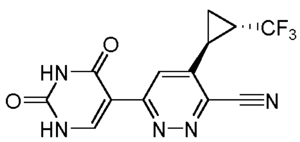

15

5-(6-chloro-5-((1S,2S)-2-(trifluoromethyl)cyclopropyl)pyridazin-3-yl)-1H-pyrimidine-2,4-dione (400 mg, 1.08 mmol) (prepared with reference to Example 4) was dissolved in N,N-dimethylformamide (10 mL), and under the protection of nitrogen gas, zinc cyanide (152 mg, 1.30 mmol), tris(dibenzylideneacetone)dipalladium (98.9 mg, 0.108 mmol), and

1,1'-bis(diphenylphosphino)ferrocene (59.9 mg, 0.108 mmol) were added. The reaction solution was heated to 120°C and reacted for 12 h. The reaction solution was concentrated and then separated by high-performance liquid chromatography (chromatographic column: Phenomenex luna C18 (150 mm × 40 mm, 15 μm), mobile phases: A: water + 0.225 vol% of formic acid (99.9%), B: acetonitrile; gradient: 22%-52%, 10 min) to obtain a pale yellow solid compound 6-(2,4-dioxo-1H-pyrimidin-5-yl)-4-((1S,2S)-2-(trifluoromethyl)cyclopropyl)pyridazine-3-carbonitrile (15) (199.68 mg, yield=56.5%).

$^1$H NMR (400 MHz, CDsOD): δ 8.68 (s, 1H), 8.32 (s, 1H), 2.64-2.67 (m, 1H), 2.42-2.45 (m, 1H), 1.69-1.72 (m, 1H), 1.57-1.67 (m, 1H).

LC-MS, M/Z: 324.1 [M+H]$^+$.

Example 16: preparation of target compound 16

5-(6-chloro-5-((lS,2S)-2-(ethoxymethyl)cyclopropyl)pyridazin-3-yl)-1H-pyrimidine-2,4-dione (target compound 16)

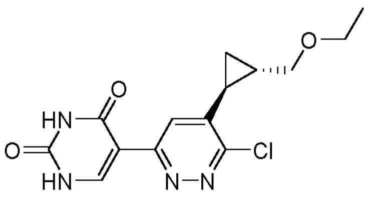

16

The synthesis route of target compound 16 was shown as follows:

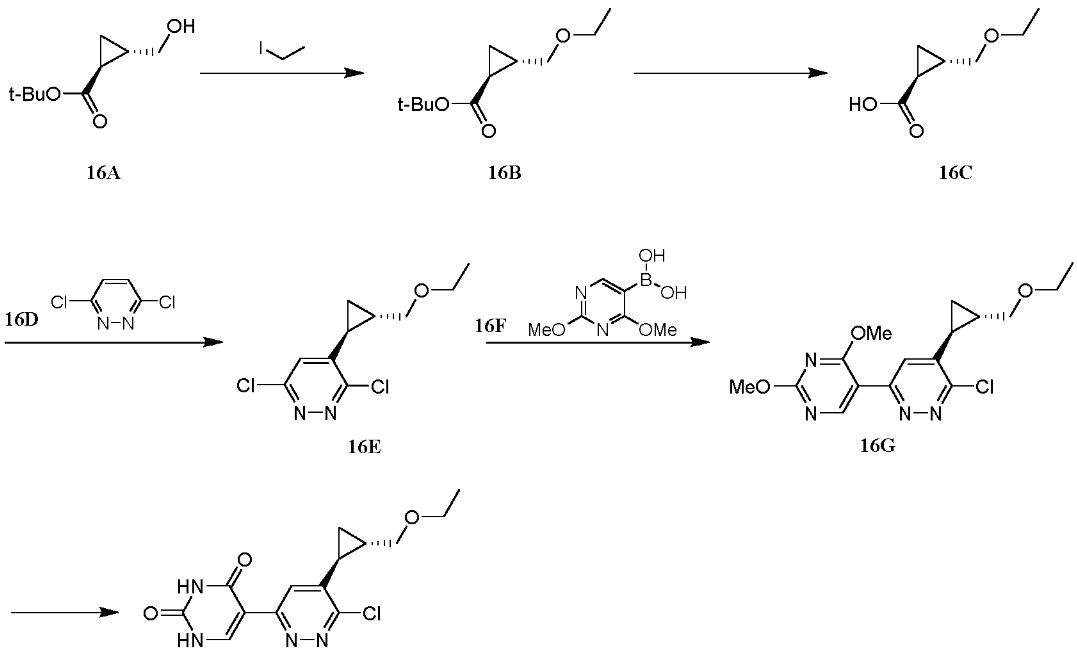


16A 16B 16C 16E 16G 16

First step: synthesis of tert-butyl (lS,2S)-2-(ethoxymethyl)cyclopropanecarboxylate (16B)

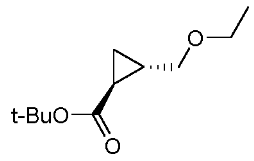

16B

Tert-butyl (IS,2S)-2-(hydroxymethyl)cyclopropanecarboxylate (2 g, 11.6 mmol), iodoethane (3.62 g, 23.2 mmol, 2.18 mL), and silver oxide (I) (5.38 g, 23.2 mmol) were dissolved in toluene (15 mL), and the reaction solution reacted at 70°C for 24 h. After the reaction was completed, the reaction solution was filtered, an obtained residue was washed with ethyl acetate (20 mL × 2), and obtained filtrates were combined. A combined filtrate was concentrated to obtain a yellow oily compound tert-butyl (IS,2S)-2-(ethoxymethyl)cyclopropanecarboxylate (16B) (2.00 g, crude product).

Second step: synthesis of (IS,2S)-2-(ethoxymethyl)cyclopropanecarboxylic acid (16C)

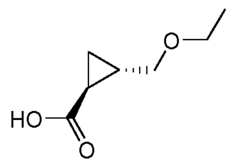

16C

Tert-butyl (IS,2S)-2-(ethoxymethyl)cyclopropanecarboxylate (2.00 g, 9.33 mmol) was dissolved in a dioxane solution (4 M, 23.3 mL) of hydrogen chloride. The reaction solution reacted at 20°C for 2 h. After the reaction was completed, the reaction solution was concentrated to obtain (IS,2S)-2-(ethoxymethyl)cyclopropanecarboxylic acid (16C) (1.90 g, crude product).

Third step: synthesis of 3,6-dichloro-4-((IS,2S)-2-(ethoxymethyl)cyclopropyl)pyridazine (16E)

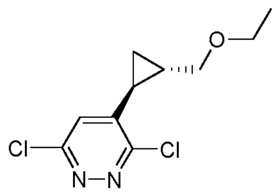

16E

(IS,2S)-2-(ethoxymethyl)cyclopropanecarboxylic acid (1.90 g, 11.9 mmol), 3,6-dichloropyridazine (1.79 g, 12.0 mmol), and sulfuric acid (3.30 g, 33.6 mmol, 1.79 mL) were dissolved in water (15 mL), and under the protection of nitrogen gas, the reaction solution reacted at 70°C for 30 min. Then, silver nitrate (816.1 mg, 4.80 mmol) was dissolved in water (2 mL) and added to the reaction solution, and then ammonium persulfate (8.22 g, 36.0 mmol) was dissolved in water (20 mL) and added dropwise to the reaction solution. Then, the reaction solution reacted at 70°C for 1 h. After the reaction was completed, the reaction solution was regulated with ammonia water until the pH was about 9, and extracted with ethyl acetate (20 mL × 2), and organic phases were combined, washed with a salt solution (50 mL), dried with anhydrous sodium sulfate, filtered, concentrated, and then separated by high-performance liquid chromatography (chromatographic column: Phenomenex luna C18 (150 mm × 40 mm, 15 μm); mobile phases: A: water + 0.225 vol% of formic acid (99%), B: acetonitrile; gradient: 18%-48%, 7.5 min) to obtain a yellow oily compound 3,6-dichloro-4-((IS,2S)-2-(ethoxymethyl)cyclopropyl)pyridazine (16E) (250 mg, yield=9.28%).

LC-MS, M/Z: 247.1 $[M+H]^+$.

Fourth step: synthesis of 3-chloro-6-(2,4-dimethoxypyrimidin-5-yl)-4-((lS,2S)-2-(ethoxymethyl)cyclopropyl)pyridazine (16G)

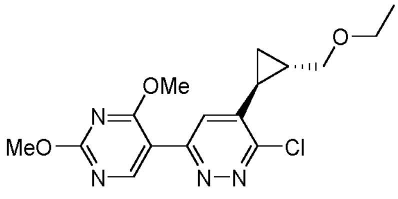

16G

3,6-dichloro-4-((lS,2S)-2-(ethoxymethyl)cyclopropyl)pyridazine (250 mg, 765 μmol), (2,4-dimethoxypyrimidin-5-yl)boric acid (140 mg, 765 μmol), 1,1'-bis(diphenylphosphino)ferrocene-palladium dichloride dichloromethane complex (62.5 mg, 76.5 μmol), and sodium carbonate (202 mg, 1.91 mmol) were dissolved in dioxane (3 mL) and water (0.5 mL), and under the protection of nitrogen gas, the reaction solution reacted at 50°C for 2 h. After the reaction was completed, the reaction solution was poured into water (10 mL), the mixture was extracted with ethyl acetate (20 mL × 2), and organic phases were combined, washed with a salt solution (50 mL), dried with anhydrous sodium sulfate, filtered, concentrated, and separated by high-performance liquid chromatography (chromatographic column: Phenomenex luna C18 (150 mm × 40 mm, 15 μm); mobile phases: A: water + 0.225 vol% of formic acid (99%), B: acetonitrile; gradient: 47%-67%, 7 min) to obtain a yellow oily compound 3-chloro-6-(2,4-dimethoxypyrimidin-5-yl)-4-((lS,2S)-2-(ethoxymethyl)cyclopropyl)pyridazine (16G) (80.0 mg, yield=21.2%).

LC-MS, M/Z: 351.1 $[M+H]^+$.

Fifth step: synthesis of 5-(6-chloro-5-((lS,2S)-2-(ethoxymethyl)cyclopropyl)pyridazin-3-yl)-1H-pyrimidine-2,4-dione (target compound 16)

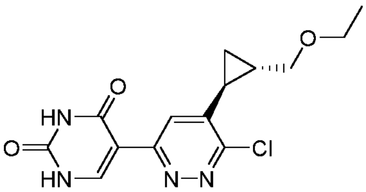

16

3-chloro-6-(2,4-dimethoxypyrimidin-5-yl)-4-((1S,2S)-2-(ethoxymethyl)cyclopropyl)pyridazine (80.0 mg, 175 μmol) was dissolved in hydrochloric acid (1 M, 2.00 mL), and the reaction solution reacted at 50°C for 12 h. After the reaction was completed, the reaction solution was concentrated, and then separated by high-performance liquid chromatography (chromatographic column: Phenomenex luna C18 (150 mm × 40 mm, 15 μm); mobile phases: A: water + 0.225 vol% of formic acid (99%), B: acetonitrile; gradient: 18%-48%, 7 min) to obtain a white solid compound 5-(6-chloro-5-((1S,2S)-2-(ethoxymethyl)cyclopropyl)pyridazin-3-yl)-1H-pyrimidine-2,4-dione (16) (31.0 mg, yield=54.9%).

$^1$H NMR (400 MHz, CDsOD): δ 8.38 (s, 1H), 8.02 (s, 1H), 3.54-3.60 (m, 4H), 2.18-2.22 (m, 1H),1.59-1.61 (m, 1H), 1.18-1.26 (m, 5H).

LC-MS, M/Z: 323.1 $[M+H]^+$.

Example 17: preparation of target compound 17

5-(6-chloro-5-((1S,2S)-2-(isopropoxymethyl)cyclopropyl)pyridazin-3-yl)-1H-pyrimidine-2,4-dione (target compound 17)

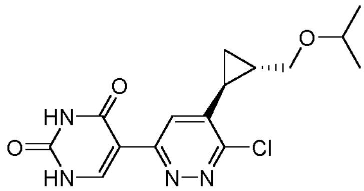

17

The synthesis route of target compound 17 was shown as follows:

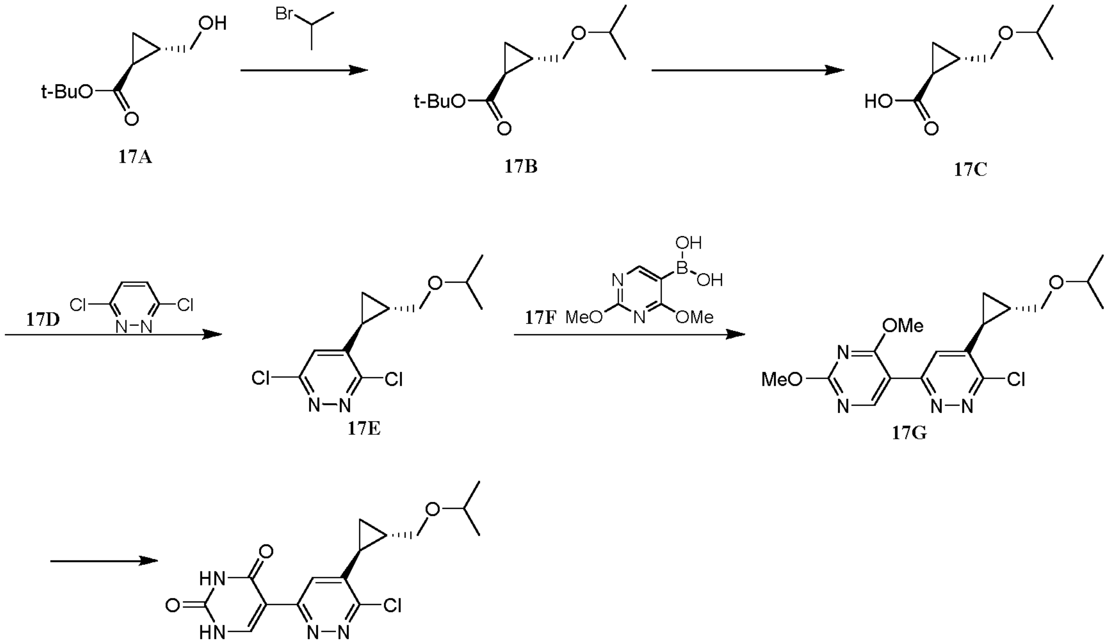


17A 17B 17C 17E 17G 17

First step: synthesis of tert-butyl (1S,2S)-2-(isopropoxymethyl)cyclopropanecarboxylate (17B)

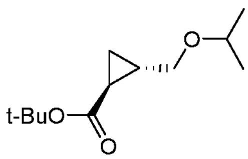

17B

Tert-butyl (lS,2S)-2-(hydroxymethyl)cyclopropanecarboxylate (2.00 g, 11.6 mmol), 2-bromopropane (2.86 g, 23.2 mmol, 2.18 mL), and silver oxide (I) (5.38 g, 23.2 mmol) were dissolved in toluene (15 mL), and the reaction solution reacted at 70°C for 24 h. After the reaction was completed, the reaction solution was filtered, an obtained residue was washed with ethyl acetate (20 mL × 2), obtained filtrates were combined, and a combined filtrate was concentrated to obtain a yellow oily compound tert-butyl (1S,2S)-2-(isopropoxymethyl)cyclopropanecarboxylate (17B) (2.00 g, crude product).

Second step: synthesis of (lS,2S)-2-(isopropoxymethyl)cyclopropanecarboxylic acid (17C)

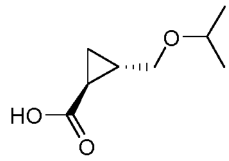

17C

Tert-butyl (IS,2S)-2-(isopropoxymethyl)cyclopropanecarboxylate (2.00 g, 9.33 mmol) was dissolved in a dioxane solution (4 M, 23.3 mL) of hydrogen chloride. The reaction solution reacted at 20°C for 2 h. After the reaction was completed, the reaction solution was concentrated to obtain (1S,2S)-2-(isopropoxymethyl)cyclopropanecarboxylic acid (17C) (1.90, crude product).

Third step: synthesis of 3,6-dichloro-4-((IS,2S)-2-(isopropoxymethyl)cyclopropyl)pyridazine (17E)

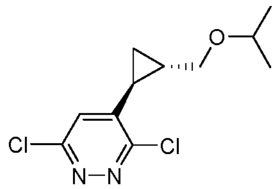

17E

(IS,2S)-2-(isopropoxymethyl)cyclopropanecarboxylic acid (1.90 g, 12.0 mmol), 3,6-dichloropyridazine (1.79 g, 12.0 mmol), and sulfuric acid (3.30 g, 33.6 mmol, 1.79 mL) were dissolved in water (15 mL), and under the protection of nitrogen gas, the reaction solution reacted at 70°C for 30 min. Then, silver nitrate (816.1 mg, 4.80 mmol) was dissolved in water (2 mL) and added to the reaction solution, and ammonium persulfate (8.22 g, 36.0 mmol) was dissolved in water (20 mL) and added dropwise to the reaction solution. Then, the reaction solution reacted at 70°C for 1 h. After the reaction was completed, the reaction solution was regulated with ammonia water until the pH was about 9, and extracted with ethyl acetate (20 mL × 2), and organic phases were combined, washed with a salt solution (50 mL), dried with anhydrous sodium sulfate, filtered, concentrated, and separated by high-performance liquid chromatography (chromatographic column: Phenomenex luna C18 (150 mm × 40 mm, 15 μm); mobile phases: A: water + 0.225 vol% of formic acid (99%), B: acetonitrile; gradient: 20%-55%, 7.5 min) to obtain a yellow oily compound 3,6-dichloro-4-((IS,2S)-2-(isopropoxymethyl)cyclopropyl)pyridazine (17E) (250 mg, yield=8.20%).

LC-MS, M/Z: 261.1 $[M+H]^+$.

Fourth step: synthesis of 3-chloro-6-(2,4-dimethoxypyrimidin-5-yl)-4-((IS,2S)-2-(isopropoxymethyl)cyclopropyl)pyridazine (17G)

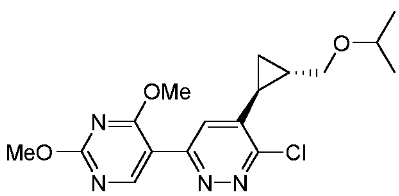

17G

3,6-dichloro-4-((IS,2S)-2-(isopropoxymethyl)cyclopropyl)pyridazine (250 mg, 765 μmol), (2,4-dimethoxypyrimidin-5-yl)boric acid (140 mg, 765 μmol), 1,1'-bis(diphenylphosphino)ferrocene-palladium dichloride dichloromethane complex (62.5 mg, 76.5 μmol), and sodium carbonate (202 mg, 1.91 mmol) were dissolved in dioxane (3 mL) and water (0.5 mL), and under the protection of nitrogen gas, the reaction solution reacted at 50°C for 2 h. After the reaction was completed, the reaction solution was poured into water (10 mL), the mixture was extracted with ethyl acetate (20 mL × 2), and organic phases were combined, washed with a salt solution (50 mL), dried with anhydrous sodium sulfate, filtered,

concentrated, and separated and purified by silica gel column (petroleum ether: ethyl acetate (v/v) = (10: 1) to (1: 1)) to obtain a yellow oily compound 3-chloro-6-(2,4-dimethoxypyrimidin-5-yl)-4-((1S,2S)-2-(isopropoxymethyl)cyclopropyl)pyridazine (17G) (100 mg, crude product).

LC-MS, M/Z: 365.1 $[M+H]^+$.

Fifth step: synthesis of 5-(6-chloro-5-((1S,2S)-2-(isopropoxymethyl)cyclopropyl)pyridazin-3-yl)-1H-pyrimidine-2,4-dione (target compound 17)

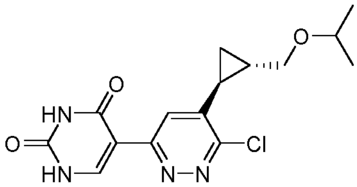

17

3-chloro-6-(2,4-dimethoxypyrimidin-5-yl)-4-((1S,2S)-2-(isopropoxymethyl)cyclopropyl)pyridazine (100 mg, 135 μmol) was dissolved in hydrochloric acid (1 M, 2.00 mL), and the reaction solution reacted at 50°C for 12 h. After the reaction was completed, the reaction solution was concentrated to obtain a product, and the product was separated by high-performance liquid chromatography (chromatographic column: Phenomenex luna C18 (150 mm × 40 mm, 15 μm); mobile phases: A: water + 0.225 vol% of formic acid (99%), B: acetonitrile; gradient: 22%-52%, 7 min) to obtain a yellow solid compound 5-(6-chloro-5-((lS,2S)-2-(isopropoxymethyl)cyclopropyl)pyridazin-3-yl)-1H-pyrimidine-2,4-dione (17) (11.0 mg, yield=16.3%).

$^1H$ NMR (400 MHz, CDsOD): δ 8.38 (s, 1H), 8.02 (s, 1H), 3.66-3.69 (m, 1H), 3.59-3.62 (m, 1H),3.52-3.59 (m, 1H), 2.17-2.19 (m, 1H),1.57-1.58 (m, 1H),1.24-1.26 (m, 1H), 1.20-1.24 (m, 1H),1.18-1.19 (m, 3H),1.16-1.18 (m, 3H).

LC-MS, M/Z: 337.1 $[M+H]^+$.

Example 18: preparation of target compound 18

5-(6-chloro-5-((1S,2R)-2-(2,2-difluoroethyl)cyclopropyl)pyridazin-3-yl)pyrimidine-2,4(1H,3H)-dione (target compound 18)

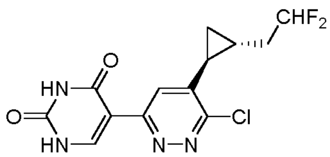

18

The synthesis route of target compound 18 was shown as follows:

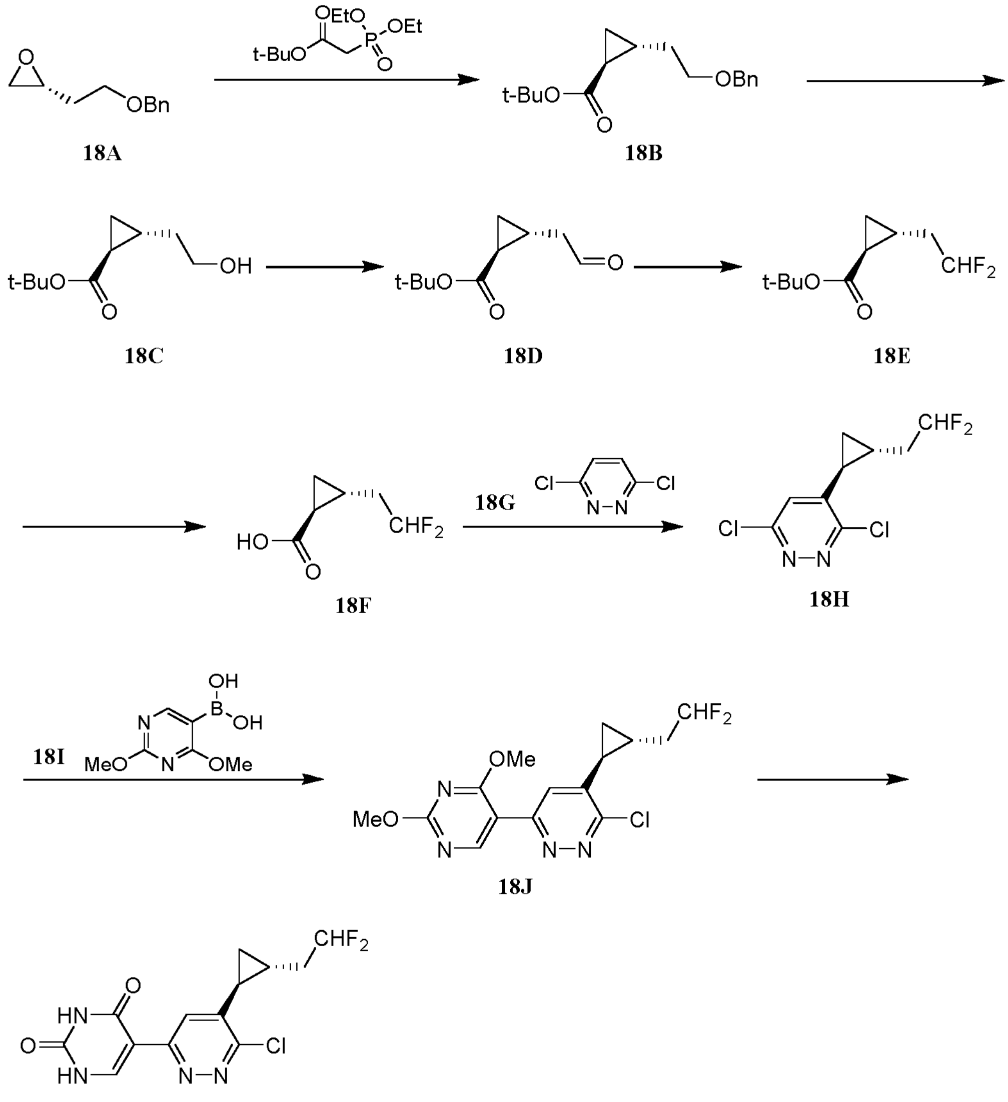


18A 18B 18C 18D 18E 18F 18H 18J 18

First step: synthesis of tert-butyl (IS,2R)-2-(2-(benzyloxy)ethyl)cyclopropanecarboxylate (18B)

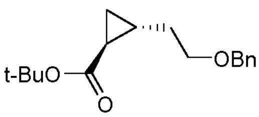

18B

Under the protection of nitrogen gas, sodium hydride (1.46 g, 36.5 mmol, 60%) was suspended in toluene (50 mL), tert-butyl 2-ethoxyphosphoryl acetate (8.49 g, 33.7 mmol) was added dropwise at 0°C to 10°C, the reaction solution was stirred at 25°C for 1 h after the dropwise addition was completed, then (R)-2-(2-(benzyloxy)ethyl)oxirane (5.00 g, 28.1 mmol) was added to the reaction solution, and the reaction solution was heated to 130°C and reacted for 3 h. The reaction mixture was diluted with a saturated ammonium chloride aqueous solution (40 mL) and extracted with ethyl acetate (40 mL × 2), and organic phases were combined, washed with a saturated salt solution (20 mL), dried with sodium sulfate, and concentrated to obtain a crude product. The crude product was separated and purified by silica gel column (petroleum ether: ethyl acetate (v/v) = (300: 1) to (50: 1)) to obtain a yellow oily compound tert-butyl (IS,2R)-2-(2-(benzyloxy)ethyl)cyclopropanecarboxylate (18B) (4.50 g, yield=58.0%).

Second step: synthesis of tert-butyl (IS,2R)-2-(2-hydroxyethyl)cyclopropanecarboxylate (18C)

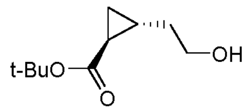

18C

Tert-butyl (1S,2R)-2-(2-(benzyloxy)ethyl)cyclopropanecarboxylate (4.5 g, 16.3 mmol) was dissolved in ethanol (50 mL), under the protection of nitrogen gas, Pd/C (1.4 g, 10%) was added, and the reaction solution was subjected to hydrogen gas replacement 3 times and reacted under 50 Psi at 60°C for 24 h. After the reaction was completed, the reaction solution was filtered with diatomite, an obtained filter cake was washed twice with ethanol, and a filtrate was collected and concentrated to obtain a colorless oily compound tert-butyl (IS,2R)-2-(2-hydroxyethyl)cyclopropanecarboxylate (18C) (3.20 g, crude product).

Third step: synthesis of tert-butyl (IS,2R)-2-(2-oxoethyl)cyclopropanecarboxylate (18D)

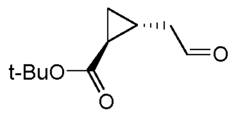

18D

Tert-butyl (IS,2R)-2-(2-hydroxyethyl)cyclopropanecarboxylate (3.10 g, 16.6 mmol) was dissolved in dichloromethane (40 mL), Dess-Martin periodinane (10.6 g, 25.0 mmol) was added in batches at 0°C to 10°C under the protection of nitrogen gas, and then the reaction solution reacted at 25°C for 2 h. A saturated sodium sulfite aqueous solution (30 mL) was added to the reaction solution to quench the reaction, the mixture was extracted with dichloromethane (30 mL × 3), and organic phases were combined, washed with a saturated salt solution (20 mL × 2), dried with sodium sulfate, and concentrated to obtain a crude product. The crude product was separated and purified by silica gel column (petroleum ether: ethyl acetate (v/v) = (200: 1) to (30: 1)) to obtain a colorless oily compound tert-butyl (1S,2R)-2-(2-oxoethyl)cyclopropanecarboxylate (18D) (2.20 g, yield=71.7%).

Fourth step: synthesis of tert-butyl (IS,2R)-2-(2,2-difluoroethyl)cyclopropanecarboxylate (18E)

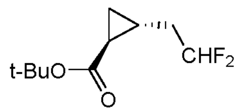

18E

Tert-butyl (1S,2R)-2-(2-oxoethyl)cyclopropanecarboxylate (2.1 g, 11.4 mmol) was dissolved in dichloromethane (20 mL), diethylaminosulfur trifluoride (4.04 g, 25.1 mmol) was added in batches at 0°C to 10°C under the protection of nitrogen gas, and then the reaction solution reacted at 25°C for 1 h. A saturated sodium bicarbonate aqueous solution (40 mL) was added to the reaction mixture to quench the reaction, the mixture was extracted with dichloromethane (30 mL × 2), and organic phases were combined, washed with a saturated salt solution (10 mL × 2), dried with sodium sulfate, and concentrated to obtain a crude product. The crude product was separated and purified by silica gel column (petroleum ether: ethyl acetate (v/v) = (200: 1) to (30: 1)) to obtain a yellow oily compound tert-butyl (IS,2R)-2-(2,2-difluoroethyl)cyclopropanecarboxylate (18E) (1.15 g, yield=48.9%).

Fifth step: synthesis of (1S,2R)-2-(2,2-difluoroethyl)cyclopropanecarboxylic acid (18F)

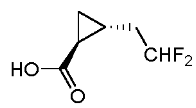

18F

Tert-butyl (1S,2R)-2-(2,2-difluoroethyl)cyclopropanecarboxylate (650 mg, 3.15 mmol) was dissolved in a dioxane solution (5 mL, 4 M) of hydrochloric acid, and the reaction solution reacted at 25°C for 1 h. The reaction mixture was concentrated under reduced pressure to obtain a colorless oily compound (1S,2R)-2-(2,2-difluoroethyl)cyclopropanecarboxylic acid (18F) (475 mg, crude product).

Sixth step: synthesis of 3,6-dichloro-4-((lS,2R)-2-(2,2-difluoroethyl)cyclopropyl)pyridazine (18H)

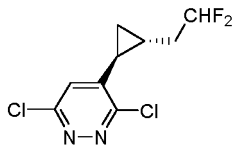

18H

3,6-dichloropyridazine (466 mg, 3.13 mmol) and (1S,2R)-2-(2,2-difluoroethyl)cyclopropanecarboxylic acid (470 mg, 3.13 mmol) were dissolved in water (4.0 mL), concentrated sulfuric acid (467 μL) was added, and under the protection of nitrogen gas, the reaction solution was heated to 70°C. Then, an aqueous solution (170 mg, 1.00 mmol, 4.4 mL) of silver nitrate was added quickly, an aqueous solution (1.60 g, 6.99 mmol, 4.0 mL) of ammonium persulfate was added slowly dropwise, and the reaction solution reacted at 70°C for 1 h. The reaction solution was regulated with ammonia water until the pH was about 9, and extracted with ethyl acetate (20 mL × 3), and organic phases were combined, washed with a saturated salt solution (10 mL × 2), dried with sodium sulfate, and concentrated to obtain a crude product. The crude product was separated together with the previous batch of product by reversed-phase flash chromatography to obtain a yellow oily compound 3,6-dichloro-4-((1S,2R)-2-(2,2-difluoroethyl)cyclopropyl)pyridazine (18H) (210 mg, yield=15.2%).

LC-MS, M/Z (ESI): 253.0 $[M+H]^+$.

Seventh step: synthesis of 3-chloro-4-((lS,2R)-2-(2,2-difluoroethyl)cyclopropyl)-6-(2,4-dimethoxypyrimidin-5-yl)pyridazine (18J)

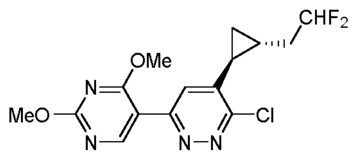

18J

3,6-dichloro-4-((lS,2R)-2-(2,2-difluoroethyl)cyclopropyl)pyridazine (200 mg, 790.3 umol) and 2,4-dimethoxypyrimidine-5-boric acid (145.4 mg, 790.3 umol) were dissolved in dioxane (8.0 mL) and water (2.0 mL), sodium carbonate (251.3 mg, 2.37 mmol) and [1,1'-bis(diphenylphosphino)ferrocene]dichloropalladium(II) (115.6 mg, 158.1 μmol) were added under the protection of nitrogen gas, and the reaction solution was heated to 50°C and reacted for 8 h. The reaction mixture was concentrated under reduced pressure to obtain a crude product. The crude product was separated by reversed phase high-performance liquid chromatography (chromatographic column: 3_Phenomenex Luna C18 (75 mm × 30 mm, 3 μm), mobile phases: A: water + 0.1 vol% of trifluoroacetic acid (99.9%), B: acetonitrile; gradient: 48%-68%, 7 min) to obtain a white solid compound 3-chloro-4-((1S,2R)-2-(2,2-difluoroethyl)cyclopropyl)-6-(2,4-dimethoxypyrimidin-5-yl)pyridazine (18J) (95 mg, yield=33.7%).

LC-MS, M/Z (ESI): 357.0 $[M+H]^+$.

Eighth step: synthesis of 5-(6-chloro-5-((lS,2R)-2-(2,2-difluoroethyl)cyclopropyl)pyridazin-3-yl)pyrimidine-2,4(1H,3H)-dione (18)

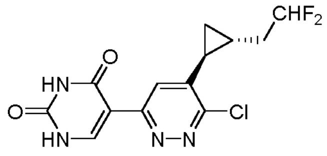

18

3-chloro-4-((1S,2R)-2-(2,2-difluoroethyl)cyclopropyl)-6-(2,4-dimethoxypyrimidin-5-yl)pyridazine (90 mg, 252.3 μmol) was added to a hydrochloric acid aqueous solution (1 M, 2.0 mL), and the reaction solution was heated to 50°C and reacted for 8 h. The reaction system was concentrated to dryness to obtain a crude product, and the crude product was separated by reversed phase high-performance liquid chromatography (chromatographic column: 3_Phenomenex Luna C18 (75 mm × 30 mm, 3 μm), mobile phases: A: water + 0.1 vol% of trifluoroacetic acid (99%), B: acetonitrile; gradient: 30%-50%, 7 min) to obtain a white solid compound 5-(6-chloro-5-((lS,2R)-2-(2,2-difluoroethyl)cyclopropyl)pyridazin-3-yl)pyrimidine-2,4(lH,3H)-dione (18) (35.3 mg, yield=42.6%).

$^1$H NMR (400 MHz, DMSO_$d_6$): δ 11.53 (s, 2H), 8.29 (s, 1H),7.90 (s, 1H), 6.02-6.30 (m, 1H), 2.05-2.12 (m, 3H), 1.13-1.25 (m, 3H).

LC-MS, M/Z (ESI): 329.0 $[M+H]^+$.

Example 19: preparation of target compound 19

5-(6-chloro-5-((1R,2S)-2-(2,2-difluoroethyl)cyclopropyl)pyridazin-3-yl)pyrimidine-2,4(1H,3H)-dione (target compound 19)

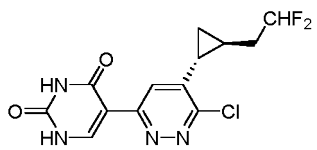

19

The synthesis route of target compound 19 was shown as follows:

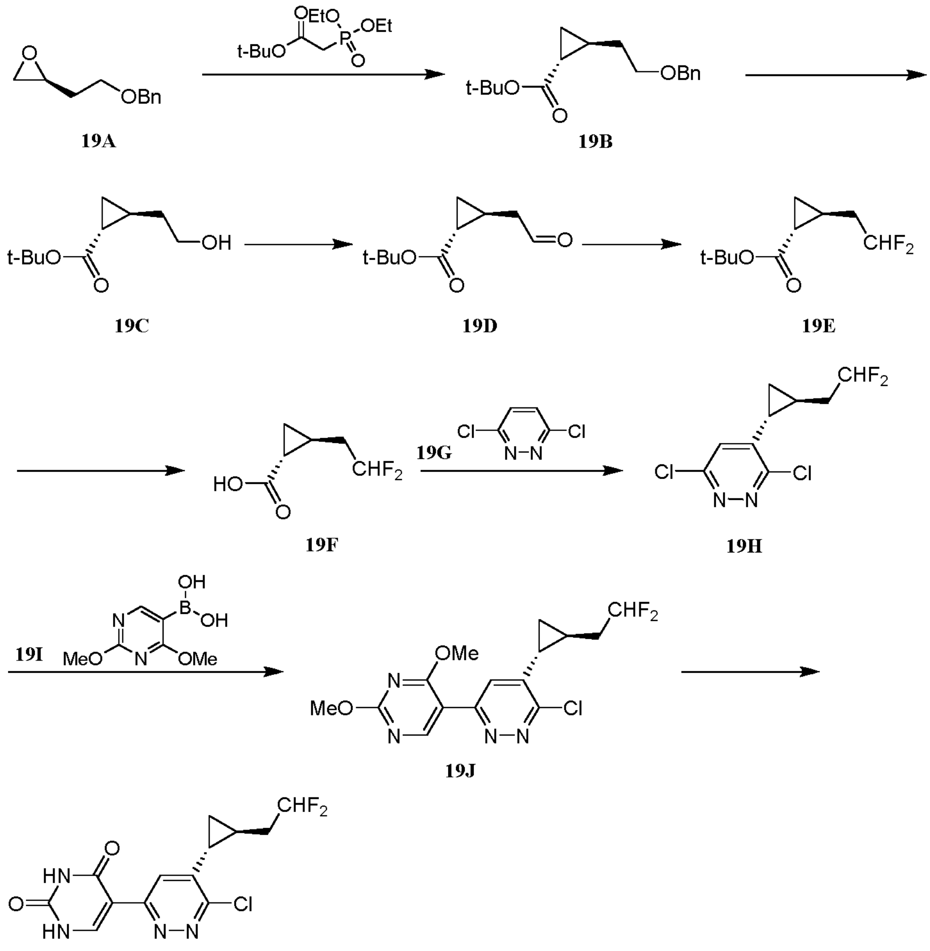


19A 19B 19C 19D 19E 19F 19H 19J 19

First step: synthesis of tert-butyl (1R,2S)-2-(2-(benzyloxy)ethyl)cyclopropanecarboxylate (19B)

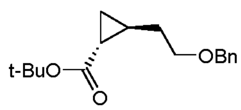

19B

Under the protection of nitrogen gas, sodium hydride (1.40 g, 35.0 mmol, 60%) was suspended in toluene (50 mL), tert-butyl 2-ethoxyphosphoryl acetate (7.06 g, 28.1 mmol) was added dropwise at 0°C to 10°C, the reaction solution was stirred at 25°C for 1 h after the dropwise addition was complete, then (S)-2-(2-(benzyloxy)ethyl)oxirane (5.00 g, 28.1 mmol) was added to the reaction solution, and the reaction solution was heated to 130°C and reacted for 3 h. The reaction mixture was diluted with a saturated ammonium chloride aqueous solution (40 mL) and extracted with ethyl acetate (40 mL × 2), and organic phases were combined, washed with a saturated salt solution (20 mL), dried with sodium sulfate, and concentrated to obtain a crude product. The crude product was separated and purified by silica gel column (petroleum ether: ethyl acetate (v/v) = (1: 0) to (10: 1)) to obtain a yellow oily compound tert-butyl (IR,2S)-2-(2-(benzyloxy)ethyl)cyclopropanecarboxylate (19B) (5.00 g, yield=64.5%).

Second step: synthesis of tert-butyl (IR,2S)-2-(2-hydroxyethyl)cyclopropanecarboxylate (19C)

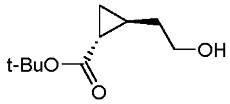

19C

Tert-butyl (1R,2S)-2-(2-(benzyloxy)ethyl)cyclopropanecarboxylate (4.50 g, 16.3 mmol) was dissolved in ethanol (50 mL), Pd/C (1.40 g, 10%) was added under the protection of nitrogen gas, and the reaction solution was subjected to hydrogen gas replacement 3 times and reacted under 50 Psi at 60°C for 24 h. After the reaction was completed, the reaction solution was cooled and filtered with diatomite, an obtained filter cake was washed with ethanol, and a filtrate was collected and concentrated to obtain a colorless oily compound tert-butyl (lR,2S)-2-(2-hydroxyethyl)cyclopropanecarboxylate (19C) (2.80 g, crude product).

Third step: synthesis of tert-butyl (lR,2S)-2-(2-oxoethyl)cyclopropanecarboxylate (19D)

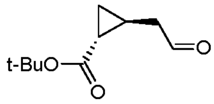

19D

Tert-butyl (lR,2S)-2-(2-hydroxyethyl)cyclopropanecarboxylate (1.00 g, 5.37 mmol) was dissolved in dichloromethane (10 mL), Dess-Martin periodinane (3.42 g, 8.05 mmol) was added in batches at 0°C to 10°C under the protection of nitrogen gas, and then the reaction solution reacted at 25°C for 2 h. A saturated sodium sulfite aqueous solution (15 mL) was added to the reaction mixture to quench the reaction, the mixture was extracted with dichloromethane (10 mL × 2), and organic phases were combined, washed with a saturated salt solution (30 mL × 2), dried with sodium sulfate, and concentrated to obtain a crude product. The crude product was separated and purified by silica gel column (petroleum ether: ethyl acetate (v/v) = (200: 1) to (30: 1)) to obtain a yellow oily compound tert-butyl (1R,2S)-2-(2-oxoethyl)cyclopropanecarboxylate (19D) (730 mg, yield=73.8%).

Fourth step: synthesis of tert-butyl (1R,2S)-2-(2,2-difluoroethyl)cyclopropanecarboxylate (19E)

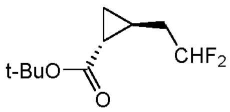

19E

Tert-butyl (1R,2S)-2-(2-oxoethyl)cyclopropanecarboxylate (730 mg, 3.96 mmol) was dissolved in dichloromethane (12 mL), diethylaminosulfur trifluoride (1.41 g, 8.72 mmol) was added in batches at 0°C to 10°C under the protection of nitrogen gas, and then the reaction solution reacted at 25°C for 1 h. A saturated sodium bicarbonate aqueous solution (20 mL) was added to the reaction mixture to quench the reaction, the mixture was extracted with dichloromethane (15 mL × 3), and organic phases were combined, washed with a saturated salt solution (20 mL × 2), dried with sodium sulfate, and concentrated to obtain a crude product. The crude product was separated and purified by silica gel column (petroleum ether: ethyl acetate (v/v) = (100: 1) to (10: 1)) to obtain a yellow oily compound tert-butyl (1R,2S)-2-(2,2-difluoroethyl)cyclopropanecarboxylate (19E) (510 mg, yield=62.4%).

Fifth step: synthesis of (lR,2S)-2-(2,2-difluoroethyl)cyclopropanecarboxylic acid (19F)

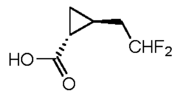

19F

Tert-butyl (1R,2S)-2-(2,2-difluoroethyl)cyclopropanecarboxylate (500 mg, 2.42 mmol) was dissolved in a dioxane solution (8 mL, 4 M) of hydrochloric acid, and the reaction solution reacted at 25°C for 2 h. The reaction mixture was concentrated under reduced pressure to obtain a yellow oily compound (1R,2S)-2-(2,2-difluoroethyl)cyclopropanecarboxylic acid (19F) (360 mg, crude product).

Sixth step: synthesis of 3,6-dichloro-4-((lR,2S)-2-(2,2-difluoroethyl)cyclopropyl)pyridazine (19H)

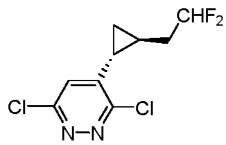

19H

3,6-dichloropyridazine (347 mg, 2.33 mmol) and (1R,2S)-2-(2,2-difluoroethyl)cyclopropanecarboxylic acid (350 mg, 2.33 mmol) were dissolved in water (3.5 mL), concentrated sulfuric acid (363 μL) was added, and under the protection of nitrogen gas, the reaction solution was heated to 70°C. Then, an aqueous solution (170 mg, 1.00 mmol, 3.5 mL) of silver nitrate was added quickly, an aqueous solution of ammonium persulfate (1.60 g, 6.99 mmol, 5.0 mL) was added slowly dropwise, and the reaction solution reacted at 70°C for 1 h. The reaction solution was regulated with ammonia water until the pH was about 9, and extracted with ethyl acetate (20 mL × 3), and organic phases were combined, washed with a saturated salt solution (20 mL × 2), dried with sodium sulfate, and concentrated to obtain a crude product. Then, the crude product was separated by reversed-phase flash chromatography to obtain a yellow oily compound 3,6-dichloro-4-((lR,2S)-2-(2,2-difluoroethyl)cyclopropyl)pyridazine (19H) (330 mg, yield=55.9%).

LC-MS, M/Z (ESI): 253.0 $[M+H]^+$.

Seventh step: synthesis of 3-chloro-4-((lR,2S)-2-(2,2-difluoroethyl)cyclopropyl)-6-(2,4-dimethoxypyrimidin-5-yl)pyridazine (19J)

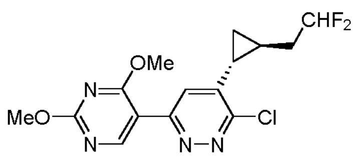

19J

3,6-dichloro-4-((lR,2S)-2-(2,2-difluoroethyl)cyclopropyl)pyridazine (330 mg, 1.30 mmol) and 2,4-dimethoxypyrimidine-5-boric acid (240 mg, 1.30 mmol) were dissolved in dioxane (10 mL) and water (2.0 mL), sodium carbonate (415 mg, 3.91 mmol) and [1,1 '-bis(diphenylphosphino)ferrocene]dichloropalladium(II) (191 mg, 111 μmol) were added under the protection of nitrogen gas, and the reaction solution was heated to 70°C and reacted for 12 h. The reaction mixture was concentrated under reduced pressure to obtain a crude product. The crude product was separated by reversed phase high-performance liquid chromatography (chromatographic column: Phenomenex Gemini-NX C18 (150 mm × 40 mm, 15 μm); mobile phases: A: water + 0.225 vol% of formic acid (99.9%), B: acetonitrile; gradient: 33%-63%, 11 min) to obtain a yellow solid compound 3-chloro-4-((lR,2S)-2-(2,2-difluoroethyl)cyclopropyl)-6-(2,4-dimethoxypyrimidin-5-yl)pyridazine (19J) (140 mg, yield=30.1%).

LC-MS, M/Z (ESI): 357.1 $[M+H]^+$.

Eighth step: synthesis of 5-(6-chloro-5-((lR,2S)-2-(2,2-difluoroethyl)cyclopropyl)pyridazin-3-yl)pyrimidine-2,4(1H,3H)-dione (19)

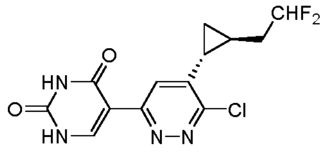

19

3-chloro-4-((1R,2S)-2-(2,2-difluoroethyl)cyclopropyl)-6-(2,4-dimethoxypyrimidin-5-yl)pyridazine (130 mg, 364 μmol) was added to a hydrochloric acid aqueous solution (1 M, 2.0 mL), and the reaction solution was heated to 50°C and reacted for 8 h. The reaction system was concentrated to dryness to obtain a crude product, and the crude product was first separated by reversed phase high-performance liquid chromatography (chromatographic column: Phenomenex Gemini-NX C18 (150 mm × 25 mm, 10 μm); mobile phases: A: water + 0.225 vol% of formic acid (99%), B: acetonitrile; gradient: 19%-49%, 10 min) and then separated by supercritical fluid chromatography (chromatographic column: DAICEL CHIRALPAK AD (250 mm × 30 mm, 10 μm), mobile phase: 0.1% ammonia methanol solution; gradient: 70%-70%, 80 min) to obtain a white solid compound 5-(6-chloro-5-((1R,2S)-2-(2,2-difluoroethyl)cyclopropyl)pyridazin-3-yl)pyrimidine-2,4(1H,3H)-dione (19) (40.3 mg, yield=49.4%).

$^1$H NMR (400 MHz, DMSO_$d_6$): δ 11.53 (s, 2H), 8.29 (s, 1H), 7.90 (s, 1H), 6.02-6.30 (m, 1H), 2.05-2.12 (m, 3H), 1.13-1.25 (m, 3H).

LC-MS, M/Z (ESI): 329.0 $[M+H]^+$.

Example 20: Preparation of target compound 20

5-(5-((1S,2S)-2-(fluoromethyl)cyclopropyl)-6-methylpyridazin-3-yl)pyrimidine-2,4(1H,3H)-dione (target compound 20)

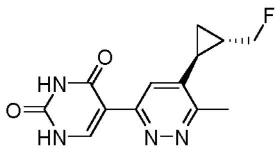

20

The synthesis route of target compound 20 was shown as follows:

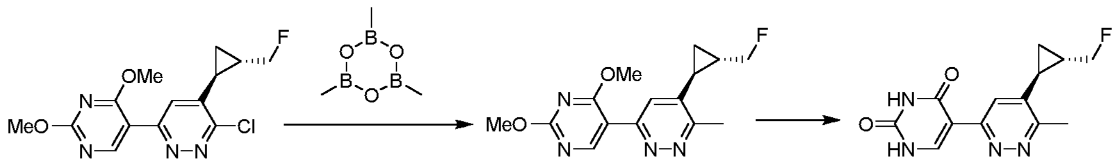

20A 20B 20

First step: synthesis of 6-(2,4-dimethoxypyrimidin-5-yl)-4-((1S,2S)-2-(fluoromethyl)cyclopropyl)-3-methylpyridazine (20B)

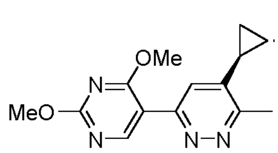

20B

Under the protection of nitrogen gas, 3-chloro-6-(2,4-dimethoxypyrimidin-5-yl)-4-((IS,2S)-2-(fluoromethyl)cyclopropyl)pyridazine (20A) (2.0 g, 6.16 mmol) (prepared with reference to Example 1), a tetrahydrofuran solution (3.5 M, 4.93 mL, 17.24 mmol) of 2,4,6-trimethyl-1,3,5,2,4,6-trioxatriborinane, cesium carbonate (6.02 g, 18.48 mmol), and [1,1'-bis(diphenylphosphino)ferrocene]dichloropalladium(II) (0.451 g, 0.616 mmol) were dissolved in dioxane (40 mL) and water (10 mL), and the reaction solution was heated to 100°C and reacted for 2 h. The reaction mixture was diluted with water (100 mL) and extracted with ethyl acetate (80 mL × 3), and organic phases were combined, dried with sodium sulfate, and concentrated to obtain a crude product. The crude product was separated by reversed phase high-performance liquid chromatography (chromatographic column: Phenomenex Luna C18 (150 mm × 25 mm, 10 μm); mobile phase: water (0.01% $NH_3.H_2O$)-acetonitrile; B%: 35%-65%, 10 min) to obtain a pale yellow solid 6-(2,4-dimethoxypyrimidin-5-yl)-4-((IS,2S)-2-(fluoromethyl)cyclopropyl)-3-methylpyridazine (20B) (1.6 g, yield=85.0%).

LC-MS, M/Z: 305.1 $[M+H]^+$.

Second step: synthesis of 5-(5-((1S,2S)-2-(fluoromethyl)cyclopropyl)-6-methylpyridazin-3-yl)pyrimidine-2,4(1H,3H)-dione (target compound 20)

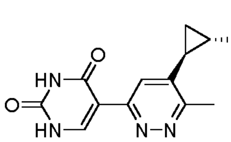

20

6-(2,4-dimethoxypyrimidin-5-yl)-4-((IS,2S)-2-(fluoromethyl)cyclopropyl)-3-methylpyridazine (20B) (1.6 g, 5.26 mmol) was dissolved in tetrahydrofuran (10 mL) and added to a hydrochloric acid aqueous solution (1 M, 26.3 mL), and the reaction solution was heated to 50°C and reacted for 20 h. The reaction solution was concentrated to remove the organic solvent, and filtered, and a white solid was collected and dried to obtain 5-(5-((IS,2S)-2-(fluoromethyl)cyclopropyl)-6-methylpyridazin-3-yl)pyrimidine-2,4(1H,3H)-dione (target compound 20) (1.1 g, yield=75.8%).

$^1H$ NMR (400 MHz, DMSO_d6) δ 11.98-11.97 (d, 1H), 11.72 (s, 1H), 8.40-8.39 (d, 1H), 8.14 (s, 1H), 4.64-4.31 (m, 2H), 2.78 (s, 3H), 2.26-2.20 (m, 1H), 1.80-1.71 (m, 1H), 1.36-1.21 (m, 2H).

LC-MS, M/Z: 277.3 $[M+H]^+$.

Example 21: Preparation of target compound 21

5-(6-methyl-5-((1S,2S)-2-((trifluoromethoxy)methyl)cyclopropyl)pyridazin-3-yl)pyrimidine-2,4(1H,3H)-dione (target compound 21)

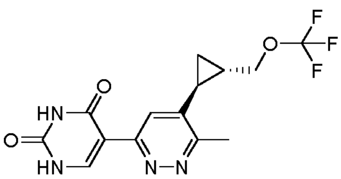

21

The synthesis route of target compound 21 was shown as follows:

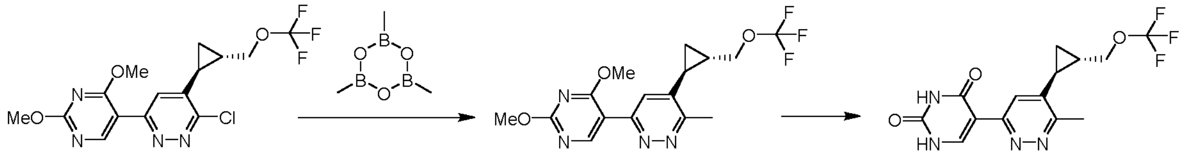

21A 21B 21

First step: synthesis of 6-(2,4-dimethoxypyrimidin-5-yl)-3-methyl-4-((lS,2S)-2-((trifluoromethoxy)methyl)cyclopropyl)pyridazine (21B)

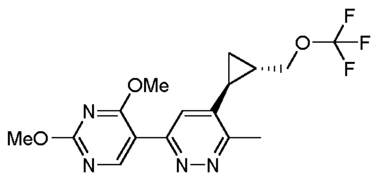

21B

Under the protection of nitrogen gas, 3-chloro-6-(2,4-dimethoxypyrimidin-5-yl)-4-((lS,2S)-2-((trifluoromethoxy)methyl)cyclopropyl)pyridazine (21A) (1.0 g, 2.56 mmol) (the intermediate was synthesized with reference to Example 11), a tetrahydrofuran solution (3.5 M, 2 mL, 7.17 mmol) of 2,4,6-trimethyl-1,3,5,2,4,6-trioxatriborinane, cesium carbonate (2.5 g, 7.68 mmol), and [l,l'-bis(diphenylphosphino)ferrocene]dichloropalladium(II) (0.187 g, 0.256 mmol) were dissolved in dioxane (20 mL) and water (5 mL), and the reaction solution was heated to 100°C and reacted for 2 h. The reaction mixture was diluted with water (50 mL) and extracted with ethyl acetate (50 mL × 3), and organic phases were combined, dried with sodium sulfate and concentrated to obtain a crude product. The crude product was separated by reversed phase high-performance liquid chromatography (column: Phenomenex Luna C18 (150 mm × 25mm, 10 μm), mobile phase: water (0.01% $NH_3.H_2O$)-ACN; B%: 35%-65%, 10 min) to obtain a pale yellow oily compound 6-(2,4-dimethoxypyrimidin-5-yl)-3-methyl-4-((lS,2S)-2-((trifluoromethoxy)methyl)cyclopropyl)pyridazine (21B) (0.8 g, yield=84.3%).

LC-MS, M/Z: 371.1 $[M+H]^+$.

Second step: synthesis of 5-(6-methyl-5-((lS,2S)-2-((trifluoromethoxy)methyl)cyclopropyl)pyridazin-3-yl)pyrimidine-2,4(1H,3H)-dione (target compound 21)

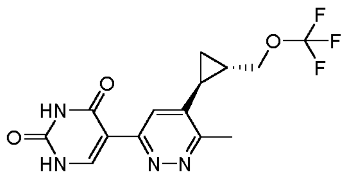

21

6-(2,4-dimethoxypyrimidin-5-yl)-3-methyl-4-((lS,2S)-2-((trifluoromethoxy)methyl)cyclopropyl)pyridazine (21B) (0.8 g, 2.16 mmol) was dissolved in tetrahydrofuran (5 mL) and added to a hydrochloric acid aqueous solution (1 M, 10.8 mL), and the reaction solution was heated to 50°C and reacted for 20 h. The reaction solution was concentrated to remove the organic solvent, and filtered, and a white solid was collected and dried to obtain 5-(6-methyl-5-((lS,2S)-2-((trifluoromethoxy)methyl)cyclopropyl)pyridazin-3-yl)pyrimidine-2,4(1H,3H)-dione (target compound 21) (0.61 g, yield=82.5%).

$^1H$ NMR (400 MHz, DMSO_d6) δ 11.85-11.83 (d, 1H), 11.67 (s, 1H), 8.36-8.35 (d, 1H), 8.05 (s, 1H), 4.26-4.12 (m, 2H), 2.74 (s, 3H), 2.27-2.22 (m, 1H), 1.72-1.67 (m, 1H), 1.34-1.25 (m, 2H).

LC-MS, M/Z: 343.3 $[M+H]^+$.

Example 22: preparation of target compound 22

5-(6-methyl-5-((IS,2R)-2-(2,2,2-trifluoroethyl)cyclopropyl)pyridazin-3-yl)pyrimidine-2,4(1H, 3H)-dione (target compound 22)

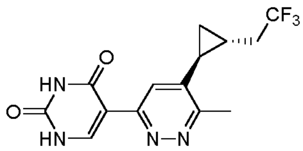

22

The synthesis route of target compound 22 was shown as follows:

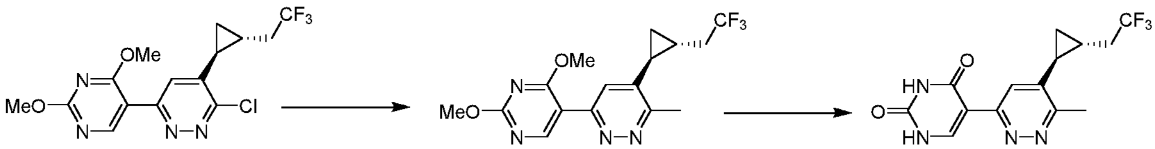

First step: synthesis of 6-(2,4-dimethoxypyrimidin-5-yl)-3-methyl-4-((1S,2R)-2-(2,2,2-trifluoroethyl)cyclopropyl)pyridazine (22B)

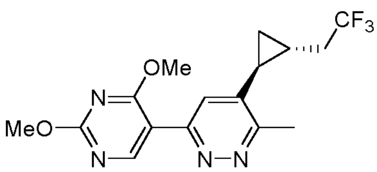

22B

3-chloro-6-(2,4-dimethoxypyrimidin-5-yl)-4-((1S,2R)-2-(2,2,2-trifluoroethyl)cyclopropyl)pyridazine (400 mg, 1.04 mmol), 2,4,6-trimethyl-1,3,5,2,4,6-trioxatriborinane (893 μL, 3.13 mmol), and sodium carbonate (331 mg, 3.13 mmol) were dissolved in dioxane (12 mL) and water (3 mL), [1,1'-bis(diphenylphosphino)ferrocene]dichloropalladium(II) (152 mg, 208 μmol) was added under the protection of nitrogen gas, and the reaction solution was heated to 100°C and reacted for 2 h. After the reaction was completed, the reaction system was spin-dried to obtain a crude product. The crude product was separated and purified by silica gel column (petroleum ether: ethyl acetate (v/v) = (2: 1) to (1: 2)) to obtain a brown oily compound 6-(2,4-dimethoxypyrimidin-5-yl)-3-methyl-4-((1S,2R)-2-(2,2,2-trifluoroethyl)cyclopropyl)pyridazine (2) (400 mg, yield=75.6%).

LC-MS, M/Z (ESI): 355.3 $[M+H]^+$.

Second step: synthesis of 5-(6-methyl-5-((IS,2R)-2-(2,2,2-trifluoroethyl)cyclopropyl)pyridazin-3-yl)pyrimidine-2,4(1H, 3H)-dione (target compound 22)

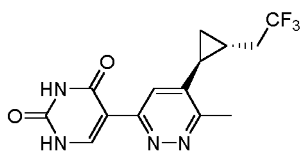

22

6-(2,4-dimethoxypyrimidin-5-yl)-3-methyl-4-((lS,2R)-2-(2,2,2-trifluoroethyl)cyclopropyl)pyridazine (400 mg, 853 umol) was dissolved in tetrahydrofuran (2.00 mL), hydrochloric acid (1 M, 10.0 mL) was added, and the reaction solution reacted at 50°C for 12 h. After the reaction was completed, the reaction solution was concentrated to obtain a crude product, and the crude product was first separated by reversed phase high-performance liquid chromatography (chromatographic column: Phenomenex Gemini-NX C18 (75 mm × 30 mm, 3 μm), mobile phases: A: water + 0.225 vol% of formic acid (99%), B: acetonitrile; gradient: 12%-42%, 7 min), lyophilized, and then separated by supercritical fluid chromatography (column: DAICEL CHIRALPAK AD (250 mm × 30 mm, 10μm); solvent: 0.1% ammonia methanol solution; gradient: 40%-40%, 45 min) to obtain a white solid compound 5-(6-methyl-5-((1S,2R)-2-(2,2,2-trifluoroethyl)cyclopropyl)pyridazin-3-yl)pyrimidine-2,4(1H, 3H)-dione (22) (96.1 mg, yield=34.1%).

LC-MS, M/Z (ESI): 326.9 $[M+H]^+$.

SFC: Rt = 1.641 min, de% = 100%, detection method: column: Chiralpak AD-3 (50 mm × 4.6 mm I.D., 3 um); mobile phases: A: $CO_2$, B: 0.05% diethylamine methanol solution; eluent: 40% methanol (containing 0.05% diethylamine) and 60% carbon dioxide; flow rate: 3 mL/min; detector: PDA column temperature: 35°C; column pressure: 100 Bar.

$^1$H NMR (400 MHz, CDsOD): δ 8.29 (s, 1H), 7.86 (s, 1H), 2.77 (s, 3H), 2.37-2.44 (m, 2H), 2.02-2.05 (m, 1H), 1.41-1.43 (m, 1H), 1.18-1.22 (m, 2H).

Test Example 1: in vitro inhibitory activity of compound to recombinant human CD73 enzyme

The test was performed using Tris-MgCh buffer containing 25 mM Tris (B io sharp; 77-86-1) and 25 mM $MgCl_2$ (Nanjing Chemical Reagent Co., Ltd.; 7791-18-6). Human-CD73 (Novoprotein; C446) was diluted with Tris-MgCh buffer to form a stock solution (3×), which was placed in a 96-well plate according to 20 μL/well to obtain a final concentration of 0.1 μg/mL. The compound was diluted with Tris-$MgCl_2$ buffer to form a stock solution (3 ×) of an appropriate concentration gradient, the compound solution was added to the above 96-well plate according to 20 μL/well and uniformly mixed with the Human-CD73 solution, and the mixture was incubated at the room temperature for 30 min. Meanwhile, a positive reference group (without the compound) and a negative reference group (without CD73) were set. AMP (Sigma; A1752-5G) was diluted with Tris-MgCh buffer to form a stock solution (3 ×), which was added to the above 96-well plate according to 20 μL/well to obtain a final concentration of 100 μM, and the mixture was uniformly mixed and incubated at 37°C for 60 min; ATP (Sigma; A7699-1G) was diluted with Tris-MgCh buffer to form a stock solution (7×), which was added to the above 96-well plate according to 10 μL/well to obtain a final concentration of 100 μM, and and the mixture was uniformly mixed, incubated for 5 min, and tested by using an ATP-GLO kit (Promega; G7573).

Human-CD73 inhibition rates of the compound at different concentrations were calculated according to the following formula, the concentration of the compound was taken as the X-axis, the inhibition rate was taken as the Y-axis, and an $IC_{50}$ value of the compound in inhibiting Human-CD73 was calculated by using Prism software.

$$\text{Inhibition rate (\%)} = \frac{\text{signal value of positive control group} - \text{signal value of compound group at different concentrations}}{\text{signal value of positive control group} - \text{signal value of negative control group}} \times 100\%$$

Table 1 In vitro inhibitory activity of test compounds to Human-CD73 enzyme

| Test compound | $IC_{50}$ (nM) |
|---|---|
| Reference compound 1 | 12.47 |
| Reference compound 2 | 16.84 |
| Reference compound 3 | 27.03 |
| 1 | 4.59 |
| 2 | 23.21 |
| 3 | 11.62 |
| 4 | 58.42 |
| 5 | 11.59 |
| 6 | 24.5 |
| 7 | 32.13 |

(continued)

| Test compound | $IC_{50}$ (nM) |
|---|---|
| 8 | 16.53 |
| 9 | 8.17 |
| 11 | 12.22 |
| 12 | 15.44 |
| 13 | 6871 |
| 14 | 3682 |
| 15 | 123.4 |
| 16 | 172.8 |
| 17 | 164.2 |
| 19 | 1248 |
| 20 | 9.55 |

The results of the in vitro enzyme test indicate that the compounds of the present disclosure have good inhibitory effects on CD73 enzyme, and some compounds show better inhibitory effects on CD73 enzyme compared to the reference compounds.

Test Example 2: inhibitory activity of compound to CD73 enzyme bound to the surfaces of human melanoma A375 cells

A375 cells (ATCC; CRL-1619) were cultured in DMEM (Gibco; 11995-040) containing 10% FBS (Gibco; 10099-141C) and 1% P/S (Thermo; 10378016). The cells were digested with trypsin when the cells were in good condition, centrifuged to remove a supernatant, collected, washed with serum-free DMEM, resuspended in serum-free DMEM, counted, and inoculated into a 96-well plate with a round bottom according to $1\times10^4$ cells/well and 60 μL/well, and meanwhile, a positive reference (A375+AMP) and a negative reference (AMP only) were set.

The compound was diluted with serum-free DMEM to form a stock solution (5 ×) of an appropriate concentration gradient, the compound solution was added to the cells in the above 96-well plate according to 20 μL/well, and the mixture was placed in an incubator and pre-incubated for 30 min. AMP was diluted with serum-free DMEM to form a stock solution (5×), the AMP solution was added to the cells according to 20 μL/well to obtain a final concentration of 200 μM, and the mixture was incubated at 37°C for 16 h. After the incubation was completed, the 96-well plate was centrifuged at 1,500 rpm for 3 min, a supernatant was aspirated to a new 96-well plate according to 50 μL/well, an ATP stock solution (25 ×) formed by diluting ATP with serum-free DMEM was added to the 96-well plate according to 2 μL/well to obtain a final concentration of 100 μM, then a CellTiter-Glo® Luminescent Cell Viability Assay reagent (Promega; G7573) was added in the 96-well plate according to 50 μL/well, and the mixture was uniformly mixed and tested.

A375-bound CD73 inhibition rates of the compound at different concentrations were calculated according to the following formula, the concentration of the compound was taken as the X-axis, the inhibition rate was taken as the Y-axis, and an IC50 value of the compound in inhibiting A375-bound CD73 was calculated by using Prism software.

$$\text{Inhibition rate (\%)} = \frac{signal\ value\ of\ positive\ control\ group - signal\ value\ of\ compound\ group\ at\ different\ concentrations}{signal\ value\ of\ positive\ control\ group - signal\ value\ of\ negative\ control\ group}$$

$$\times 100\%$$

Table 2 Inhibitory effects of test compounds on CD73 bound to the surfaces of A375 cells

| Test compound | $IC_{50}$ (nM) |
|---|---|
| Reference compound 1 | 247 |
| Reference compound 2 | 67.3 |

(continued)

| | |
|---|---|
| Reference compound 3 | 162 |
| 1 | 30.7 |
| 3 | 26.8 |
| 4 | 134 |
| 7 | 54.9 |
| 9 | 5.56 |
| 11 | 39.8 |
| 18 | 17.6 |

The test results indicate that the compounds of the present disclosure have relatively strong inhibitory activity to CD73 enzyme bound to the surfaces of A375 cells, and the inhibitory activity is significantly stronger than that of the reference compounds.

Test Example 3: plasma protein binding rate of compound

Plasma protein binding rates of the compounds were detected by equilibrium dialysis (HTDialysis, HTD 96b). The compound was diluted with DMSO to form a 0.5 mM stock solution, and the stock solution was diluted 25 times with a 0.05 M sodium phosphate buffer to form a 20 μM working solution. Plasma was placed in a new 96-well plate according to 380 μL/well, then the working solution was added to and uniformly mixed with the plasma according to 20 μL/well, the final concentration of the compound was 1 μM, and each well contained 0.2% DMSO.

100 μL of 0.05 M sodium phosphate buffer was added to a receiving side of each dialysis chamber (HTD 96b), and 100 μL of plasma containing the compound was added to a supply side. The dialysis apparatus was closed with a plastic lid, and incubated with shaking at 37°C for 5 h.

After the incubation was completed, 25 μL of sample was collected respectively from the supply side and the receiving side of the dialysis chamber, and placed in a new 96-well plate, an equal volume of blank plasma was added to and uniformly mixed with each sample on the supply side, an equal volume of 0.05 M sodium phosphate buffer was added to and uniformly mixed with each sample on the receiving side. 200 μL of acetonitrile solution containing the internal standard was added to each well, the 96-well plate was vortex-shaken at 600 rpm for 10 min, and centrifuged at 5,594 g for 15 min (Thermo Multifuge × 3R), 50 μL of supernatant was transferred to a new 96-well plate, and the sample was mixed with 50 μL of ultrapure water and subjected to LC-MS/MS analysis.

A plasma protein binding rate and a fraction unbound in plasma were calculated according to the following formulas:

$$\%\ \text{binding rate} = 100 \times ([\text{supply side concentration}]_{5h} - [\text{receiving side concentration}]_{5h}) / [\text{supply side concentration}]_{5h};$$

and

$$\%\ \text{fraction unbound in plasma} = 100 - \%\ \text{binding rate}.$$

Table 3 Fractions unbound in plasma of test compounds

| Compound | Human (%) | Mouse (%) | Canine (%) |
|---|---|---|---|
| Reference compound 2 | 9.8 | 26.1 | 17.7 |
| Reference compound 3 | 10.3 | 12.2 | 12.6 |
| 1 | 27.36 | 47.42 | 24.5 |

The results of the plasma protein binding rate test indicate that the compound of the present disclosure has a high fraction unbound in plasma, and shows better druggability compared to the reference compounds.

Test Example 4: activity of compound in relieving inhibition of AMP-induced proliferations of Human $CD4^+$ T cells

Primary Human $CD4^+$ T cells were cultured in RPMI1640 (BasalMedia, L210KJ) medium containing 10% FBS (Gibco; 10099-141C) and 1% P/S (Thermo; 10378016). On day 1 of the experiment, $CD4^+$ T cells (Allcells Shanghai, PB009-2F-C) were thawed, and inoculated into two replicate wells according to $3\times10^4$ cells/well and 50 μL/well. The compound was diluted with a complete medium to form a stock solution (4×) of an appropriate concentration gradient, the compound solution was added to the above cells according to 50 μL/well, and the mixture was pre-incubated at 37°C for 30 min. IL-2 (Sino Biological; GMP-11848-HNAE) was diluted with a complete medium to form an IL-2 solution (4×), CD3/CD28 beads (Gibco; 11131D) were resuspended in the IL-2 solution (4×), and added to the above cells according to 50 μL/well, the final concentration of IL-2 was 5 U/mL, each well contained 1 μL of cleaned CD3/CD28 beads, and the mixture was incubated at 37°C for 60 min. Meanwhile, a positive reference group (Human $CD4^+$ T+IL-2+CD3/CD28 beads) and a negative reference group (Human $CD4^+$ T+IL-2+CD3/CD28 beads+AMP) were set. AMP (Sigma; A1752-5G) was diluted with a complete medium to form an AMP solution (4×), the AMP solution was added to the above cells according to 50 μL/well, the final concentration was 0.3 mM, and the mixture was incubated at 37°C. On day 3 of the experiment, the AMP solution was added to the above plate according to 20 μL/well, and the final concentration was 0.3 mM. On day 5, the proliferation of the cells was detected by using a CCK8 kit (DojinDO; CK04).

Inhibition rates of the compound at different concentrations in relieving AMP-induced proliferation inhibition of $CD4^+$ T cells were calculated according to the following formula, the concentration of the compound was taken as the X-axis, the inhibition rate was taken as the Y-axis, and an $EC_{50}$ value of the compound in relieveing the AMP-induced proliferation inhibition of $CD4^+$ T cells was calculated by using Prism software.

$$\text{Inhibition rate (\%)} = \frac{signal\ value\ of\ positive\ control\ group - signal\ value\ of\ compound\ group\ at\ different\ concentrations}{signal\ value\ of\ positive\ control\ group - signal\ value\ of\ negative\ control\ group}$$

$$\times 100\%$$

Table 4 Effects of test compounds on relieving AMP-induced proliferation inhibition of $CD4^+$ T cells

| Test compound | $EC_{50}$ (nM) |
|---|---|
| Reference compound 2 | 93.7 |
| Reference compound 3 | 341 |
| 1 | 71.9 |
| 3 | 111 |
| 4 | 1070 |
| 9 | 32.6 |
| 11 | 169 |

The experimental results indicate that the compounds of the present disclosure can significantly relieve AMP-induced proliferation inhibition of $CD4^+$ T cells, showing better activities than the reference compounds.

Test Example 5: pharmacokinetic test

In a pharmacokinetic test on mice, male ICR mice of 20 g to 25 g were used and fasted overnight. 3 mice were selected for orally intragastric administration (10 mg/kg). Before administration, and 15 min, 30 min, 1 h, 2 h, 4 h, 8 h, and 24 h after administration, blood was sampled from each mouse. Another 3 mice were selected for intravenous injection administration (3 mg/kg), and before administration, and 15 min, 30 min, 1 h, 2 h, 4 h, 8 h, and 24 h after administration, blood was sampled from each mouse. The blood sample was centrifuged at 6,800 g and 2 to 8°C for 6 min, and plasma was collected and stored at -80°C. The plasma of each time point was vortex-mixed for 1 min with an acetonitrile solution containing the internal standard with a volume 3-5 times that of the plasma of each time point, and centrifuged at 13,000 rpm and 4°C for 10 min, a supernatant was collected and mixed with water with a volume 3 times

that of the supernatant, and an appropriate amount of the mixed solution was used for LC-MS/MS analysis. The main pharmacokinetic parameters were analyzed by using a WinNonlin 7.0 non-compartmental model.

In a pharmacokinetic test on dogs, male Beagle dogs of 8 to10 kg were used and fasted overnight. 3 Beagle dogs were selected for orally intragastric administration (5 mg/kg). Another 3 Beagle dogs were selected for intravenous injection administration (1 mg/kg). Other operations were the same as the pharmacokinetic test on mice.

Table 5 Results of pharmacokinetic test on mice

| Test compound | Pharmacokinetic parameters of mouse | | | | | | | |
|---|---|---|---|---|---|---|---|---|
| | Intravenous injection administration (3 mg/kg) | | | | Orally intragastric administration (10 mg/kg) | | | |
| | CL (L/h/kg) | $V_z$ (L/kg) | $AUC_{0-t}$ (h*ng/mL) | $T_{1/2}$ (h) | Cmax (ng/mL) | Tmax (hr) | AUC0-t (h*ng/mL) | $T_{1/2}$ (h) |
| Reference compound 1 | 5.11 | 14.72 | 585 | 1.99 | 1147.73 | 0.25 | 821 | 1.17 |
| Reference compound 2 | 1.32 | 2.40 | 2359 | 1.25 | 4539.87 | 0.42 | 5379 | 1.09 |
| Reference compound 3 | 4.2 | 1.67 | 730.2 | 0.28 | 1266.2 | 0.33 | 1272.1 | 1.19 |
| 1 | - | - | - | | 4302.67 | 0.42 | 5717 | 3.31 |
| 3 | 0.22 | 1.17 | 13846.1 | 3.72 | 12112.9 | 0.83 | 106704.8 | 2.70 |
| 4 | 0.20 | 1.05 | 15243 | 3.68 | 16062.40 | 3.00 | 185389.27 | 2.85 |
| 9 | - | - | - | | 6601.90 | 1.17 | 65441 | 4.87 |
| 11 | 0.47 | 2.57 | 6952 | 3.72 | 5531.70 | 0.67 | 36211 | 3.06 |
| 18 | 0.62 | 1.50 | 5073 | 1.82 | 4409.70 | 0.42 | 9799 | 1.43 |
| Note: - indicates undetected | | | | | | | | |

The results of the pharmacokinetic test on mice indicate that the compounds of the present disclosure show good pharmacokinetic properties, especially compound 3, compound 4, compound 9, and compound 11, of which pharmacokinetic properties are significantly improved compared to the reference compounds.

Table 6 Results of pharmacokinetic test on dogs

| Test compound | Pharmacokinetic parameters of dog | | | | | | | |
|---|---|---|---|---|---|---|---|---|
| | Intravenous injection administration (1 mg/kg) | | | | Orally intragastric administration (5mg/kg) | | | |
| | CL (L/h/kg) | $V_z$ (L/kg) | $AUC_{0-t}$ (h*ng/mL) | $T_{1/2}$ (h) | Cmax (ng/mL) | Tmax (hr) | AUC0-t (h*ng/mL) | $T_{1/2}$ (h) |
| Reference compound 2 | 0.65 | 1.33 | 1556.26 | 1.37 | 3623.17 | 0.50 | 9001.85 | 3.30 |
| Reference compound 3 | 2.16 | 1.14 | 511.74 | 0.39 | 1204.70 | 0.42 | 1541.90 | 0.93 |
| 1 | 0.73 | 0.99 | 1455.50 | 0.94 | 5475.10 | 1.17 | 16899.20 | 2.67 |
| 4 | 0.18 | 0.95 | 5814.00 | 3.63 | 7551.00 | 1.17 | 65716.30 | 4.13 |
| 11 | 0.53 | 0.66 | 2099.92 | 0.89 | 5713.20 | 0.83 | 16571.90 | 2.27 |

The results of the pharmacokinetic test on dogs indicate that the compounds of the present disclosure all have comparable or better exposure compared to the reference compounds, especially compound 4 and compound 11, of which the exposure is much higher than that of the reference compounds, showing excellent pharmacokinetic properties and good druggability.

Test Example 6: In vivo efficacy on A375 melanoma

A375 cells in logarithmic growth phase were collected, cultured in Mitomycin C for 2 h, and washed three times with PBS. After 5 days of co-culture of PBMC and A375 cells, PBMC and freshly digested A375 cells were collected, $5\times10^5$ PBMC and $4\times10^6$ A375 cells were inoculated subcutaneously on the right side of NCG mice according to 0.2 mL/mouse (containing 50% Matrigel). After inoculation, the mice were randomly divided into a model group and an administration group according to body weight of the mice, tumor size and animal weight were measured and recorded before and during administration, and tumor sizes of the model group and the administration group were compared after treatment to determine the efficacy.

Table 7 In vivo efficacy on A375 cells

| Group | Mean±SEM | P value (vs Reference) | %TGI |
|---|---|---|---|
| Reference group | 1965±266.1 | - | - |
| anti PD-1, 10 mg/kg | 1472±194.9 | 0.0453* | 25.08 |
| Reference compound 3, 40 mg/kg + anti PD-1, 10 mg/kg | 1149±167.8 | 0.0042** | 41.52 |
| Compound 1, 40 mg/kg + anti PD-1, 10 mg/kg | 713.7±89.22 | <0.0001**** | 63.68 |

One-way ANOVALSD(L) test

The results of the in vivo efficacy test indicate that the compound of the present disclosure can significantly improve an inhibitory effect of a PD-1 antibody (Toripalimab, TopAlliance, 202001002) on the growth of A375 melanoma when used in combination with the PD-1 antibody, and a synergetic effect of compound 1 is better than the reference compound at the same dose (see FIG. 1).

## Claims

1. A compound represented by formula I, or a tautomer, stereoisomer, hydrate, solvate, pharmaceutically acceptable salt, or prodrug thereof:

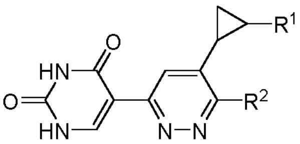

I

,

wherein:

$R^1$ is selected from

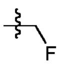, 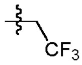, 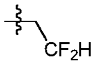, 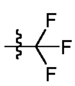, 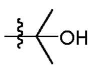, 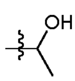, 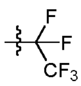, or 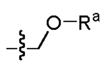,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; and the four- to eight-membered heterocycloalkyl contains 1 to 3 heteroatoms selected from one or more of N, S, O, and P; and the four-to eight-membered heterocycloalkenyl contains 1 to 3 heteroatoms selected from one or more of N, S, O, and P; and

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five- to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four-to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the ($C_1$-$C_6$ alkyl)-O- substituted with $R^b$, the ($C_1$-$C_6$ alkyl)-S- substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituent groups are identical or different;

and wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four-to eight-membered heterocycloalkenyl unsubstituted or substituted with $R^b$ contains 1 to 3 heteroatoms selected from one or more of N, S, O and P.

**2.** A compound represented by formula I, or a tautomer, stereoisomer, hydrate, solvate, pharmaceutically acceptable salt, or prodrug thereof:

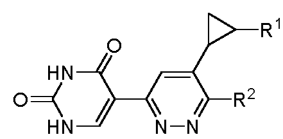

I ,

wherein:

$R^1$ is selected from

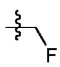, 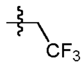, 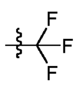, 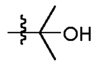, 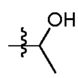, 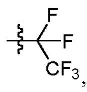, or 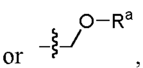,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four- to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P; and

$R^2$ is selected from hydrogen, halogen, hydroxyl, cyano, amino, $C_1$-$C_6$ alkyl unsubstituted or substituted with $R^b$, ($C_1$-$C_6$ alkyl)-O- unsubstituted or substituted with $R^b$, ($C_1$-$C_6$ alkyl)-S- unsubstituted or substituted with $R^b$, five- to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four-to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five- to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different, and

wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

**3.** The compound represented by formula I, or the tautomer, stereoisomer, hydrate, solvate, pharmaceutically acceptable salt, or prodrug thereof according to claim 1 or 2, wherein

when $R^a$ is $C_1$-$C_6$ alkyl, the $C_1$-$C_6$ alkyl is $C_1$-$C_4$ alkyl, and preferably methyl, ethyl, n-propyl, isopropyl, n-butyl, or isobutyl; and/or
when $R^a$ is $C_1$-$C_6$ alkyl substituted with 1 to 5 identical or different halogen atoms, the $C_1$-$C_6$ alkyl is $C_1$-$C_4$ alkyl, and preferably methyl, ethyl, n-propyl, isopropyl, n-butyl, or isobutyl; and/or
when $R^a$ is $C_1$-$C_6$ alkyl substituted with 1 to 5 identical or different halogen atoms, the halogen atoms are F, Cl, Br or I, and preferably F or Cl; and/or
when $R^a$ is $C_1$-$C_6$ alkyl substituted with 1 to 5 identical or different halogen atoms, the number of the halogen atoms is 1, 2, or 3, and preferably 3; and/or
when $R^a$ is $C_3$-$C_6$ cycloalkyl, the $C_3$-$C_6$ cycloalkyl is independently cyclopropyl, cyclobutyl, cyclopentyl, or cyclohexyl, and preferably cyclopropyl; and/or
when $R^a$ is five- to eight-membered aryl, the five- to eight-membered aryl is independently phenyl or naphthyl, and preferably phenyl; and/or
when $R^a$ is five- to eight-membered heteroaryl, the five- to eight-membered heteroaryl is independently pyrrole, pyrazole, triazole, furan, oxazole, thiophene, thiazole, pyridine, pyrazine, or pyrimidine, and preferably pyrazole, furan, thiophene, or pyridine; and/or
when $R^a$ is four- to eight-membered heterocycloalkyl, the four- to eight-membered heterocycloalkyl is independently azetidine, oxetane, tetrahydropyrrolidinyl, tetrahydrofuranyl, hexahydropyran, or tetrahydro-2H-thiopyran 1,1-dioxide, and preferably azetidine or oxetane; and/or
when $R^a$ is four- to eight-membered heterocycloalkenyl, the four- to eight-membered heterocycloalkenyl is independently dihydropyridyl, tetrahydropyridyl, tetrahydropyrimidinyl, pyrrolinyl, imidazolinyl, pyrazolinyl, dihydroimidazolyl, dihydropyrazolyl, dihydrooxazolyl, dihydrooxadiazolyl, dihydrothiazolyl, dihydroisothiazolyl, dihydrothienyl, dihydropyrrolyl, 3,4-dihydro-2H-pyranyl, dihydrofuranyl, dihydropyrazinyl, dihydropyrimidyl or fluorodihydrofuranyl, and preferably 1,2,3,4-tetrahydropyridyl, 1,2-dihydropyridyl, 1,4-dihydropyridyl, 1,2,3,6-tetrahydropyridyl, 3,4-dihydro-2H-pyranyl, or dihydrofuranyl.

**4.** The compound represented by formula I, or the tautomer, stereoisomer, hydrate, solvate, pharmaceutically acceptable salt, or prodrug thereof according to claim 1 or 2, wherein

$R^2$ is cyano; and/or
when $R^2$ is halogen, the halogen is F, Cl, Br, or I, and preferably Cl; and/or
when $R^2$ is $C_1$-$C_6$ alkyl unsubstituted or substituted with $R^b$, the $C_1$-$C_6$ alkyl is $C_1$-$C_4$ alkyl, and preferably methyl, ethyl, n-propyl, isopropyl, n-butyl, or isobutyl; and/or
when $R^2$ is ($C_1$-$C_6$ alkyl)-O- unsubstituted or substituted with $R^b$, the ($C_1$-$C_6$ alkyl)-O- is ($C_1$-$C_4$ alkyl)-O-, and preferably is methyl-O-; and/or
when $R^2$ is ($C_1$-$C_6$ alkyl)-S- unsubstituted or substituted with $R^b$, the ($C_1$-$C_6$ alkyl)-S- is ($C_1$-$C_4$ alkyl)-S-, and preferably methyl-S-; and/or
when $R^2$ is five- to eight-membered aryl unsubstituted or substituted with $R^b$, the five- to eight-membered aryl is independently phenyl or naphthyl, and preferably phenyl; and/or
when $R^2$ is five- to eight-membered heteroaryl unsubstituted or substituted with $R^b$, the five- to eight-membered heteroaryl is independently pyrrole, pyrazole, triazole, furan, oxazole, thiophene, thiazole, pyridine, pyrazine, or pyrimidine, and preferably pyrazole, furan, thiophene, or pyridine; and/or
when $R^2$ is four- to eight-membered heterocycloalkyl unsubstituted or substituted with $R^b$, the four- to eight-membered heterocycloalkyl is independently azetidine, oxetane, tetrahydropyrrolidinyl, tetrahydrofuranyl, hexahydropyran, or tetrahydro-2H-thiopyran 1,1-dioxide, and preferably azetidine or oxetane; and/or
when $R^2$ is four- to eight-membered heterocycloalkenyl unsubstituted or substituted with $R^b$, the four- to eight-membered heterocycloalkenyl is independently dihydropyridyl, tetrahydropyridyl, tetrahydropyrimidinyl, pyrrolinyl, imidazolinyl, pyrazolinyl, dihydroimidazolyl, dihydropyrazolyl, dihydrooxazolyl, dihydrooxadiazolyl, dihydrothiazolyl, dihydroisothiazolyl, dihydrothienyl, dihydropyrrolyl, 3,4-dihydro-2H-pyranyl, dihydrofuranyl, dihydropyrazinyl, dihydropyrimidyl, or fluorodihydrofuranyl, and preferably 1,2,3,4-tetrahydropyridyl, 1,2-dihydropyridyl, 1,4-dihydropyridyl, 1,2,3,6-tetrahydropyridyl, 3,4-dihydro-2H-pyranyl, or dihydrofuranyl; and/or
when $R^2$ is $C_2$-$C_6$ alkenyl unsubstituted or substituted with $R^b$, the $C_2$-$C_6$ alkenyl is vinyl, 1-propenyl, 2-propenyl, or allyl, and preferably vinyl or allyl.

**5.** The compound represented by formula I, or the tautomer, stereoisomer, hydrate, solvate, pharmaceutically acceptable salt, or prodrug thereof according to claim 1 or 2, wherein

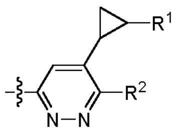 is 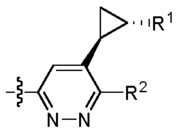 or 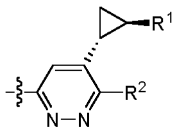 .

6. The compound represented by formula I, or the tautomer, stereoisomer, hydrate, solvate, pharmaceutically acceptable salt, or prodrug thereof according to claim 1 or 2, wherein

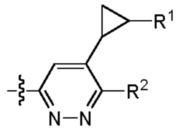 is 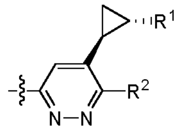 ,

whereinR$^1$ is selected from 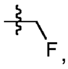,

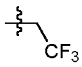, 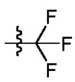, 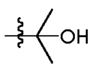, 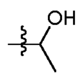, 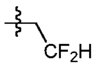, or 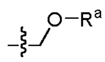,

wherein $R^a$ is $C_1$-$C_6$ alkyl unsubstituted or substituted with one or more identical or different halogen atoms.

7. The compound represented by formula I, or the tautomer, stereoisomer, hydrate, solvate, pharmaceutically acceptable salt, or prodrug thereof according to claim 6, wherein

$R^a$ is $C_1$-$C_4$ alkyl unsubstituted or substituted with 1 to 5 identical or different halogen atoms, and
$R^2$ is selected from hydrogen, halogen, cyano, or $C_1$-$C_4$ alkyl unsubstituted or substituted with $R^b$, wherein $R^b$ is each independently halogen.

8. The compound represented by formula I, or the tautomer, stereoisomer, hydrate, solvate, pharmaceutically acceptable salt, or prodrug thereof according to claim 1 or 2, wherein

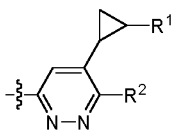 is 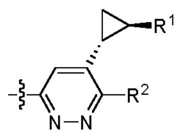 ,

wherein $R^1$ is selected from

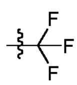 or 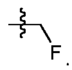.

9. The compound represented by formula I, or the tautomer, stereoisomer, hydrate, solvate, pharmaceutically acceptable salt, or prodrug thereof according to claim 8, wherein $R^2$ is selected from hydrogen, halogen, cyano, or $C_1$-$C_4$ alkyl unsubstituted or substituted with $R^b$, wherein $R^b$ is each independently halogen.

10. The compound represented by formula I, or the tautomer, stereoisomer, hydrate, solvate, pharmaceutically acceptable salt, or prodrug thereof according to claim 1, being:

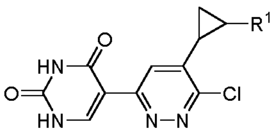
II
,
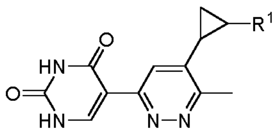
III
, or
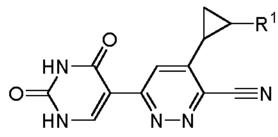
III-A
,

wherein $R^1$ is

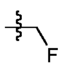 , 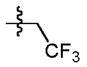 , 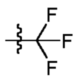 , 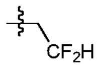 , 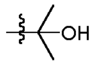 , 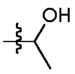 , 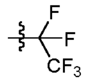 , or 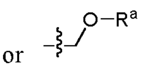 ,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four-to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P.

**11.** The compound represented by formula I, or the tautomer, stereoisomer, hydrate, solvate, pharmaceutically acceptable salt, or prodrug thereof according to claim 1 or 2, being:

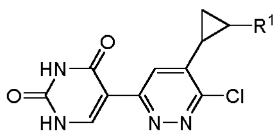
II
or
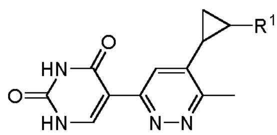
III
,

wherein $R^1$ is

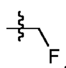 , 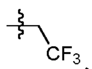 , 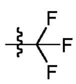 , 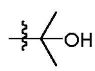 , 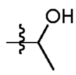 , 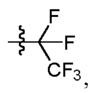 , or 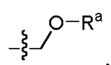 ,

wherein $R^a$ is independently selected from hydrogen, $C_1$-$C_6$ alkyl, $C_3$-$C_6$ cycloalkyl, five- to eight-membered aryl, five- to eight-membered heteroaryl, four- to eight-membered heterocycloalkyl, or $C_1$-$C_6$ alkyl substituted with 1 to 5 identical or different halogen atoms, wherein the five- to eight-membered heteroaryl contains 1 to 3 heteroatoms selected from one or more of N, S, O, and P; the four- to eight-membered heterocycloalkyl contains 1 to 3 heteroatoms selected from one or more of N, S, O, and P; and the four- to eight-membered heterocycloalkenyl contains 1 to 3 heteroatoms selected from one or more of N, S, O, and P.

**12.** The compound represented by formula I, or the tautomer, stereoisomer, hydrate, solvate, pharmaceutically acceptable salt, or prodrug thereof according to claim 1 or 2, wherein the compound has a structural formula of:

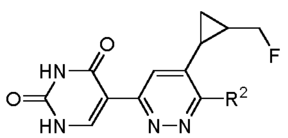
IV
,

wherein $R^2$ is selected from hydrogen, halogen, hydroxyl, cyano, amino, $C_1$-$C_6$ alkyl unsubstituted or substituted with $R^b$, (Ci-$C_6$ alkyl)-O- unsubstituted or substituted with $R^b$, ($C_1$-$C_6$ alkyl)-S- unsubstituted or substituted with $R^b$, five- to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsub-

stituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five-to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different,

wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

**13.** The compound represented by formula I, or the tautomer, stereoisomer, hydrate, solvate, pharmaceutically acceptable salt, or prodrug thereof according to claim 1 or 2, wherein the compound has a structural formula of:

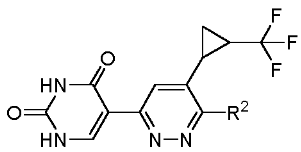

V

wherein $R^2$ is selected from hydrogen, halogen, hydroxyl, cyano, amino, $C_1$-$C_6$ alkyl unsubstituted or substituted with $R^b$, (Ci-$C_6$ alkyl)-O- unsubstituted or substituted with $R^b$, ($C_1$-$C_6$ alkyl)-S- unsubstituted or substituted with $R^b$, five- to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five-to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-,wherein when more than one substituents are present, the more than one substituents are identical or different,

wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

**14.** The compound represented by formula I, or the tautomer, stereoisomer, hydrate, solvate, pharmaceutically acceptable salt, or prodrug thereof according to claim 1 or 2, wherein the compound has a structural formula of:

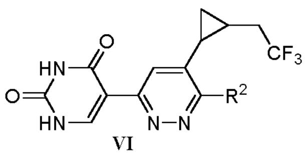

VI

,

wherein $R^2$ is selected from hydrogen, halogen, hydroxyl, cyano, amino, $C_1$-$C_6$ alkyl unsubstituted or substituted with $R^b$, (Ci-$C_6$ alkyl)-O- unsubstituted or substituted with $R^b$, ($C_1$-$C_6$ alkyl)-S- unsubstituted or substituted with $R^b$, five- to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five-

to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different,
wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

**15.** The compound represented by formula I, or the tautomer, stereoisomer, hydrate, solvate, pharmaceutically acceptable salt, or prodrug thereof according to claim 1 or 2, wherein the compound has a structural formula of:

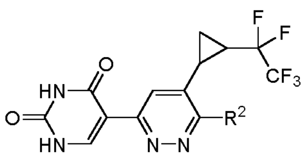
VII

,

wherein $R^2$ is selected from hydrogen, halogen, hydroxyl, cyano, amino, $C_1$-$C_6$ alkyl unsubstituted or substituted with $R^b$, (Ci-$C_6$ alkyl)-O- unsubstituted or substituted with $R^b$, ($C_1$-$C_6$ alkyl)-S- unsubstituted or substituted with $R^b$, five- to eight-membered aryl unsubstituted or substituted with $R^b$, five- to eight-membered heteroaryl unsubstituted or substituted with $R^b$, four- to eight-membered heterocycloalkyl unsubstituted or substituted with $R^b$, four- to eight-membered heterocycloalkenyl unsubstituted or substituted with $R^b$, or $C_2$-$C_6$ alkenyl unsubstituted or substituted with $R^b$, wherein, in the $C_1$-$C_6$ alkyl substituted with $R^b$, the (Ci-$C_6$ alkyl)-O- substituted with $R^b$, the ($C_1$-$C_6$ alkyl)-S- substituted with $R^b$, the five- to eight-membered aryl substituted with $R^b$, the five-to eight-membered heteroaryl substituted with $R^b$, the four- to eight-membered heterocycloalkyl substituted with $R^b$, the four-to eight-membered heterocycloalkenyl substituted with $R^b$, and the $C_2$-$C_6$ alkenyl substituted with $R^b$, one or more $R^b$ substituents are present and each independently selected from halogen, hydroxyl, cyano, amino, $C_1$-$C_6$ alkyl, $C_3$-$C_6$ cycloalkyl, or ($C_1$-$C_6$ alkyl)-O-, wherein when more than one substituents are present, the more than one substituents are identical or different,
wherein the five- to eight-membered heteroaryl unsubstituted or substituted with $R^b$ contains 1 to 3 heteroatoms selected from one or more of N, S, O, and P; the four- to eight-membered heterocycloalkyl unsubstituted or substituted with $R^b$ contains 1 to 3 heteroatoms selected from one or more of N, S, O, and P; and the four- to eight-membered heterocycloalkenyl unsubstituted or substituted with $R^b$ contains 1 to 3 heteroatoms selected from one or more of N, S, O and P.

**16.** The compound represented by formula I, or the tautomer, stereoisomer, hydrate, solvate, pharmaceutically acceptable salt, or prodrug thereof according to claim 1, wherein the compound represented by formula I is any one of the following compounds:

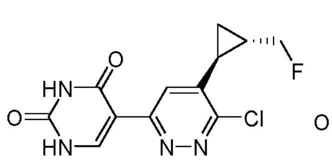
1

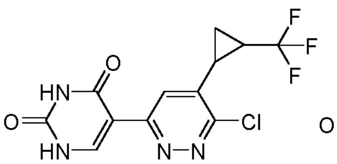
Trans racemic mixtures
2

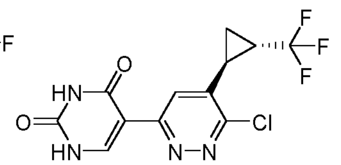
3

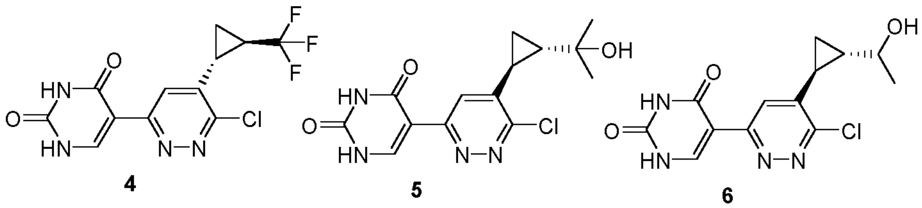

4 5 6

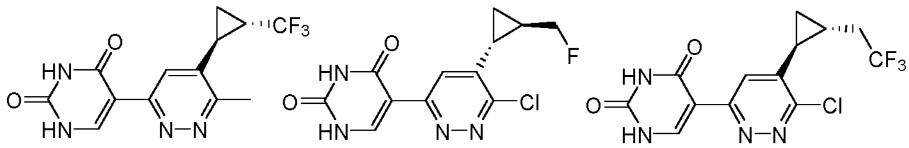

7 8 9

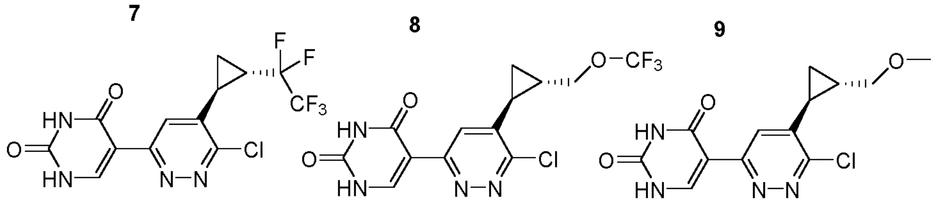

10 11 12

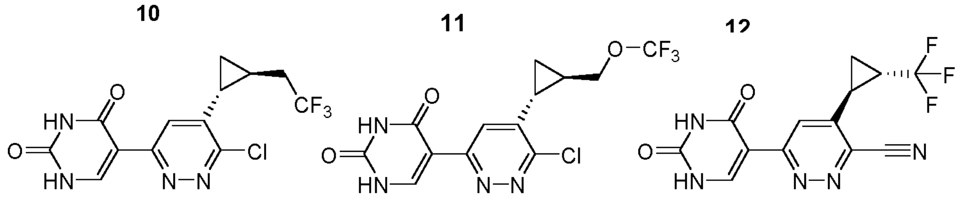

13 14 15

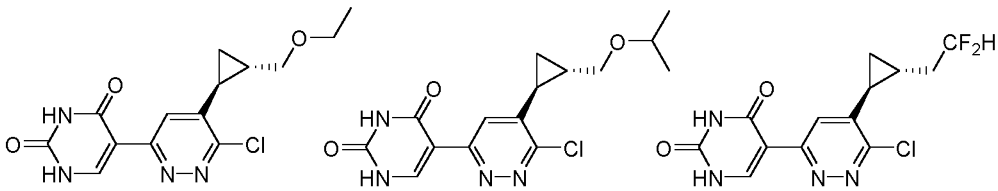

16 17 18

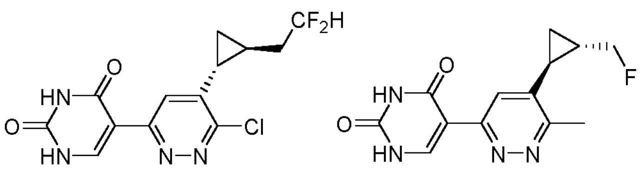

19 20

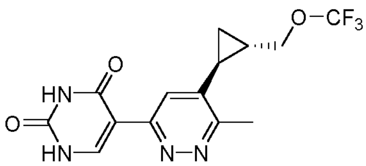

21

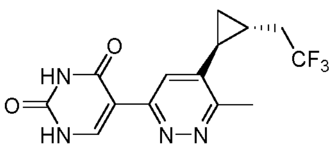

22

**17.** A pharmaceutical composition, comprising:

the compound represented by formula I, or the tautomer, stereoisomer, hydrate, solvate, pharmaceutically acceptable salt, or prodrug thereof according to any one of claims 1 to 16; and
a pharmaceutically acceptable excipient.

**18.** Use of the compound represented by formula I, or the tautomer, stereoisomer, hydrate, solvate, pharmaceutically acceptable salt, or prodrug thereof according to any one of claims 1 to 16 in combination with PD-1/PD-L1/CTLA-4 antibodies or PD-1/PD-L1/CTLA-4 inhibitors in the preparation of a drug for treating a CD73-associated disease.

**19.** Use of the compound represented by formula I, or the tautomer, stereoisomer, hydrate, solvate, pharmaceutically acceptable salt, or prodrug thereof according to any one of claims 1 to 16, or the pharmaceutical composition according to claim 17 in the preparation of a drug for treating a CD73-associated disease.

**20.** The use according to claim 18 or 19, wherein the CD73-associated disease is cancer.

**21.** The use according to claim 20, wherein the cancer is bladder cancer, breast cancer, cholangiocarcinoma, rectal cancer, colon cancer, gastric cancer, gallbladder cancer, glioblastoma, head and neck cancer, liver cancer, lung cancer, lymphoma, medulloblastoma, melanoma, ovarian cancer, pancreatic cancer, prostate cancer or kidney cancer.

**22.** A method for treating a CD73-associated disease, **characterized by** comprising: administering the compound represented by formula I, or the tautomer, stereoisomer, hydrate, solvate, pharmaceutically acceptable salt, or prodrug thereof according to any one of claims 1 to 16 and/or the pharmaceutical composition according to claim 17 to a subject in need.

**23.** The method according to claim 22, wherein the CD73-associated disease is cancer.

**24.** The method according to claim 23, wherein the cancer is bladder cancer, breast cancer, cholangiocarcinoma, rectal cancer, colon cancer, gastric cancer, gallbladder cancer, glioblastoma, head and neck cancer, liver cancer, lung cancer, lymphoma, medulloblastoma, melanoma, ovarian cancer, pancreatic cancer, prostate cancer, or kidney cancer.

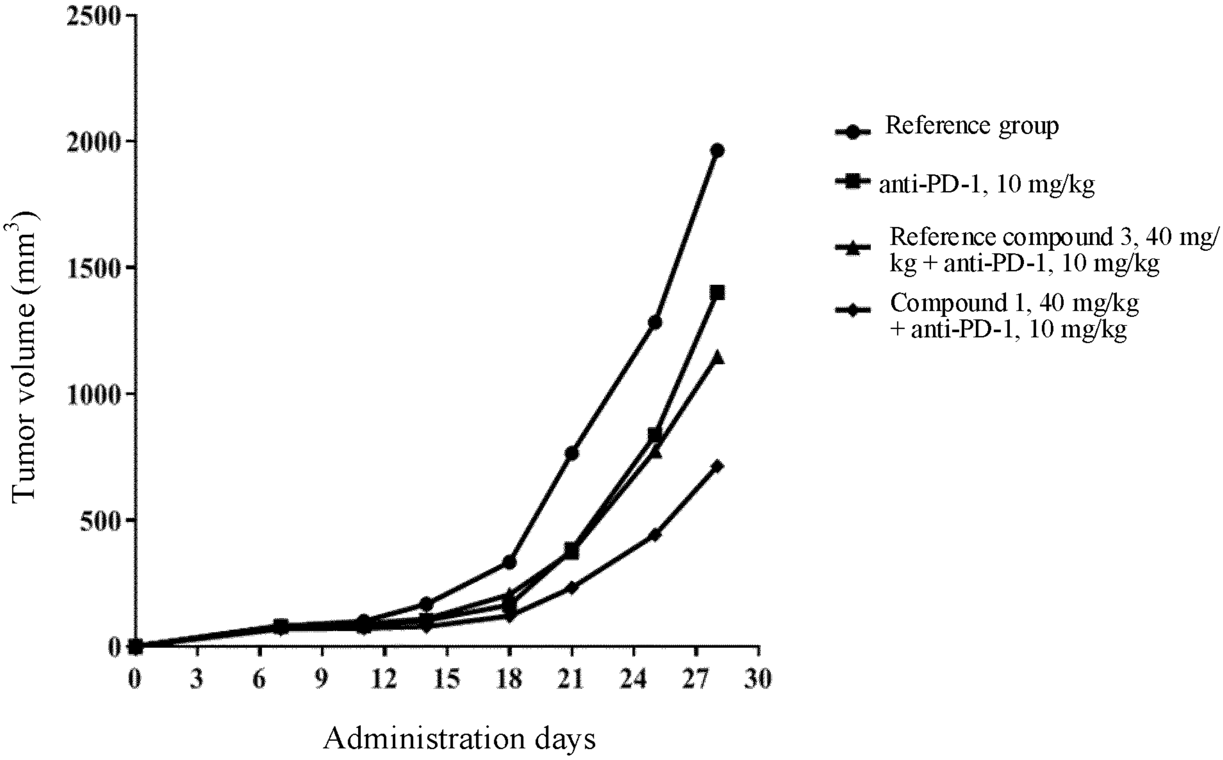
2500
2000
1500
1000
500
0
Tumor volume (mm3)
0
3
6
9
12
15
18
21
24
27
30
Administration days
Reference group
anti-PD-1, 10 mg/kg
Reference compound 3, 40 mg/kg + anti-PD-1, 10 mg/kg
Compound 1, 40 mg/kg + anti-PD-1, 10 mg/kg

FIG. 1

**INTERNATIONAL SEARCH REPORT**

International application No.
**PCT/CN2021/129079**

**A. CLASSIFICATION OF SUBJECT MATTER**

C07D 403/04(2006.01)i； A61P 35/00(2006.01)i； A61K 31/513(2006.01)i

According to International Patent Classification (IPC) or to both national classification and IPC

**B. FIELDS SEARCHED**

Minimum documentation searched (classification system followed by classification symbols)

C07D A61P A61K

Documentation searched other than minimum documentation to the extent that such documents are included in the fields searched

Electronic data base consulted during the international search (name of data base and, where practicable, search terms used)

CNABS, CNKI, EPODOC, WPI, REGISTRY(STN), CAPLUS(STN): CD73, 抑制剂, 嘧啶, 二酮, 癌症, 肿瘤, inhibitor, pyrimidine, dione, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| X | CN 111819173 A (ELI LILLY AND COMPANY) 23 October 2020 (2020-10-23) description paragraphs [0007], [0051]-[0069], embodiments 1-8, 10-11, 16-18, 22, 24, 30 | 1-24 |
| PX | WO 2021041319 A1 (ELI LILLY AND COMPANY) 04 March 2021 (2021-03-04) description, page 1, paragraph 1, page 2, paragraphs 1-3, page 8, paragraph 1 to page 11, paragraph 3, embodiment 1, preparation examples 13-15 | 1-24 |
| A | WO 2017120508 A1 (ARCUS BIOSCIENCES, INC.) 13 July 2017 (2017-07-13) entire document | 1-24 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2022** | **26 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/129079**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-24**
because they relate to subject matter not required to be searched by this Authority, namely:

[1] The method of claims 22-24 is a method for the treatment of a disease, however, the present search report is made on the basis of the corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/129079**

| Patent document cited in search report | Publication date (day/month/year) | Patent family member(s) | Publication date (day/month/year) |
|---|---|---|---|
| CN 111819173 A | 23 October 2020 | JP 2020517655 A | 18 June 2020 |
| | | JP 6794560 B2 | 02 December 2020 |
| | | CR 20200376 A | 23 October 2020 |
| | | EP 3759096 A1 | 06 January 2021 |
| | | WO 2019168744 A1 | 06 September 2019 |
| | | CL 2020002158 A1 | 13 November 2020 |
| | | AU 2019228473 A1 | 20 August 2020 |
| | | AU 2019228473 B2 | 25 February 2021 |
| | | TW 201945002 A | 01 December 2019 |
| | | TW I702954 B | 01 September 2020 |
| | | PH 12020551464 A1 | 01 September 2021 |
| | | EC SP20052897 A | 30 September 2020 |
| | | MA 52413 A | 06 January 2021 |
| | | JO P20200209 A1 | 01 September 2019 |
| | | US 2021002257 A1 | 07 January 2021 |
| | | US 11028074 B2 | 08 June 2021 |
| | | KR 20200116965 A | 13 October 2020 |
| | | SG 11202008366 R A | 29 September 2020 |
| | | DO P2020000148 A | 15 September 2020 |
| | | UA 123890 C2 | 16 June 2021 |
| | | IL 277006 D0 | 29 October 2020 |
| | | PE 20210177 A1 | 29 January 2021 |
| | | CA 3092661 A1 | 06 September 2019 |
| | | BR 112020016066 A2 | 08 December 2020 |
| | | CO 2020010191 A2 | 31 August 2020 |
| WO 2021041319 A1 | 04 March 2021 | None | |
| WO 2017120508 A1 | 13 July 2017 | KR 20180100638 A | 11 September 2018 |
| | | US 2021371449 A1 | 02 December 2021 |
| | | MX 2018008350 A | 30 May 2019 |
| | | US 2019309010 A1 | 10 October 2019 |
| | | US 10981944 B2 | 20 April 2021 |
| | | CA 3009196 A1 | 13 July 2017 |
| | | IL 260260 D0 | 31 July 2018 |
| | | PH 12018501361 A1 | 27 February 2019 |
| | | BR 112018013827 A2 | 11 December 2018 |
| | | UA 124529 C2 | 05 October 2021 |
| | | US 2021002322 A1 | 07 January 2021 |
| | | US 11001603 B2 | 11 May 2021 |
| | | TW 201805006 A | 16 February 2018 |
| | | CN 108697719 A | 23 October 2018 |
| | | WO 2017120508 A8 | 05 July 2018 |
| | | EP 3399984 A1 | 14 November 2018 |
| | | EP 3399984 A4 | 01 January 2020 |
| | | AU 2017206061 A1 | 19 July 2018 |
| | | AU 2017206061 A8 | 26 July 2018 |
| | | US 2017267710 A1 | 21 September 2017 |
| | | US 10239912 B2 | 26 March 2019 |
| | | EA 201891583 A1 | 31 January 2019 |
| | | EA 038565 B1 | 15 September 2021 |
| | | JP 2021167351 A | 21 October 2021 |

Form PCT/ISA/210 (patent family annex) (January 2015)

**INTERNATIONAL SEARCH REPORT**
**Information on patent family members**

International application No.
**PCT/CN2021/129079**

| Patent document cited in search report | Publication date (day/month/year) | Patent family member(s) | | | Publication date (day/month/year) |
|---|---|---|---|---|---|
| | | JP | 2019501223 | A | 17 January 2019 |
| | | JP | 6920344 | B2 | 18 August 2021 |
| | | CL | 2018001845 | A1 | 07 December 2018 |
| | | SG | 11201805506 Y | A | 30 July 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202011225900
- CN 202110480100
- WO 2019168744 A1

### Non-patent literature cited in the description


- **SOLEIMANI A et al.** *Biochimie,* 2020, vol. 176, 21-30
- **GAO Z et al.** *Biomed Res Int,* 2014, (2014), 460654
- **LINDEN J et al.** *Annu. Rev. Immunol.,* 2019, vol. 37, 325-347
- **KLEMENS M R et al.** *Biochem. Biophys. Res. Commun.,* 1990, vol. 172, 1371-7
- **YOUNG AET.** *Cancer Cell,* 2016, vol. 30 (3), 391-403
- **HARVEY JERRY B et al.** *Front Immunol,* 2020, vol. 11, 508
- **ALLARD B et al.** *Clin. Cancer Res.,* 2013, vol. 19, 5626-35
- **GOSWAMI S et al.** *Nat. Med.,* 2020, vol. 26, 39-46
- **WENNERBERG E et al.** *Cancer Immunol Res,* 2020, vol. 8, 465-478
- **MAEHR.** *J. Chem.,* 1985, vol. 62, 114-120